# EUROPEAN PATENT APPLICATION

(11) **EP 3 848 049 A1**
(43) Date of publication of application: **14.07.2021**
(21) Application number: 19855752.2
(22) Date of filing: 27.08.2019
(51) Int. Cl.: A61K 39/395, A61P 35/00, A61P 37/00

(54) **ANTI-TIM3 ANTIBODY PHARMACEUTICAL COMPOSITION AND USE THEREOF**

(30) Priority: 28.08.2018 CN 201810987138
(71) Applicant: Jiangsu Hengrui Medicine Co., Ltd., Lianyungang, Jiangsu 222047 (CN); Shanghai Hengrui Pharmaceutical Co., Ltd., Shanghai 200245 (CN)
(72) Inventor: TIAN, Chenmin, Shanghai 200245 (CN); LI, Hao, Shanghai 200245 (CN); LIU, Xun, Shanghai 200245 (CN); CAO, Zhuoxiao, Shanghai 200245 (CN)
(74) Representative: Potter Clarkson
(86) International application number: PCT/CN2019/102839
(87) International publication number: WO 2020/043095

(57) **Abstract**

Disclosed in the present application are a TIM3 antibody pharmaceutical composition and use thereof. In particular, the pharmaceutical composition comprises a TIM3 antibody or antigen-binding fragment thereof in a buffer. In addition, the pharmaceutical composition further comprises a saccharide and a nonionic surfactant. The pharmaceutical composition of the present invention has good stability.

Sequence Listing

<110>  JIANGSU HENGRUI MEDICINE CO., LTD.; SHANGHAI HENGRUI PHARMACEUTICAL
CO., LTD.

<120>  ANTI-TIM3 ANTIBODY PHARMACEUTICAL COMPOSITION AND USE THEREOF

<130>  GECBP/P76180EP

<140>  EP 19855752.2
<141>  2019-08-27

<150>  PCT/CN2019/102839
<151>  2019-08-27

<150>  CN 201810987138.3
<151>  2018-08-28

<160>  74

<170>  SIPOSequenceListing 1.0

<210>  1
<211>  430
<212>  PRT
<213>  Artificial Sequence

<220>
<221>  DOMAIN
<223>  Amino acid sequence for Fusion protein of TIM-3 extracellular
       domain and hIgG1 Fc

<400>  1
Met Glu Phe Gly Leu Ser Trp Leu Phe Leu Val Ala Ile Leu Lys Gly
1               5                   10                  15
Val Gln Cys Ser Glu Val Glu Tyr Arg Ala Glu Val Gly Gln Asn Ala
            20                  25                  30
Tyr Leu Pro Cys Phe Tyr Thr Pro Ala Ala Pro Gly Asn Leu Val Pro
        35                  40                  45
Val Cys Trp Gly Lys Gly Ala Cys Pro Val Phe Glu Cys Gly Asn Val
    50                  55                  60
Val Leu Arg Thr Asp Glu Arg Asp Val Asn Tyr Trp Thr Ser Arg Tyr
65                  70                  75                  80
Trp Leu Asn Gly Asp Phe Arg Lys Gly Asp Val Ser Leu Thr Ile Glu
                85                  90                  95
Asn Val Thr Leu Ala Asp Ser Gly Ile Tyr Cys Cys Arg Ile Gln Ile
            100                 105                 110
Pro Gly Ile Met Asn Asp Glu Lys Phe Asn Leu Lys Leu Val Ile Lys
        115                 120                 125
Pro Ala Lys Val Thr Pro Ala Pro Thr Arg Gln Arg Asp Phe Thr Ala
    130                 135                 140
Ala Phe Pro Arg Met Leu Thr Thr Arg Gly His Gly Pro Ala Glu Thr
145                 150                 155                 160
Gln Thr Leu Gly Ser Leu Pro Asp Ile Asn Leu Thr Gln Ile Ser Thr
                165                 170                 175
Leu Ala Asn Glu Leu Arg Asp Ser Arg Leu Ala Asn Asp Leu Arg Asp
            180                 185                 190
Ser Gly Ala Thr Ile Arg Glu Pro Lys Ser Ser Asp Lys Thr His Thr
        195                 200                 205
Cys Pro Pro Cys Pro Ala Pro Glu Leu Leu Gly Gly Pro Ser Val Phe
    210                 215                 220
Leu Phe Pro Pro Lys Pro Lys Asp Thr Leu Met Ile Ser Arg Thr Pro

225                 230                 235                 240
Glu Val Thr Cys Val Val Val Asp Val Ser His Glu Asp Pro Glu Val
                245                 250                 255
Lys Phe Asn Trp Tyr Val Asp Gly Val Glu Val His Asn Ala Lys Thr
            260                 265                 270
Lys Pro Arg Glu Glu Gln Tyr Asn Ser Thr Tyr Arg Val Val Ser Val
        275                 280                 285
Leu Thr Val Leu His Gln Asp Trp Leu Asn Gly Lys Glu Tyr Lys Cys
    290                 295                 300
Lys Val Ser Asn Lys Ala Leu Pro Ala Pro Ile Glu Lys Thr Ile Ser
305                 310                 315                 320
Lys Ala Lys Gly Gln Pro Arg Glu Pro Gln Val Tyr Thr Leu Pro Pro
                325                 330                 335
Ser Arg Asp Glu Leu Thr Lys Asn Gln Val Ser Leu Thr Cys Leu Val
            340                 345                 350
Lys Gly Phe Tyr Pro Ser Asp Ile Ala Val Glu Trp Glu Ser Asn Gly
        355                 360                 365
Gln Pro Glu Asn Asn Tyr Lys Thr Thr Pro Pro Val Leu Asp Ser Asp
    370                 375                 380
Gly Ser Phe Phe Leu Tyr Ser Lys Leu Thr Val Asp Lys Ser Arg Trp
385                 390                 395                 400
Gln Gln Gly Asn Val Phe Ser Cys Ser Val Met His Glu Ala Leu His
                405                 410                 415
Asn His Tyr Thr Gln Lys Ser Leu Ser Leu Ser Pro Gly Lys
            420                 425                 430

<210>  2
<211>  215
<212>  PRT
<213>  Artificial Sequence

<220>
<221>  DOMAIN
<223>  Amino acid sequence for TIM-3 extracellular domain with
       Flag-tag and His-tag


<400>  2
Met Glu Phe Gly Leu Ser Trp Leu Phe Leu Val Ala Ile Leu Lys Gly
1               5                   10                  15
Val Gln Cys Ser Glu Val Glu Tyr Arg Ala Glu Val Gly Gln Asn Ala
            20                  25                  30
Tyr Leu Pro Cys Phe Tyr Thr Pro Ala Ala Pro Gly Asn Leu Val Pro
        35                  40                  45
Val Cys Trp Gly Lys Gly Ala Cys Pro Val Phe Glu Cys Gly Asn Val
    50                  55                  60
Val Leu Arg Thr Asp Glu Arg Asp Val Asn Tyr Trp Thr Ser Arg Tyr
65                  70                  75                  80
Trp Leu Asn Gly Asp Phe Arg Lys Gly Asp Val Ser Leu Thr Ile Glu
                85                  90                  95
Asn Val Thr Leu Ala Asp Ser Gly Ile Tyr Cys Cys Arg Ile Gln Ile
            100                 105                 110
Pro Gly Ile Met Asn Asp Glu Lys Phe Asn Leu Lys Leu Val Ile Lys
        115                 120                 125
Pro Ala Lys Val Thr Pro Ala Pro Thr Arg Gln Arg Asp Phe Thr Ala
    130                 135                 140
Ala Phe Pro Arg Met Leu Thr Thr Arg Gly His Gly Pro Ala Glu Thr
145                 150                 155                 160
Gln Thr Leu Gly Ser Leu Pro Asp Ile Asn Leu Thr Gln Ile Ser Thr
                165                 170                 175
Leu Ala Asn Glu Leu Arg Asp Ser Arg Leu Ala Asn Asp Leu Arg Asp
            180                 185                 190
Ser Gly Ala Thr Ile Arg Gly Ser Ser Asp Tyr Lys Asp Asp Asp Asp

195                 200                 205
Lys His His His His His His
    210                 215

<210>  3
<211>  301
<212>  PRT
<213>  Artificial Sequence

<220>
<221>  DOMAIN
<223>  Amino acid sequence for full-length human TIM-3


<400>  3
Met Phe Ser His Leu Pro Phe Asp Cys Val Leu Leu Leu Leu Leu Leu
1               5                   10                  15
Leu Leu Thr Arg Ser Ser Glu Val Glu Tyr Arg Ala Glu Val Gly Gln
            20                  25                  30
Asn Ala Tyr Leu Pro Cys Phe Tyr Thr Pro Ala Ala Pro Gly Asn Leu
        35                  40                  45
Val Pro Val Cys Trp Gly Lys Gly Ala Cys Pro Val Phe Glu Cys Gly
    50                  55                  60
Asn Val Val Leu Arg Thr Asp Glu Arg Asp Val Asn Tyr Trp Thr Ser
65                  70                  75                  80
Arg Tyr Trp Leu Asn Gly Asp Phe Arg Lys Gly Asp Val Ser Leu Thr
                85                  90                  95
Ile Glu Asn Val Thr Leu Ala Asp Ser Gly Ile Tyr Cys Cys Arg Ile
            100                 105                 110
Gln Ile Pro Gly Ile Met Asn Asp Glu Lys Phe Asn Leu Lys Leu Val
        115                 120                 125
Ile Lys Pro Ala Lys Val Thr Pro Ala Pro Thr Arg Gln Arg Asp Phe
    130                 135                 140
Thr Ala Ala Phe Pro Arg Met Leu Thr Thr Arg Gly His Gly Pro Ala
145                 150                 155                 160
Glu Thr Gln Thr Leu Gly Ser Leu Pro Asp Ile Asn Leu Thr Gln Ile
                165                 170                 175
Ser Thr Leu Ala Asn Glu Leu Arg Asp Ser Arg Leu Ala Asn Asp Leu
            180                 185                 190
Arg Asp Ser Gly Ala Thr Ile Arg Ile Gly Ile Tyr Ile Gly Ala Gly
        195                 200                 205
Ile Cys Ala Gly Leu Ala Leu Ala Leu Ile Phe Gly Ala Leu Ile Phe
    210                 215                 220
Lys Trp Tyr Ser His Ser Lys Glu Lys Ile Gln Asn Leu Ser Leu Ile
225                 230                 235                 240
Ser Leu Ala Asn Leu Pro Pro Ser Gly Leu Ala Asn Ala Val Ala Glu
                245                 250                 255
Gly Ile Arg Ser Glu Glu Asn Ile Tyr Thr Ile Glu Glu Asn Val Tyr
            260                 265                 270
Glu Val Glu Glu Pro Asn Glu Tyr Tyr Cys Tyr Val Ser Ser Arg Gln
        275                 280                 285
Gln Pro Ser Gln Pro Leu Gly Cys Arg Phe Ala Met Pro
    290                 295                 300

<210>  4
<211>  121
<212>  PRT
<213>  Artificial Sequence

<220>
<221>  DOMAIN
<223>  Amino acid sequence for mAb-1701 heavy chain variable region

<400>  4
Glu Val Gln Leu Gln Gln Ser Gly Pro Glu Leu Val Lys Pro Gly Ala
1               5                   10                  15
Ser Val Lys Ile Ser Cys Lys Ala Ser Gly Tyr Thr Phe Thr Asp Tyr
            20                  25                  30
Tyr Met Asn Trp Val Lys Gln Ser His Gly Lys Ser Leu Glu Trp Ile
        35                  40                  45
Ala Asp Ile Ile Pro Asn Asn Gly Gly Ser Lys Tyr Asn Gln Lys Phe
    50                  55                  60
Lys Asp Lys Ala Thr Leu Thr Val Asp Lys Ser Ser Ser Thr Ala Tyr
65                  70                  75                  80
Met Glu Leu Arg Ser Leu Thr Ser Glu Asp Ser Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Ser Val Ser Ala
        115                 120

<210>  5
<211>  107
<212>  PRT
<213>  Artificial Sequence

<220>
<221>  DOMAIN
<223>  Amino acid sequence for mAb-1701 light chain variable region


<400>  5
Asp Ile Gln Met Thr Gln Ser Pro Ala Ser Gln Ser Ala Ser Leu Gly
1               5                   10                  15
Glu Ser Val Thr Ile Thr Cys Leu Ala Ser Gln Pro Ile Gly Ile Trp
            20                  25                  30
Leu Ala Trp Tyr Gln Gln Lys Pro Gly Lys Ser Pro Gln Leu Leu Ile
        35                  40                  45
Tyr Ala Ala Thr Ser Leu Ala Asp Gly Val Pro Ser Arg Phe Ser Gly
    50                  55                  60
Ser Gly Ser Gly Thr Lys Phe Ser Phe Lys Ile Ser Ser Leu Gln Ala
65                  70                  75                  80
Glu Asp Phe Val Ser Tyr Tyr Cys Gln Gln Leu Tyr Ser Ser Pro Trp
                85                  90                  95
Thr Phe Gly Gly Gly Thr Lys Leu Glu Ile Lys
            100                 105

<210>  6
<211>  122
<212>  PRT
<213>  Artificial Sequence

<220>
<221>  DOMAIN
<223>  Amino acid sequence for mAb-1799 heavy chain variable region


<400>  6
Glu Val Lys Leu Val Glu Ser Glu Gly Gly Leu Val Gln Pro Gly Ser
1               5                   10                  15
Ser Met Lys Leu Ser Cys Thr Ala Ser Gly Phe Thr Phe Ser Asp Tyr
            20                  25                  30
Tyr Met Ala Trp Val Arg Gln Val Pro Glu Lys Gly Leu Glu Trp Val
        35                  40                  45
Ala Asn Ile Asn Tyr Asp Gly Ser Ser Thr Tyr Tyr Leu Asp Ser Leu

50                  55                  60
Lys Ser Arg Phe Ile Ile Ser Arg Asp Asn Ala Lys Asn Ile Leu Tyr
65                  70                  75                  80
Leu Gln Met Asn Ser Leu Lys Ser Asp Asp Thr Ala Thr Tyr Tyr Cys
                85                  90                  95
Ala Arg Asp Val Gly Tyr Tyr Gly Gly Asn Tyr Gly Phe Ala Tyr Trp
            100                 105                 110
Gly Gln Gly Thr Leu Val Thr Val Ser Ala
        115                 120

<210>  7
<211>  107
<212>  PRT
<213>  Artificial Sequence

<220>
<221>  DOMAIN
<223>  Amino acid sequence for mAb-1799 light chain variable region


<400>  7
Asp Ile Gln Met Thr Gln Ser Pro Ala Ser Leu Ser Ala Ser Val Gly
1               5                   10                  15
Glu Thr Val Thr Ile Thr Cys Arg Ala Ser Asp Asn Ile Tyr Ser Tyr
            20                  25                  30
Leu Ala Trp Tyr Gln Gln Lys Gln Gly Lys Ser Pro Gln Leu Leu Val
        35                  40                  45
Tyr Asn Ala Lys Thr Leu Ala Glu Gly Val Pro Ser Arg Phe Ser Gly
    50                  55                  60
Ser Gly Ser Gly Thr Gln Phe Ser Leu Lys Ile Asn Ser Leu Gln Pro
65                  70                  75                  80
Glu Asp Phe Gly Ser Tyr Tyr Cys Gln Gln His Tyr Gly Ser Pro Leu
                85                  90                  95
Thr Phe Gly Ala Gly Thr Lys Leu Glu Leu Lys
            100                 105

<210>  8
<211>  5
<212>  PRT
<213>  Artificial Sequence

<220>
<221>  DOMAIN
<223>  Amino acid sequence for mAb-1701 heavy chain HCDR1


<400>  8
Asp Tyr Tyr Met Asn
1               5

<210>  9
<211>  17
<212>  PRT
<213>  Artificial Sequence

<220>
<221>  DOMAIN
<223>  Amino acid sequence for mAb-1701 heavy chain HCDR2


<400>  9
Asp Ile Ile Pro Asn Asn Gly Gly Ser Lys Tyr Asn Gln Lys Phe Lys
1               5                   10                  15

Asp

<210>  10
<211>  12
<212>  PRT
<213>  Artificial Sequence

<220>
<221>  DOMAIN
<223>  Amino acid sequence for mAb-1701 heavy chain HCDR3

<400>  10
Trp Gly Tyr Gly Ser Ser Tyr Arg Trp Phe Asp Tyr
1               5                   10

<210>  11
<211>  11
<212>  PRT
<213>  Artificial Sequence

<220>
<221>  DOMAIN
<223>  Amino acid sequence for mAb-1701 light chain LCDR1

<400>  11
Leu Ala Ser Gln Pro Ile Gly Ile Trp Leu Ala
1               5                   10

<210>  12
<211>  7
<212>  PRT
<213>  Artificial Sequence

<220>
<221>  DOMAIN
<223>  Amino acid sequence for mAb-1701 light chain LCDR2

<400>  12
Ala Ala Thr Ser Leu Ala Asp
1               5

<210>  13
<211>  9
<212>  PRT
<213>  Artificial Sequence

<220>
<221>  DOMAIN
<223>  Amino acid sequence for mAb-1701 light chain LCDR3

<400>  13
Gln Gln Leu Tyr Ser Ser Pro Trp Thr
1               5

<210>  14
<211>  5
<212>  PRT
<213>  Artificial Sequence

<220>
<221>  DOMAIN
<223>  Amino acid sequence for mAb-1799 heavy chain HCDR1

<400>  14
Asp Tyr Tyr Met Ala
1               5

<210>  15
<211>  17
<212>  PRT
<213>  Artificial Sequence

<220>
<221>  DOMAIN
<223>  Amino acid sequence for mAb-1799 heavy chain HCDR2

<400>  15
Asn Ile Asn Tyr Asp Gly Ser Ser Thr Tyr Tyr Leu Asp Ser Leu Lys
1               5                   10                  15
Ser

<210>  16
<211>  13
<212>  PRT
<213>  Artificial Sequence

<220>
<221>  DOMAIN
<223>  Amino acid sequence for mAb-1799 heavy chain HCDR3

<400>  16
Asp Val Gly Tyr Tyr Gly Gly Asn Tyr Gly Phe Ala Tyr
1               5                   10

<210>  17
<211>  11
<212>  PRT
<213>  Artificial Sequence

<220>
<221>  DOMAIN
<223>  Amino acid sequence for mAb-1799 light chain LCDR1

<400>  17
Arg Ala Ser Asp Asn Ile Tyr Ser Tyr Leu Ala
1               5                   10

<210>  18
<211>  7
<212>  PRT
<213>  Artificial Sequence

<220>
<221>  DOMAIN
<223>  Amino acid sequence for mAb-1799 light chain LCDR2

<400>  18
Asn Ala Lys Thr Leu Ala Glu
1               5

<210>  19
<211>  9
<212>  PRT
<213>  Artificial Sequence

<220>
<221>  DOMAIN
<223>  Amino acid sequence for mAb-1799 light chain LCDR3


<400>  19
Gln Gln His Tyr Gly Ser Pro Leu Thr
1               5

<210>  20
<211>  121
<212>  PRT
<213>  Artificial Sequence

<220>
<221>  DOMAIN
<223>  Amino acid sequence for h1701 heavy chain variable region

<400>  20
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn Asn Gly Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  21
<211>  107
<212>  PRT
<213>  Artificial Sequence

<220>
<221>  DOMAIN
<223>  Amino acid sequence for h1701 light chain variable region


<400>  21
Asp Ile Gln Met Thr Gln Ser Pro Ser Ser Leu Ser Ala Ser Val Gly
1               5                   10                  15
Asp Arg Val Thr Ile Thr Cys Leu Ala Ser Gln Pro Ile Gly Ile Trp
            20                  25                  30
Leu Ala Trp Tyr Gln Gln Lys Pro Gly Lys Ala Pro Lys Leu Leu Ile
        35                  40                  45

Tyr Ala Ala Thr Ser Leu Ala Asp Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Leu Tyr Ser Ser Pro Trp
                85                  90                  95
Thr Phe Gly Gly Gly Thr Lys Val Glu Ile Lys
            100                 105

<210>  22
<211>  121
<212>  PRT
<213>  Artificial Sequence

<220>
<221>  DOMAIN
<223>  Amino acid sequence for humanized antibody h1701-005/h1701-006
       heavy chain variable region


<400>  22
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn Asn Gly Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  23
<211>  121
<212>  PRT
<213>  Artificial Sequence

<220>
<221>  DOMAIN
<223>  Amino acid sequence for humanized antibody h1701-007/h1701-008
       heavy chain variable region


<400>  23
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Ile
        35                  40                  45
Gly Asp Ile Ile Pro Asn Asn Gly Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Trp Gly Tyr Gly Ser Ser Tyr Arg Trp Phe Asp Tyr Trp Gly

                100                 105                 110
Gln Gly Thr Leu Val Thr Val Ser Ser
        115                 120

<210>  24
<211>  121
<212>  PRT
<213>  Artificial Sequence

<220>
<221>  DOMAIN
<223>  Amino acid sequence for humanized antibody h1701-009/h1701-010
       heavy chain variable region


<400>  24
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn Asn Gly Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  25
<211>  121
<212>  PRT
<213>  Artificial Sequence

<220>
<221>  DOMAIN
<223>  Amino acid sequence for humanized antibody h1701-011/h1701-012
       heavy chain variable region


<400>  25
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Ile
        35                  40                  45
Gly Asp Ile Ile Pro Asn Asn Gly Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  26

<211>  121
<212>  PRT
<213>  Artificial Sequence

<220>
<221>  DOMAIN
<223>  Amino acid sequence for humanized antibody h1701-013/h1701-014
       heavy chain variable region


<400>  26
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Lys Gln Ala Pro Gly Gln Gly Leu Glu Trp Ile
        35                  40                  45
Gly Asp Ile Ile Pro Asn Asn Gly Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Glu Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  27
<211>  121
<212>  PRT
<213>  Artificial Sequence

<220>
<221>  DOMAIN
<223>  Amino acid sequence for humanized antibody h1701-015/h1701-016
       heavy chain variable region


<400>  27
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Ile
        35                  40                  45
Ala Asp Ile Ile Pro Asn Asn Gly Gly Ser Lys Tyr Asn Gln Lys Phe
    50                  55                  60
Lys Asp Arg Ala Thr Leu Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  28
<211>  121
<212>  PRT
<213>  Artificial Sequence

<220>

<221>  DOMAIN
<223>  Amino acid sequence for humanized antibody h1701-017/h1701-018
       heavy chain variable region

<400>  28
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Lys Gln Ala Pro Gly Gln Gly Leu Glu Trp Ile
        35                  40                  45
Ala Asp Ile Ile Pro Asn Asn Gly Gly Ser Lys Tyr Asn Gln Lys Phe
    50                  55                  60
Lys Asp Arg Ala Thr Leu Thr Thr Asp Thr Ser Thr Ser Thr Ala Tyr
65                  70                  75                  80
Met Glu Leu Arg Ser Leu Arg Ser Glu Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  29
<211>  107
<212>  PRT
<213>  Artificial Sequence

<220>
<221>  DOMAIN
<223>  Amino acid sequence for humanized antibody
h1701-005/h1701-007/h1701-009
       /h1701-011/h1701-013/h1701-015/h1701-017 light chain variable region

<400>  29
Asp Ile Gln Met Thr Gln Ser Pro Ser Ser Leu Ser Ala Ser Val Gly
1               5                   10                  15
Asp Arg Val Thr Ile Thr Cys Leu Ala Ser Gln Pro Ile Gly Ile Trp
            20                  25                  30
Leu Ala Trp Tyr Gln Gln Lys Pro Gly Lys Ala Pro Lys Leu Leu Ile
        35                  40                  45
Tyr Ala Ala Thr Ser Leu Ala Asp Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Leu Tyr Ser Ser Pro Trp
                85                  90                  95
Thr Phe Gly Gly Gly Thr Lys Val Glu Ile Lys
            100                 105

<210>  30
<211>  107
<212>  PRT
<213>  Artificial Sequence

<220>
<221>  DOMAIN
<223>  Amino acid sequence for humanized antibody
h1701-006/h1701-008/h1701-010
       /h1701-012/h1701-014/h1701-016/h1701-018 light chain variable region

<400>  30
Asp Ile Gln Met Thr Gln Ser Pro Ser Ser Leu Ser Ala Ser Val Gly
1               5                   10                  15
Asp Arg Val Thr Ile Thr Cys Leu Ala Ser Gln Pro Ile Gly Ile Trp
            20                  25                  30
Leu Ala Trp Tyr Gln Gln Lys Pro Gly Lys Ser Pro Lys Leu Leu Ile
        35                  40                  45
Tyr Ala Ala Thr Ser Leu Ala Asp Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Leu Tyr Ser Ser Pro Trp
                85                  90                  95
Thr Phe Gly Gly Gly Thr Lys Val Glu Ile Lys
            100                 105

<210>  31
<211>  122
<212>  PRT
<213>  Artificial Sequence

<220>
<221>  DOMAIN
<223>  Amino acid sequence for h1799 heavy chain variable region


<400>  31
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Tyr
            20                  25                  30
Tyr Met Ala Trp Val Arg Gln Ala Pro Gly Lys Gly Leu Glu Trp Val
        35                  40                  45
Ala Asn Ile Asn Tyr Asp Gly Ser Ser Thr Tyr Tyr Leu Asp Ser Leu
    50                  55                  60
Lys Ser Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Val Gly Tyr Tyr Gly Gly Asn Tyr Gly Phe Ala Tyr Trp
            100                 105                 110
Gly Gln Gly Thr Leu Val Thr Val Ser Ser
        115                 120

<210>  32
<211>  107
<212>  PRT
<213>  Artificial Sequence

<220>
<221>  DOMAIN
<223>  Amino acid sequence for h1799 light chain variable region


<400>  32
Asp Ile Gln Met Thr Gln Ser Pro Ser Ser Leu Ser Ala Ser Val Gly
1               5                   10                  15
Asp Arg Val Thr Ile Thr Cys Arg Ala Ser Asp Asn Ile Tyr Ser Tyr
            20                  25                  30
Leu Ala Trp Tyr Gln Gln Lys Pro Gly Lys Ala Pro Lys Leu Leu Ile
        35                  40                  45
Tyr Asn Ala Lys Thr Leu Ala Glu Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln His Tyr Gly Ser Pro Leu
                85                  90                  95
Thr Phe Gly Gln Gly Thr Lys Leu Glu Ile Lys
            100                 105

<210>  33
<211>  122
<212>  PRT
<213>  Artificial Sequence

<220>
<221>  DOMAIN
<223>  Amino acid sequence for humanized antibody h1799-005/h1799-006
       /h1799-007/h1799-008/h1799-009 heavy chain variable region


<400>  33
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Tyr
            20                  25                  30
Tyr Met Ala Trp Val Arg Gln Ala Pro Gly Lys Gly Leu Glu Trp Val
        35                  40                  45
Ala Asn Ile Asn Tyr Asp Gly Ser Ser Thr Tyr Tyr Leu Asp Ser Leu
    50                  55                  60
Lys Ser Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Val Gly Tyr Tyr Gly Gly Asn Tyr Gly Phe Ala Tyr Trp
            100                 105                 110
Gly Gln Gly Thr Leu Val Thr Val Ser Ser
        115                 120

<210>  34
<211>  122
<212>  PRT
<213>  Artificial Sequence

<220>
<221>  DOMAIN
<223>  Amino acid sequence for humanized antibody h1799-010/h1799-011/
       h1799-012/h1799-013/h1799-014 heavy chain variable region


<400>  34
Glu Val Lys Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Tyr
            20                  25                  30
Tyr Met Ala Trp Val Arg Gln Ala Pro Gly Lys Gly Leu Glu Trp Val
        35                  40                  45
Ala Asn Ile Asn Tyr Asp Gly Ser Ser Thr Tyr Tyr Leu Asp Ser Leu
    50                  55                  60
Lys Ser Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Val Gly Tyr Tyr Gly Gly Asn Tyr Gly Phe Ala Tyr Trp
            100                 105                 110
Gly Gln Gly Thr Leu Val Thr Val Ser Ser

115                 120

<210>  35
<211>  122
<212>  PRT
<213>  Artificial Sequence

<220>
<221>  DOMAIN
<223>  Amino acid sequence for humanized antibody h1799-015/h1799-016/
       h1799-017/h1799-018/h1799-019 heavy chain variable region


<400>  35
Glu Val Lys Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Tyr
            20                  25                  30
Tyr Met Ala Trp Val Arg Gln Ala Pro Gly Lys Gly Leu Glu Trp Val
        35                  40                  45
Ala Asn Ile Asn Tyr Asp Gly Ser Ser Thr Tyr Tyr Leu Asp Ser Leu
    50                  55                  60
Lys Ser Arg Phe Thr Ile Ser Arg Asp Asn Ala Lys Asn Ser Leu Tyr
65                  70                  75                  80
Leu Gln Met Asn Ser Leu Lys Ala Glu Asp Thr Ala Val Tyr Tyr Cys
                85                  90                  95
Ala Arg Asp Val Gly Tyr Tyr Gly Gly Asn Tyr Gly Phe Ala Tyr Trp
            100                 105                 110
Gly Gln Gly Thr Leu Val Thr Val Ser Ser
        115                 120

<210>  36
<211>  107
<212>  PRT
<213>  Artificial Sequence

<220>
<221>  DOMAIN
<223>  Amino acid sequence for humanized antibody h1799-005/h1799-010
       /h1799-015 light chain variable region


<400>  36
Asp Ile Gln Met Thr Gln Ser Pro Ser Ser Leu Ser Ala Ser Val Gly
1               5                   10                  15
Asp Arg Val Thr Ile Thr Cys Arg Ala Ser Asp Asn Ile Tyr Ser Tyr
            20                  25                  30
Leu Ala Trp Tyr Gln Gln Lys Pro Gly Lys Ala Pro Lys Leu Leu Ile
        35                  40                  45
Tyr Asn Ala Lys Thr Leu Ala Glu Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln His Tyr Gly Ser Pro Leu
                85                  90                  95
Thr Phe Gly Gln Gly Thr Lys Leu Glu Ile Lys
            100                 105

<210>  37
<211>  107
<212>  PRT
<213>  Artificial Sequence

<220>
<221>  DOMAIN
<223>  Amino acid sequence for humanized antibody h1799-006/h1799-011/
       h1799-016 light chain variable region


<400>  37
Asp Ile Gln Met Thr Gln Ser Pro Ser Ser Leu Ser Ala Ser Val Gly
1               5                   10                  15
Asp Arg Val Thr Ile Thr Cys Arg Ala Ser Asp Asn Ile Tyr Ser Tyr
            20                  25                  30
Leu Ala Trp Tyr Gln Gln Lys Pro Gly Lys Ala Pro Lys Leu Leu Val
        35                  40                  45
Tyr Asn Ala Lys Thr Leu Ala Glu Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln His Tyr Gly Ser Pro Leu
                85                  90                  95
Thr Phe Gly Gln Gly Thr Lys Leu Glu Ile Lys
            100                 105

<210>  38
<211>  107
<212>  PRT
<213>  Artificial Sequence

<220>
<221>  DOMAIN
<223>  Amino acid sequence for humanized antibody h1799-007/h1799-012
       /h1799-017 light chain variable region


<400>  38
Asp Ile Gln Met Thr Gln Ser Pro Ser Ser Leu Ser Ala Ser Val Gly
1               5                   10                  15
Asp Arg Val Thr Ile Thr Cys Arg Ala Ser Asp Asn Ile Tyr Ser Tyr
            20                  25                  30
Leu Ala Trp Tyr Gln Gln Lys Pro Gly Lys Ala Pro Gln Leu Leu Val
        35                  40                  45
Tyr Asn Ala Lys Thr Leu Ala Glu Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln His Tyr Gly Ser Pro Leu
                85                  90                  95
Thr Phe Gly Gln Gly Thr Lys Leu Glu Ile Lys
            100                 105

<210>  39
<211>  107
<212>  PRT
<213>  Artificial Sequence

<220>
<221>  DOMAIN
<223>  Amino acid sequence for humanized antibody h1799-008/h1799-013
       /h1799-018 light chain variable region


<400>  39
Asp Ile Gln Met Thr Gln Ser Pro Ser Ser Leu Ser Ala Ser Val Gly
1               5                   10                  15

Asp Arg Val Thr Ile Thr Cys Arg Ala Ser Asp Asn Ile Tyr Ser Tyr
            20                  25                  30
Leu Ala Trp Tyr Gln Gln Lys Pro Gly Lys Ser Pro Gln Leu Leu Val
        35                  40                  45
Tyr Asn Ala Lys Thr Leu Ala Glu Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln His Tyr Gly Ser Pro Leu
                85                  90                  95
Thr Phe Gly Gln Gly Thr Lys Leu Glu Ile Lys
            100                 105

<210>  40
<211>  107
<212>  PRT
<213>  Artificial Sequence

<220>
<221>  DOMAIN
<223>  Amino acid sequence for humanized antibody h1799-009/h1799-014
       /h1799-019 light chain variable region


<400>  40
Asp Ile Gln Met Thr Gln Ser Pro Ser Ser Leu Ser Ala Ser Val Gly
1               5                   10                  15
Asp Arg Val Thr Ile Thr Cys Arg Ala Ser Asp Asn Ile Tyr Ser Tyr
            20                  25                  30
Leu Ala Trp Tyr Gln Gln Lys Pro Gly Lys Ser Pro Gln Leu Leu Val
        35                  40                  45
Tyr Asn Ala Lys Thr Leu Ala Glu Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Ser Tyr Tyr Cys Gln Gln His Tyr Gly Ser Pro Leu
                85                  90                  95
Thr Phe Gly Gln Gly Thr Lys Leu Glu Ile Lys
            100                 105

<210>  41
<211>  327
<212>  PRT
<213>  Artificial Sequence

<220>
<221>  DOMAIN
<223>  Amino acid sequence for human IgG4 heavy chain constant region with
mutant Fc


<400>  41
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys

                85                  90                  95
Arg Val Glu Ser Lys Tyr Gly Pro Pro Cys Pro Pro Cys Pro Ala Pro
            100                 105                 110
Glu Phe Leu Gly Gly Pro Ser Val Phe Leu Phe Pro Pro Lys Pro Lys
        115                 120                 125
Asp Thr Leu Met Ile Ser Arg Thr Pro Glu Val Thr Cys Val Val Val
    130                 135                 140
Asp Val Ser Gln Glu Asp Pro Glu Val Gln Phe Asn Trp Tyr Val Asp
145                 150                 155                 160
Gly Val Glu Val His Asn Ala Lys Thr Lys Pro Arg Glu Glu Gln Phe
                165                 170                 175
Asn Ser Thr Tyr Arg Val Val Ser Val Leu Thr Val Leu His Gln Asp
            180                 185                 190
Trp Leu Asn Gly Lys Glu Tyr Lys Cys Lys Val Ser Asn Lys Gly Leu
        195                 200                 205
Pro Ser Ser Ile Glu Lys Thr Ile Ser Lys Ala Lys Gly Gln Pro Arg
    210                 215                 220
Glu Pro Gln Val Tyr Thr Leu Pro Pro Ser Gln Glu Glu Met Thr Lys
225                 230                 235                 240
Asn Gln Val Ser Leu Thr Cys Leu Val Lys Gly Phe Tyr Pro Ser Asp
                245                 250                 255
Ile Ala Val Glu Trp Glu Ser Asn Gly Gln Pro Glu Asn Asn Tyr Lys
            260                 265                 270
Thr Thr Pro Pro Val Leu Asp Ser Asp Gly Ser Phe Phe Leu Tyr Ser
        275                 280                 285
Arg Leu Thr Val Asp Lys Ser Arg Trp Gln Glu Gly Asn Val Phe Ser
    290                 295                 300
Cys Ser Val Met His Glu Ala Leu His Asn His Tyr Thr Gln Lys Ser
305                 310                 315                 320
Leu Ser Leu Ser Leu Gly Lys
                325

<210>  42
<211>  107
<212>  PRT
<213>  Artificial Sequence

<220>
<221>  DOMAIN
<223>  Amino acid sequence for human kappa light chain constant region

<400>  42

<210>  43
<211>  17
<212>  PRT
<213>  Artificial Sequence

<220>

<221>  DOMAIN
<223>  humanized antibody h1701 HCDR2 mutant sequence

<220>
<221>  DOMAIN
<222>  (5)..(5)
<223>  Xaa could be Asn, Leu, Val, Met or Glu

<220>
<221>  DOMAIN
<222>  (6)..(6)
<223>  Xaa could be Asn, Glu, Met, His, Lys, Leu, Ala or Val

<220>
<221>  DOMAIN
<222>  (7)..(7)
<223>  Xaa could be Gly or Ala

<400>  43
Asp Ile Ile Pro Xaa Xaa Xaa Gly Ser Lys Tyr Asn Gln Lys Phe Lys
1               5                   10                  15
Asp

<210>  44
<211>  121
<212>  PRT
<213>  Artificial Sequence

<220>
<221>  DOMAIN
<223>  Amino acid sequence for antibody heavy chain variable region of
       h1701-005/h1701-006 comprising mutant heavy chain HCDR2

<220>
<221>  DOMAIN
<222>  (54)..(54)
<223>  Xaa could be Asn, Leu, Val, Met or Glu

<220>
<221>  DOMAIN
<222>  (55)..(55)
<223>  Xaa could be Asn, Glu, Met, His, Lys, Leu, Ala or Val

<220>
<221>  DOMAIN
<222>  (56)..(56)
<223>  Xaa could be Gly or Ala

<400>  44
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met

35                  40                  45
Gly Asp Ile Ile Pro Xaa Xaa Xaa Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  45
<211>  121
<212>  PRT
<213>  Artificial Sequence

<220>
<221>  DOMAIN
<223>  Amino acid sequence for antibody heavy chain variable region of
       h1701-007/h1701-008 comprising mutant heavy chain HCDR2

<220>
<221>  DOMAIN
<222>  (54)..(54)
<223>  Xaa could be Asn, Leu, Val, Met or Glu


<220>
<221>  DOMAIN
<222>  (55)..(55)
<223>  Xaa could be Asn, Glu, Met, His, Lys, Leu, Ala or Val


<220>
<221>  DOMAIN
<222>  (56)..(56)
<223>  Xaa could be Gly or Ala


<400>  45
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Ile
        35                  40                  45
Gly Asp Ile Ile Pro Xaa Xaa Xaa Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  46
<211>  121
<212>  PRT
<213>  Artificial Sequence

<220>
<221>  DOMAIN
<223>  Amino acid sequence for antibody heavy chain variable region of
      h1701-009/h1701-010 comprising mutant heavy chain HCDR2

<220>
<221>  DOMAIN
<222>  (54)..(54)
<223>  Xaa could be Asn, Leu, Val, Met or Glu


<220>
<221>  DOMAIN
<222>  (55)..(55)
<223>  Xaa could be Asn, Glu, Met, His, Lys, Leu, Ala or Val


<220>
<221>  DOMAIN
<222>  (56)..(56)
<223>  Xaa could be Gly or Ala


<400>  46
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Xaa Xaa Xaa Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  47
<211>  121
<212>  PRT
<213>  Artificial Sequence

<220>
<221>  DOMAIN
<223>  Amino acid sequence for antibody heavy chain variable region of
      h1701-011/h1701-012 comprising mutant heavy chain HCDR2

<220>
<221>  DOMAIN
<222>  (54)..(54)
<223>  Xaa could be Asn, Leu, Val, Met or Glu


<220>
<221>  DOMAIN
<222>  (55)..(55)
<223>  Xaa could be Asn, Glu, Met, His, Lys, Leu, Ala or Val

<220>
<221>  DOMAIN
<222>  (56)..(56)
<223>  Xaa could be Gly or Ala

<400>  47
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Ile
        35                  40                  45
Gly Asp Ile Ile Pro Xaa Xaa Xaa Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  48
<211>  121
<212>  PRT
<213>  Artificial Sequence

<220>
<221>  DOMAIN
<223>  Amino acid sequence for antibody heavy chain variable region of
       h1701-013/h1701-014 comprising mutant heavy chain HCDR2

<220>
<221>  DOMAIN
<222>  (54)..(54)
<223>  Xaa could be Asn, Leu, Val, Met or Glu

<220>
<221>  DOMAIN
<222>  (55)..(55)
<223>  Xaa could be Asn, Glu, Met, His, Lys, Leu, Ala or Val

<220>
<221>  DOMAIN
<222>  (56)..(56)
<223>  Xaa could be Gly or Ala

<400>  48
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Lys Gln Ala Pro Gly Gln Gly Leu Glu Trp Ile
        35                  40                  45
Gly Asp Ile Ile Pro Xaa Xaa Xaa Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80

Met Glu Leu Arg Ser Leu Arg Ser Glu Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  49
<211>  121
<212>  PRT
<213>  Artificial Sequence

<220>
<221>  DOMAIN
<223>  Amino acid sequence for antibody heavy chain variable region of
       h1701-015/h1701-016 comprising mutant heavy chain HCDR2

<220>
<221>  DOMAIN
<222>  (54)..(54)
<223>  Xaa could be Asn, Leu, Val, Met or Glu


<220>
<221>  DOMAIN
<222>  (55)..(55)
<223>  Xaa could be Asn, Glu, Met, His, Lys, Leu, Ala or Val


<220>
<221>  DOMAIN
<222>  (56)..(56)
<223>  Xaa could be Gly or Ala


<400>  49
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Ile
        35                  40                  45
Ala Asp Ile Ile Pro Xaa Xaa Xaa Gly Ser Lys Tyr Asn Gln Lys Phe
    50                  55                  60
Lys Asp Arg Ala Thr Leu Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  50
<211>  121
<212>  PRT
<213>  Artificial Sequence

<220>
<221>  DOMAIN
<223>  Amino acid sequence for antibody heavy chain variable region of
       h1701-017/h1701-018 comprising mutant heavy chain HCDR2

<220>
<221>  DOMAIN
<222>  (54)..(54)
<223>  Xaa could be Asn, Leu, Val, Met or Glu


<220>
<221>  DOMAIN
<222>  (55)..(55)
<223>  Xaa could be Asn, Glu, Met, His, Lys, Leu, Ala or Val


<220>
<221>  DOMAIN
<222>  (56)..(56)
<223>  Xaa could be Gly or Ala


<400>  50
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Lys Gln Ala Pro Gly Gln Gly Leu Glu Trp Ile
        35                  40                  45
Ala Asp Ile Ile Pro Xaa Xaa Xaa Gly Ser Lys Tyr Asn Gln Lys Phe
    50                  55                  60
Lys Asp Arg Ala Thr Leu Thr Thr Asp Thr Ser Thr Ser Thr Ala Tyr
65                  70                  75                  80
Met Glu Leu Arg Ser Leu Arg Ser Glu Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  51
<211>  121
<212>  PRT
<213>  Artificial Sequence

<220>
<221>  DOMAIN
<223>  Amino acid sequence for heavy chain variable region of h1701-009 NLG
mutant antibody


<400>  51
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn Leu Gly Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser

115                 120

<210>  52
<211>  121
<212>  PRT
<213>  Artificial Sequence

<220>
<221>  DOMAIN
<223>  Amino acid sequence for heavy chain variable region of h1701-009 NVG
mutant antibody

<400>  52
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn Val Gly Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  53
<211>  121
<212>  PRT
<213>  Artificial Sequence

<220>
<221>  DOMAIN
<223>  Amino acid sequence for heavy chain variable region of h1701-009 NNA
mutant antibody

<400>  53
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn Asn Ala Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  54
<211>  121
<212>  PRT
<213>  Artificial Sequence

<220>
<221>  DOMAIN
<223>  Amino acid sequence for heavy chain variable region of h1701-009 NMA
mutant antibody

<400>  54
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn Met Ala Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  55
<211>  121
<212>  PRT
<213>  Artificial Sequence

<220>
<221>  DOMAIN
<223>  Amino acid sequence for heavy chain variable region of h1701-009 NEA
mutant antibody

<400>  55
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn Glu Ala Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  56
<211>  121
<212>  PRT
<213>  Artificial Sequence

<220>
<221>  DOMAIN
<223>  Amino acid sequence for heavy chain variable region of h1701-009 NHA
mutant antibody

<400>  56
Gln Val Gln Leu Val Gln Ser Gly Ala Glu Val Lys Lys Pro Gly Ala

1                   5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn His Ala Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  57
<211>  121
<212>  PRT
<213>  Artificial Sequence

<220>
<221>  DOMAIN
<223>  Amino acid sequence for heavy chain variable region of h1701-009 NMG
mutant antibody

<400>  57
Gln Val Gln Leu Val Gln Ser Gly Ala Glu Val Lys Lys Pro Gly Ala
1                   5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn Met Gly Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  58
<211>  121
<212>  PRT
<213>  Artificial Sequence

<220>
<221>  DOMAIN
<223>  Amino acid sequence for heavy chain variable region of h1701-009 NEG
mutant antibody

<400>  58
Gln Val Gln Leu Val Gln Ser Gly Ala Glu Val Lys Lys Pro Gly Ala
1                   5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn Glu Gly Gly Ser Lys Tyr Asn Gln Lys Phe
    50                  55                  60

Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  59
<211>  121
<212>  PRT
<213>  Artificial Sequence

<220>
<221>  DOMAIN
<223>  Amino acid sequence for heavy chain variable region of h1701-009 NKG
mutant antibody

<400>  59
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn Lys Gly Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  60
<211>  121
<212>  PRT
<213>  Artificial Sequence

<220>
<221>  DOMAIN
<223>  Amino acid sequence for heavy chain variable region of h1701-009 NAG
mutant antibody

<400>  60
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn Ala Gly Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser

115                 120

<210>  61
<211>  121
<212>  PRT
<213>  Artificial Sequence

<220>
<221>  DOMAIN
<223>  Amino acid sequence for heavy chain variable region of h1701-009 NHG
mutant antibody

<400>  61
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asp Ile Ile Pro Asn His Gly Gly Ser Lys Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Thr Trp Gly Tyr Gly Ser Ser Tyr Arg Trp Phe Asp Tyr Trp Gly
            100                 105                 110
Gln Gly Thr Leu Val Thr Val Ser Ser
        115                 120

<210>  62
<211>  17
<212>  PRT
<213>  Artificial Sequence

<220>
<221>  DOMAIN
<223>  Amino acid sequence for HCDR2 region of h1701-009 NLG mutant antibody

<400>  62
Asp Ile Ile Pro Asn Leu Gly Gly Ser Lys Tyr Asn Gln Lys Phe Lys
1               5                   10                  15
Asp

<210>  63
<211>  17
<212>  PRT
<213>  Artificial Sequence

<220>
<221>  DOMAIN
<223>  Amino acid sequence for HCDR2 region of h1701-009 NVG mutant antibody

<400>  63
Asp Ile Ile Pro Asn Val Gly Gly Ser Lys Tyr Asn Gln Lys Phe Lys
1               5                   10                  15
Asp

<210>  64
<211>  17

<212>  PRT
<213>  Artificial Sequence

<220>
<221>  DOMAIN
<223>  Amino acid sequence for HCDR2 region of h1701-009 NNA mutant antibody

<400>  64
Asp Ile Ile Pro Asn Asn Ala Gly Ser Lys Tyr Asn Gln Lys Phe Lys
1               5                   10                  15
Asp


<210>  65
<211>  17
<212>  PRT
<213>  Artificial Sequence

<220>
<221>  DOMAIN
<223>  Amino acid sequence for HCDR2 region of h1701-009 NMA mutant antibody

<400>  65
Asp Ile Ile Pro Asn Met Ala Gly Ser Lys Tyr Asn Gln Lys Phe Lys
1               5                   10                  15
Asp


<210>  66
<211>  17
<212>  PRT
<213>  Artificial Sequence

<220>
<221>  DOMAIN
<223>  Amino acid sequence for HCDR2 region of h1701-009 NEA mutant antibody

<400>  66
Asp Ile Ile Pro Asn Glu Ala Gly Ser Lys Tyr Asn Gln Lys Phe Lys
1               5                   10                  15
Asp


<210>  67
<211>  17
<212>  PRT
<213>  Artificial Sequence

<220>
<221>  DOMAIN
<223>  Amino acid sequence for HCDR2 region of h1701-009 NHA mutant antibody

<400>  67
Asp Ile Ile Pro Asn His Ala Gly Ser Lys Tyr Asn Gln Lys Phe Lys
1               5                   10                  15
Asp


<210>  68
<211>  17
<212>  PRT
<213>  Artificial Sequence

<220>
<221>  DOMAIN
<223>  Amino acid sequence for HCDR2 region of h1701-009 NMG mutant antibody

<400>  68
Asp Ile Ile Pro Asn Met Gly Gly Ser Lys Tyr Asn Gln Lys Phe Lys
1               5                   10                  15
Asp


<210>  69
<211>  17
<212>  PRT
<213>  Artificial Sequence

<220>
<221>  DOMAIN
<223>  Amino acid sequence for HCDR2 region of h1701-009 NEG mutant antibody

<400>  69
Asp Ile Ile Pro Asn Glu Gly Gly Ser Lys Tyr Asn Gln Lys Phe Lys
1               5                   10                  15
Asp


<210>  70
<211>  17
<212>  PRT
<213>  Artificial Sequence

<220>
<221>  DOMAIN
<223>  Amino acid sequence for HCDR2 region of h1701-009 NKG mutant antibody

<400>  70
Asp Ile Ile Pro Asn Lys Gly Gly Ser Lys Tyr Asn Gln Lys Phe Lys
1               5                   10                  15
Asp


<210>  71
<211>  17
<212>  PRT
<213>  Artificial Sequence

<220>
<221>  DOMAIN
<223>  Amino acid sequence for HCDR2 region of h1701-009 NAG mutant antibody

<400>  71
Asp Ile Ile Pro Asn Ala Gly Gly Ser Lys Tyr Asn Gln Lys Phe Lys
1               5                   10                  15
Asp


<210>  72
<211>  17
<212>  PRT
<213>  Artificial Sequence

<220>
<221>  DOMAIN
<223>  Amino acid sequence for HCDR2 region of h1701-009 NHG mutant antibody

<400>  72
Asp Ile Ile Pro Asn His Gly Gly Ser Lys Tyr Asn Gln Lys Phe Lys
1               5                   10                  15
Asp

<210>  73
<211>  449
<212>  PRT
<213>  Artificial Sequence

<220>
<221>  DOMAIN
<223>  Amino acid sequence for full-length h1799-005 antibody heavy chain

<400>  73
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Tyr
            20                  25                  30
Tyr Met Ala Trp Val Arg Gln Ala Pro Gly Lys Gly Leu Glu Trp Val
        35                  40                  45
Ala Asn Ile Asn Tyr Asp Gly Ser Ser Thr Tyr Tyr Leu Asp Ser Leu
    50                  55                  60
Lys Ser Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Val Gly Tyr Tyr Gly Gly Asn Tyr Gly Phe Ala Tyr Trp
            100                 105                 110
Gly Gln Gly Thr Leu Val Thr Val Ser Ser Ala Ser Thr Lys Gly Pro
        115                 120                 125
Ser Val Phe Pro Leu Ala Pro Cys Ser Arg Ser Thr Ser Glu Ser Thr
    130                 135                 140
Ala Ala Leu Gly Cys Leu Val Lys Asp Tyr Phe Pro Glu Pro Val Thr
145                 150                 155                 160
Val Ser Trp Asn Ser Gly Ala Leu Thr Ser Gly Val His Thr Phe Pro
                165                 170                 175
Ala Val Leu Gln Ser Ser Gly Leu Tyr Ser Leu Ser Ser Val Val Thr
            180                 185                 190
Val Pro Ser Ser Ser Leu Gly Thr Lys Thr Tyr Thr Cys Asn Val Asp
        195                 200                 205
His Lys Pro Ser Asn Thr Lys Val Asp Lys Arg Val Glu Ser Lys Tyr
    210                 215                 220
Gly Pro Pro Cys Pro Pro Cys Pro Ala Pro Glu Phe Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser Gln Glu Asp
            260                 265                 270
Pro Glu Val Gln Phe Asn Trp Tyr Val Asp Gly Val Glu Val His Asn
        275                 280                 285
Ala Lys Thr Lys Pro Arg Glu Glu Gln Phe Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Gly Leu Pro Ser Ser Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Gln Glu Glu Met Thr Lys Asn Gln Val Ser Leu Thr

355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Arg Leu Thr Val Asp Lys
                405                 410                 415
Ser Arg Trp Gln Glu Gly Asn Val Phe Ser Cys Ser Val Met His Glu
            420                 425                 430
Ala Leu His Asn His Tyr Thr Gln Lys Ser Leu Ser Leu Ser Leu Gly
        435                 440                 445
Lys

<210>  74
<211>  214
<212>  PRT
<213>  Artificial Sequence

<220>
<221>  DOMAIN
<223>  Amino acid sequence for full-length h1799-005 antibody light chain

<400>  74
Asp Ile Gln Met Thr Gln Ser Pro Ser Ser Leu Ser Ala Ser Val Gly
1               5                   10                  15
Asp Arg Val Thr Ile Thr Cys Arg Ala Ser Asp Asn Ile Tyr Ser Tyr
            20                  25                  30
Leu Ala Trp Tyr Gln Gln Lys Pro Gly Lys Ala Pro Lys Leu Leu Ile
        35                  40                  45
Tyr Asn Ala Lys Thr Leu Ala Glu Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln His Tyr Gly Ser Pro Leu
                85                  90                  95
Thr Phe Gly Gln Gly Thr Lys Leu Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

## Description

### FIELD OF THE INVENTION

The present disclosure belongs to the field of pharmaceutical formulation, and specifically relates to a pharmaceutical composition comprising a TIM-3 antibody or antigen-binding fragment thereof, and the use of the same as an anti-cancer medicament.

### BACKGROUND OF THE INVENTION

The statements herein only provide background information related to the present invention, and do not necessarily constitute the prior art.

T cell immunoglobulin and mucin-domain-containing molecule 3 (TIM-3) is also referred to as hepatitis A virus cellular receptor 2 (HAVCR-2). TIM-3 is Type I membrane surface protein, and belongs to a member of TIM family. Human TIM-3 molecule is composed of 301 amino acids, comprising signal peptide, Ig variable region (IgV region), Ser/Thr-rich mucin region, trans-membrane region and cytoplasmic region; human TIM-3 shares 63% homology with murine TIM-3.

TIM-3 is selectively expressed on the surface of IFN-γ-secreting T helper cells (Th1 and Th17), T regulatory cells (Treg), dendritic cells (DCs), monocytes, mast cells, NK cells, tumor-infiltrating lymphocytes (TILs) (see, eg, Clayton et al., J. Immunol., 192(2): 782-791 (2014); Jones et al., J. Exp. Med., 205: 2763-2779 (2008)). Currently known receptors for TIM-3 involve phosphatidyl serine galectin-9 (Galectin-9, Gal-9), high mobility group protein 1 (HMGB1) and carcinoembryonic antigen cell adhesion molecule 1 (CEACAM1).

TIM-3 can regulate the function of the immune system in many ways. It can bind to ligand Gal-9 on the surface of Th1 cells to down-regulate Th1 cell response, and induce Th1 cell apoptosis. It plays an important role in auto-immune and allogeneic immune diseases (such as systemic erythema lupus, asthma) and immune tolerance. TIM-3 expressed by monocyte-macrophages interacts with phosphatidyl serine to promote the phagocytosis of apoptotic cells; in tumor-infiltrating DCs, TIM-3 binds to ligand HMGB1 to inhibit correct transportation of nucleic acids, thereby inhibiting nucleic acid immune response and being involved in immune escape. TIM-3 is not only expressed in immune cells, but also over-expressed in tumor cells such as ovarian cancer, meningioma, and melanoma, and directly promotes tumor growth. Down-regulating the expression of TIM-3 can significantly inhibit the invasion and metastasis of HeLa cells. The overexpression of TIM-3 is closely related to the poor prognosis of lung cancer, gastric cancer, prostate cancer and cervical cancer. In hematological tumors, TIM-3 is overexpressed on leukemia stem cells of acute myeloid leukemia and hematopoietic stem cells of MDS patients, and TIM-3+ hematopoietic stem cells have malignant biological characteristics (low differentiation, low apoptosis and high proliferation). Therefore, inhibiting the activity of TIM-3 (such as TIM-3 antibody) to improve the function of the innate immune system is expected to become a new method for the treatment of tumors (see, for example, Ngiow et a1., Cancer Res., 71(21): 1-5 (2011); Guo et a1., Journal of Translational Medicine, 11: 215 (2013); and Ngiow et a1., Cancer Res., 71(21): 6567-6571 (2011)).

The stability of antibody medicament is one of the key factors affecting the drugability of antibodies. Among them, the non-enzymatic deamidation of asparagine (Asn) and glutamine (Gln) can make antibodies instable and heterogeneous, and is one of the common chemical degradation pathways of antibody molecules. It has been reported in literature that several IgG1 subtype antibodies lost their biological activity due to the deamidation and isomerization of amino acids in the CDR regions. Huang et al. found that the deamidation on Asn55 located in heavy chain variable region 2 (CDR2) of IgG1 monoclonal antibodies significantly reduced their binding activity. Therefore, in the process of antibody development, the corresponding amino acids should be avoided or mutated to reduce the deamidation in antibodies, so as to increase the stability and bioavailability of the antibodies.

At present, there have been patent reports regarding TIM-3 antibodies, such as WO2011159877, WO2013006490, WO2015117002, WO2016144803, WO2016161270 and US20150218274. However, in domestic or aboard, only two TIM-3 antibodies are at the clinical research stage. Other antibody medicaments are still at the stage of discovery and research, and no TIM-3 antibody has entered clinical application. Therefore, it is necessary to further develop TIM-3 antibodies with higher activity, high affinity and high stability, for the study and application of treating TIM-3 related diseases.

The present disclosure provides a pharmaceutical composition comprising a TIM-3 antibody or antigen-binding fragment thereof with stable performance, which is more beneficial for manufacture and administration.

### SUMMARY OF THE INVENTION

The present disclosure provides a pharmaceutical composition, comprising a TIM-3 antibody or antigen-binding fragment thereof and a buffer, the buffer is selected from the group consisting of acetate, histidine salt, succinate, phosphate, citrate buffer, preferably the buffer is acetate buffer and histidine salt buffer.

In some embodiments, the acetate buffer in the pharmaceutical composition is acetic acid-sodium acetate buffer, the histidine salt buffer is histidine-acetic acid buffer or histidine-hydrochloric acid buffer; the succinate buffer is succinic acid-sodium succinate buffer; the phosphate buffer is disodium hydrogen phosphate-sodium dihydrogen phosphate buffer; the citrate buffer is citric acid-sodium citrate; preferably histidine-hydrochloric acid, acetic acid-sodium acetate or histidine-acetic acid buffer, most preferably histidine-acetic acid buffer.

In some embodiments, the concentration of the buffer in the pharmaceutical composition is about 5mM to 30mM, preferably about 10mM to 30mM, preferably about 5mM to 20mM, preferably about 10mM to 20mM; non-limiting examples involve 10mM, 12mM, 14mM, 16mM, 18mM, 20mM, 30mM, most preferably about 10mM.

In some embodiments, the concentration of the TIM-3 antibody or antigen-binding fragment thereof in the pharmaceutical composition is about 1 mg/ml to 100 mg/ml, preferably about 40 mg/ml to 60 mg/ml, preferably 50 mg/ml to 60mg/ml; non-limiting examples involve 1mg/ml, 10mg/ml, 20mg/ml, 30mg/ml, 40mg/ml, 41mg/ml, 42mg/ml, 43mg/ml, 44mg/ml, 45mg/ml, 46mg/ml, 47mg/ml, 48mg/ml, 49mg/ml, 50mg/ml, 51mg/ml, 52mg/ml, 53mg/ml, 54mg/ml, 55mg/ml, 56mg/ml, 57mg/ml, 58 mg/ml, 59 mg/ml, 60 mg/m, 70 mg/m, 80 mg/m, 90 mg/m, 100 mg/ml, most preferably 50 mg/ml.

In some embodiments, the pH value of the buffer in the pharmaceutical composition is about 5.0 to 6.5, preferably about 5.0 to 6.0, preferably about 5.2 to 5.8, or preferably about 5.5 to 6.0, most preferably 5.5, non-limiting examples involve about 5.0, about 5.1, about 5.2, about 5.3, about 5.4, about 5.5, about 5.6, about 5.7, about 5.8, about 5.9, about 6.0 and about 6.5.

Further, in some embodiments, the pharmaceutical composition described above also comprises saccharide. The "saccharide" of the present disclosure encompasses conventional composition (CH₂O)ₙ and the derivatives thereof, involving monosaccharides, disaccharides, trisaccharides, polysaccharides, saccharide alcohols, reducing saccharides, non-reducing saccharides and so on. The saccharide can be selected from the group consisting of glucose, sucrose, trehalose, lactose, fructose, maltose, dextran, glycerol, erythritol, glycerol, arabitol, xylitol, sorbitol, mannitol, melibiose, melezitose, melitriose, manninotriose, stachyose, maltose, lactulose, maltulose, sorbitol, maltitol, lactitol, iso-maltulose, etc. The preferred saccharide is a non-reducing disaccharide, more preferably trehalose or sucrose, and most preferably sucrose.

In some embodiments, the concentration of saccharide in the pharmaceutical composition described above is about 50 mg/ml to about 100 mg/ml, preferably about 60 mg/ml to about 90 mg/ml, preferably about 70 mg/ml to about 90 mg/ml, preferably 70 mg/ml to about 80mg/ml, non-limiting examples involve 50mg/ml, 60mg/ml, 65mg/ml, 70mg/ml, 75mg/ml, 80mg/ml, 85mg/ml, 90mg/ml, most preferably 80mg/ml.

Further, in some embodiments, the pharmaceutical composition also involves a surfactant. The surfactant can be selected from the group consisting of polysorbate 20, polysorbate 80, polyhydroxyalkylene, Triton, sodium dodecyl sulfonate, sodium lauryl sulfonate, sodium octyl glycoside, lauryl/myristyl/linoleyl/stearyl-sulfobetaine, lauryl/myristyl/linoleyl/stearyl-sarcosine, linoleyl/myristyl/cetyl-betaine, lauramidopropyl/cocamidopropyl/linoleamidopropyl/myristamidopropyl /palmitamidopropyl/isostearamidopropyl-betaine, myristamidopropyl/palmitamidopropyl/isostearamidopropyl-dimethylamine, sodium methyl cocoyl taurate, sodium methyl oleyl taurate, polyethylene glycol, polypropylene glycol, copolymer of ethylene and propylene glycol and so on. The preferred surfactant is polysorbate 80 or polysorbate 20, more preferably polysorbate 80.

In some embodiments, the concentration of the surfactant in the pharmaceutical composition is about 0.2mg/ml to 0.8mg/ml, preferably 0.2mg/ml to 0.6mg/ml, more preferably 0.4mg/ml to 0.6mg/ml, non-limiting examples involve 0.2mg/ml, 0.3mg/ml, 0.4mg/ml, 0.45mg/ml, 0.5mg/ml, 0.55mg/ml, 0.6mg/ml, 0.7mg/ml, 0.8mg/ml, most preferably 0.4mg/ml.

In an alternative embodiment, the pharmaceutical composition comprises:
(a) about 1mg/ml to 100mg/ml of the TIM-3 antibody or antigen-binding fragment thereof,
(b) about 5mM to 30mM of the acetate buffer or of the histidine salt buffer,
(c) about 50mg/ml to 100mg/ml of the saccharide, and
(d) about 0.2mg/ml to 0.8mg/ml of the polysorbate.

In an alternative embodiment, the pharmaceutical composition comprises:
(a) 1 mg/ml to 100mg/ml of the TIM-3 antibody or antigen-binding fragment thereof,
(b) 5mM to 30mM of the histidine-acetic acid buffer or acetic acid-sodium acetate buffer, pH is about 5.0 to 6.5,
(c) 50 mg/ml to 100 mg/ml of the saccharide, and
(d) 0.2 mg/ml to 0.8 mg/ml of the polysorbate 80.

In an alternative embodiment, the pharmaceutical composition comprises:
(a) 40 mg/ml to 60mg/ml of the TIM-3 antibody or antigen-binding fragment thereof,
(b) 10mM to 30mM of the histidine-acetic acid buffer or acetic acid-sodium acetate buffer, pH of about 5.0 to 6.0,
(c) 70 mg/ml to 90 mg/ml of the sucrose or trehalose, and
(d) 0.4 mg/ml to 0.6 mg/ml of the polysorbate 80.

In an alternative embodiment, the pharmaceutical composition comprises:
(a) about 50mg/ml of the TIM-3 antibody or antigen-binding fragment thereof,
(b) about 10mM of the histidine-acetic acid buffer, pH of about 5.5,
(c) about 80mg/ml of the sucrose, and
(d) about 0.4 mg/ml of the polysorbate 80.

In some embodiments, the TIM-3 antibody or antigen-binding fragment thereof in the pharmaceutical composition of the present disclosure is characterized in that it binds to the extracellular region of human TIM-3, and competes to bind to TIM-3 with any monoclonal antibody or antigen-binding fragment thereof selected from the group consisting of (i) to (ii), or is any monoclonal antibody or antigen-binding fragment thereof selected from the group consisting of (i) to (ii):
(i) a monoclonal antibody or antigen-binding fragment thereof, comprising one or more CDR region sequence(s) selected from the group consisting of the following, or having at least 95% sequence identity thereto:
   the sequences of the antibody heavy chain variable region HCDR regions: as shown in the amino acid sequence SEQ ID NOs: 8, 43 and 10; and/or
   the sequences of the antibody light chain variable region LCDR regions: as shown in the amino acid sequence SEQ ID NOs: 11, 12 and 13;
(ii) a monoclonal antibody or antigen-binding fragment thereof, comprising one or more CDR region sequence(s) selected from the group consisting of the following, or having at least 95% sequence identity thereto:
   the sequences of the antibody heavy chain variable region HCDR regions: as shown in the amino acid sequence SEQ ID NOs: 14, 15 and 16; and/or
   the sequences of the antibody light chain variable region LCDR regions: as shown in the amino acid sequence SEQ ID NOs: 17, 18 and 19.

In some embodiments, the TIM-3 antibody or antigen-binding fragment thereof is any monoclonal antibody or antigen-binding fragment thereof selected from the following (i) or (ii):
(i) a monoclonal antibody or antigen-binding fragment thereof, comprising: antibody heavy chain variable region HCDR1-3 regions as shown in sequence SEQ ID NOs: 8, 43 and 10 respectively, or having at least 95% sequence identity to SEQ ID NOs: 8, 43 and 10; and/or antibody light chain variable region LCDR1-3 regions as shown in sequence SEQ ID NOs: 11, 12 and 13 respectively, or having at least 95% sequence identity to SEQ ID NOs: 11, 12 and 13;
   wherein, SEQ ID NO: 43 is as shown in sequence DIIPX₁X₂X₃GSKYNQKFKD, wherein X₁ is selected from the group consisting of N, L, V, M and E, X₂ is selected from the group consisting of N, E, M, H, K, L, A and V, X₃ is selected from the group consisting of G and A;
(ii) a monoclonal antibody or antigen-binding fragment thereof, comprising: antibody heavy chain variable region HCDR1-3 regions as shown in sequence SEQ ID NOs: 14, 15 and 16 respectively, or having at least 95% sequence identity to SEQ ID NOs: 14, 15 and 16; and/or antibody light chain variable region LCDR1-3 regions as shown in sequence SEQ ID NOs: 17, 18 and 19 respectively, or having at least 95% sequence identity to SEQ ID NOs: 17, 18 and 19.

The amino acid sequence having at least 95% sequence identity described above, preferably has at least 95%, 96%, 97%, 98% or 99% sequence identity, more preferably, it has 97%, 98% or 99% or more, and most preferably it has at least 99% or more sequence identity. The amino acid sequence having at least 95% sequence identity described above can be obtained by deletion, insertion or substitution mutation of one or more amino acids.

In some embodiments, the monoclonal antibody or antigen-binding fragment thereof comprises the antibody heavy chain variable region HCDR1-3 region sequences as shown in SEQ ID NOs: 8, 43 and 10 respectively; and the antibody light chain variable region LCDR1-3 region sequences as shown in SEQ ID NOs: 11, 12 and 13;
or, comprises the antibody heavy chain variable region HCDR1-3 region sequences as shown in SEQ ID NOs: 14, 15 and 16 respectively; and the antibody light chain variable region LCDR1-3 region sequences as shown in SEQ ID NOs: 17, 18 and 19.

In some embodiments, the TIM-3 antibody or antigen-binding fragment in the pharmaceutical composition of the present disclosure competes to bind to TIM-3 with the monoclonal antibody or the antigen-binding fragment as shown in any one of (a) to (m), or is any monoclonal antibody or the antigen-binding fragment selected from the group consisting of (a) to (m):
(a) a monoclonal antibody or antigen-binding fragment thereof, comprising: antibody heavy chain variable region HCDR1-3 regions as shown in sequence SEQ ID NOs: 8, 9 and 10 respectively; and antibody light chain variable region LCDR1-3 regions as shown in sequence SEQ ID NOs: 11, 12 and 13 respectively;
(b) a monoclonal antibody or antigen-binding fragment thereof, comprising: antibody heavy chain variable region HCDR1-3 regions as shown in sequence SEQ ID NOs: 8, 62 and 10 respectively; and antibody light chain variable region LCDR1-3 regions as shown in sequence SEQ ID NOs: 11, 12 and 13 respectively;
(c) a monoclonal antibody or antigen-binding fragment thereof, comprising: antibody heavy chain variable region HCDR1-3 regions as shown in sequence SEQ ID NOs: 8, 63 and 10 respectively; and antibody light chain variable region LCDR1-3 regions as shown in sequence SEQ ID NOs: 11, 12 and 13 respectively;
(d) a monoclonal antibody or antigen-binding fragment thereof, comprising: antibody heavy chain variable region HCDR1-3 regions as shown in sequence SEQ ID NOs: 8, 64 and 10 respectively; and antibody light chain variable region LCDR1-3 regions as shown in sequence SEQ ID NO: 11, 12 and 13 respectively;
(e) a monoclonal antibody or antigen-binding fragment thereof, comprising: antibody heavy chain variable region HCDR1-3 regions as shown in sequence SEQ ID NOs: 8, 65 and 10 respectively; and antibody light chain variable region LCDR1-3 regions as shown in sequence SEQ ID NOs: 11, 12 and 13 respectively;
(f) a monoclonal antibody or antigen-binding fragment thereof, comprising: antibody heavy chain variable region HCDR1-3 regions as shown in sequence SEQ ID NOs: 8, 66 and 10 respectively; and antibody light chain variable region LCDR1-3 regions as shown in sequence SEQ ID NOs: 11, 12 and 13 respectively;
(g) a monoclonal antibody or antigen-binding fragment thereof, comprising: antibody heavy chain variable region HCDR1-3 regions as shown in sequence SEQ ID NOs: 8, 67 and 10 respectively; and antibody light chain variable region LCDR1-3 regions as shown in sequence SEQ ID NOs: 11, 12 and 13 respectively;
(h) a monoclonal antibody or antigen-binding fragment thereof, comprising: antibody heavy chain variable region HCDR1-3 regions as shown in sequence SEQ ID NOs: 8, 68 and 10 respectively; and antibody light chain variable region LCDR1-3 regions as shown in sequence SEQ ID NOs: 11, 12 and 13 respectively;
(i) a monoclonal antibody or antigen-binding fragment thereof, comprising: antibody heavy chain variable region HCDR1-3 regions as shown in sequence SEQ ID NOs: 8, 69 and 10 respectively; and antibody light chain variable region LCDR1-3 regions as shown in sequence SEQ ID NOs: 11, 12 and 13 respectively;
(j) a monoclonal antibody or antigen-binding fragment thereof, comprising: antibody heavy chain variable region HCDR1-3 regions as shown in sequence SEQ ID NOs: 8, 70 and 10 respectively; and antibody light chain variable region LCDR1-3 regions as shown in sequence SEQ ID NOs: 11, 12 and 13 respectively;
(k) a monoclonal antibody or antigen-binding fragment thereof, comprising: antibody heavy chain variable region HCDR1-3 regions as shown in sequence SEQ ID NOs: 8, 71 and 10 respectively; and antibody light chain variable region LCDR1-3 regions as shown in sequence SEQ ID NOs: 11, 12 and 13 respectively;
(l) a monoclonal antibody or antigen-binding fragment thereof, comprising: antibody heavy chain variable region HCDR1-3 regions as shown in sequence SEQ ID NOs: 8, 72 and 10 respectively; and antibody light chain variable region LCDR1-3 regions as shown in sequence SEQ ID NOs: 11, 12 and 13 respectively; and
(m) a monoclonal antibody or antigen-binding fragment thereof, comprising: antibody heavy chain variable region HCDR1-3 regions as shown in sequence SEQ ID NOs: 14, 15 and 16 respectively; and antibody light chain variable region LCDR1-3 regions as shown in sequence SEQ ID NOs: 17, 18 and 19 respectively.

In some embodiments, the TIM-3 antibody or the antigen-binding fragment in the pharmaceutical composition of the present disclosure is characterized in that it binds to the same epitope as that bound by any one of the monoclonal antibodies described above.

In some embodiments of the pharmaceutical composition of the present disclosure, with respect to the TIM-3 antibody or antigen-binding fragment thereof, the antibody is a recombinant antibody.

In some embodiments of the TIM-3 antibody or antigen-binding fragment thereof of the present disclosure, the monoclonal antibody is a recombinant antibody selected from the group consisting of murine antibody, chimeric antibody and humanized antibody, or antigen-binding fragment thereof.

In some embodiments of the pharmaceutical composition of the present disclosure, the TIM-3 antibody or antigen-binding fragment thereof comprises the heavy chain variable region sequence as shown in SEQ ID NO: 4 and/or the light chain variable region sequence as shown in SEQ ID NO: 5; or comprises the heavy chain variable region sequence as shown in SEQ ID NO: 6 and/or the light chain variable region sequence as shown in SEQ ID NO: 7.

In some embodiments of the pharmaceutical composition of the present disclosure, with respect to the TIM-3 antibody or antigen-binding fragment thereof, the light chain and heavy chain FR region sequences on the humanized antibody light chain and heavy chain variable region are from human germline light chain and heavy chain respectively, or mutant sequence thereof.

In some embodiments of the pharmaceutical composition of the present disclosure, with respect to the TIM-3 antibody or antigen-binding fragment thereof, the humanized antibody comprises the heavy chain variable region as shown in SEQ ID NO: 20 or 31 or variant thereof, the variant has 1-10 amino acid back mutation(s) in the heavy chain variable region as shown in SEQ ID NO: 20 or 31.

In some embodiments of the TIM-3 antibody or antigen-binding fragment thereof of the present disclosure, the variant has 1-10 amino acid back mutation(s) in FR regions of the heavy chain variable region as shown in SEQ ID NO: 20 or 31; preferably, the back mutation(s) is/are one or more amino acid back mutation(s) selected from the group consisting of D89E, R98T, G49A, M48I, M70L, R38K and V68A in SEQ ID NO: 20; or amino acid back mutation(s) of Q3K and/or R87K in the heavy chain variable region as shown in SEQ ID NO: 31.

In some embodiments, in order to eliminate chemical modifications (such as acetylation and deamidation) of the CDR region(s) of an antibody, the site(s) which is(are) susceptible to chemical modification in CDR regions of the TIM-3 antibody or antigen-binding fragment thereof of the present disclosure, can be subjected to amino acid substitution; preferably NNG in CDR2 is substituted; preferably, in an embodiment of the TIM-3 antibody or antigen-binding fragment thereof of the present disclosure, the humanized antibody comprises the heavy chain variable region as shown in any one selected from the group consisting of SEQ ID NOs: 45, 45, 46, 47, 48, 49 and 50.

In some embodiments of the TIM-3 antibody or antigen-binding fragment thereof of the present disclosure, the humanized antibody involves the heavy chain variable region as shown in SEQ ID NO: 34 or 35.

In some embodiments of the TIM-3 antibody or antigen-binding fragment thereof of the present disclosure, the humanized antibody comprises a light chain variable region as shown in SEQ ID NO: 21 or 32 or variant thereof; the variant has 1-10 amino acid back mutation(s) in the light chain variable region as shown in SEQ ID NO: 21 or 32.

In some embodiments, the TIM-3 antibody or antigen-binding fragment thereof comprises FR regions of the light chain variable region as shown in SEQ ID NO: 21 or 32 or variant thereof; the variant has 1-10 amino acid back mutation(s) in the light chain variable region as shown in SEQ ID NO: 21 or 32; preferably, the back mutation is at least one selected from the group consisting of A43S in SEQ ID NO: 21, Q3K, 148V, K45Q, A43S and T85S in SEQ ID NO: 32.

In some embodiments of the TIM-3 antibody or antigen-binding fragment thereof of the present disclosure, the humanized antibody comprises the light chain variable region as shown in SEQ ID NO: 30, or comprises the light chain variable region as shown in any one selected from the group consisting of SEQ ID NOs: 37, 37, 38, 39 and 40.

In some embodiments, in order to eliminate chemical modifications (such as acetylation and deamidation) of the CDR region(s) of an antibody, the site(s) which is(are) susceptible to chemical modification in CDR regions of the TIM-3 antibody or antigen-binding fragment thereof of the present disclosure can be subjected to amino acid substitution; preferably NNG in CDR2 is substituted, to form the CDR2 sequence as shown in SEQ ID NO: 43; in some embodiments of the TIM-3 antibody or antigen-binding fragment thereof of the present disclosure, the humanized antibody comprises heavy chain variable region sequence as shown in any one selected from the group consisting of SEQ ID NOs: 44, 45, 46, 47, 48, 49 and 50, and light chain variable region sequence as shown in SEQ ID NO: 29 or 30.

In some embodiments of the TIM-3 antibody or antigen-binding fragment thereof of the present disclosure, the humanized antibody comprises the heavy chain variable region sequence as shown in any one selected from the group consisting of SEQ ID NOs: 22, 23, 24, 25, 26, 27 and 28; and the light chain variable region sequence as shown in SEQ ID NO: 29 or 30; preferably, the heavy chain variable region sequence as shown in SEQ ID NO: 24 and the light chain variable region sequence as shown in SEQ ID NO: 29.

In some embodiments of the TIM-3 antibody or antigen-binding fragment thereof of the present disclosure, the humanized antibody comprises the heavy chain variable region sequence as shown in any one selected from the group consisting of SEQ ID NOs: 33, 34 and 35; and the light chain variable region sequence as shown in any one selected from the group consisting of SEQ ID NOs: 36, 37, 38, 39 and 40; preferably, the heavy chain variable region sequence as shown in SEQ ID NO: 33 and the light chain variable region sequence as shown in SEQ ID NO: 36.

In some embodiments, in order to eliminate deamidation of amino acid site(s) in the CDR region(s) of an antibody, the site(s) which is(are) susceptible to chemical modification in CDR regions of the TIM-3 antibody or antigen-binding fragment thereof of the present disclosure can be subjected to amino acid substitution; preferably NNG in CDR2 is substituted; in a preferable embodiment of the TIM-3 antibody or antigen-binding fragment thereof of the present disclosure, the humanized antibody comprises the heavy chain variable region sequence as shown in any one selected from the group consisting of SEQ ID NOs: 51, 52, 53, 54, 55, 56, 57, 58, 59, 60 and 61, and the light chain variable region sequence as shown in SEQ ID NO: 29 or 30; preferably, the heavy chain variable region sequence as shown in SEQ ID NO: 51 and the light chain variable region sequence as shown in SEQ ID NO: 29; or the heavy chain variable region sequence as shown in SEQ ID NO: 52 and the light chain variable region sequence as shown in SEQ ID NO: 29.

In some embodiments of the TIM-3 antibody or antigen-binding fragment thereof of the present disclosure, wherein the antibody is a full-length antibody, and further comprises human antibody constant region(s), preferably a human heavy chain constant region sequence as shown in SEQ ID NO: 41 or a sequence having 85% sequence identity to SEQ ID NO: 41; and preferably a human light chain constant region sequence as shown in SEQ ID NO: 42 or a sequence having 85% sequence identity to SEQ ID NO: 42. The amino acid sequence having at least 85% sequence identity described above preferably has at least 86%, 87%, 88%, 89%, 90%, 91%, 92%, 93%, 94%, 95%, 96%, 97%, 98% or 99% sequence identity; more preferably, it has 90%, 95% or 99% or more; most preferably, it has at least 95% or more sequence identity; the amino acid sequence having at least 85% sequence identity described above can be obtained by deletion, insertion or substitution mutation of one or more amino acids. Most preferably, the antibody comprises the full-length sequence of the heavy chain as shown in SEQ ID NO: 73 and the full-length sequence of the light chain as shown in SEQ ID NO: 74.

In some embodiments of the TIM-3 antibody or antigen-binding fragment thereof of the present disclosure, the antigen-binding fragment is selected from the group consisting of Fab, Fab', F(ab')2, single-chain antibody (scFv), dimerized V region (diabody), disulfide stabilized V region (dsFv) and antigen-binding fragment of peptides comprising CDR regions.

The present disclosure also provides a method for preparing the pharmaceutical composition described above, which comprises a step of replacing the stock solution of TIM3 antibody by using a buffer. In some embodiments, preferably the buffer is acetate buffer or histidine salt buffer, more preferably histidine-hydrochloric acid, acetic acid-sodium acetate or histidine-acetic acid buffer; and most preferably histidine-acetic acid buffer. In some embodiments, the concentration of the buffer is about 5mM to 30mM, preferably about 5mM to 20mM, preferably 10mM to 30mM, non-limiting examples involve 5mM, 10mM, 12mM, 14mM, 16mM, 18mM, 20mM, 30mM, most preferably about 10mM. In some embodiments, the pH value of the buffer is about 5.0 to 6.5, preferably about 5.0 to 6.0, preferably about 5.2 to 5.8, most preferably 5.5, non-limiting examples involve about 5.0, about 5.1, about 5.2, about 5.3, about 5.4, about 5.5, about 5.6, about 5.7, about 5.8, about 5.9, about 6.0.

The present disclosure also provides a method for preparing the pharmaceutical composition described above, further comprising a step of adding saccharide and surfactant into the solution obtained in the replacement step, and a step of adjusting the volume with a buffer. In some embodiments, the preferred saccharide is a non-reducing disaccharide, more preferably trehalose or sucrose, most preferably sucrose; and the concentration of saccharide is about 50 mg/ml to about 100 mg/ml, more preferably about 60 mg/ml to about 90 mg/ml, more preferably 70mg/ml to about 80mg/ml, non-limiting examples involve 60mg/ml, 65mg/ml, 70mg/ml, 75mg/ml, 80mg/ml, 85mg/ml, 90mg/ml, most preferably 80 mg/ml. In some embodiments, preferably the surfactant is polysorbate 80 or polysorbate 20, more preferably polysorbate 80; the concentration is about 0.2mg/ml to 0.8mg/ml, more preferably 0.4mg/ml to 0.6mg/ml, non-limiting examples involve 0.2mg/ml, 0.3mg/ml, 0.4mg/ml, 0.45mg/ml, 0.5mg/ml, 0.55mg/ml, 0.6mg/ml, 0.7mg/ml, 0.8mg/ml, most preferably 0.4mg/ml.

The present disclosure also provides a method for preparing a lyophilized formulation comprising TIM3 antibody, which comprises a step of freeze-drying the pharmaceutical composition described above.

In some embodiments, the pharmaceutical composition of the present disclosure comprises: 50 mg/ml anti-TIM3 antibody h1799-005 and 10 mM histidine-acetic acid, pH 5.5.

In some embodiments, the pharmaceutical composition of the present disclosure comprises: 50 mg/ml anti-TIM3 antibody h1799-005 and 10 mM histidine-acetic acid, pH 6.0.

In some embodiments, the pharmaceutical composition of the present disclosure comprises: 50 mg/ml anti-TIM3 antibody h1799-005, 10 mM histidine-hydrochloric acid, pH 6.0.

In some embodiments, the pharmaceutical composition of the present disclosure comprises: 50 mg/ml anti-TIM3 antibody h1799-005, 10 mM acetic acid-sodium acetate, pH 5.5.

In some embodiments, the pharmaceutical composition of the present disclosure comprises: 50 mg/ml anti-TIM3 antibody h1799-005, 10 mM histidine-hydrochloric acid, pH 5.5.

In some embodiments, the pharmaceutical composition of the present disclosure comprises: 50 mg/ml anti-TIM3 antibody h1799-005, 10 mM histidine-acetic acid pH 6.0, and 70 mg/ml sucrose.

In some embodiments, the pharmaceutical composition of the present disclosure comprises: 50 mg/ml anti-TIM3 antibody h1799-005, 10 mM histidine-acetic acid pH 6.0, and 70 mg/ml α,α-trehalose dihydrate.

In some embodiments, the pharmaceutical composition of the present disclosure comprises: 50 mg/ml anti-TIM3 antibody h1799-005, 10 mM histidine-acetic acid pH 5.5, 70 mg/ml sucrose, and 0.4 mg/ml polysorbate 80.

In some embodiments, the pharmaceutical composition of the present disclosure comprises: 50 mg/ml anti-TIM3 antibody h1799-005, 10 mM histidine-acetic acid pH 5.5, 70 mg/ml sucrose and 0.2 mg/ml polysorbate 80.

In some embodiments, the pharmaceutical composition of the present disclosure comprises: 50 mg/ml anti-TIM3 antibody h1799-005, 10 mM histidine-acetic acid pH 5.5, 70 mg/ml sucrose and 0.6 mg/ml polysorbate 80.

In some embodiments, the pharmaceutical composition of the present disclosure comprises: 50 mg/ml anti-TIM3 antibody h1799-005, 10 mM histidine-acetic acid pH 5.5, 70 mg/ml sucrose and 0.4 mg/ml polysorbate 20.

In some embodiments, the pharmaceutical composition of the present disclosure comprises: 80 mg/ml sucrose, 0.4 mg/ml polysorbate 80, 20 mM histidine-acetic acid pH 5.5, and 60 mg/ml TIM3 antibody h1799-005.

In some embodiments, the pharmaceutical composition of the present disclosure comprises: 80 mg/ml sucrose, 0.4 mg/ml polysorbate 80, 10 mM histidine-acetic acid pH 5.5, and 50 mg/ml TIM3 antibody h1799-005.

In some embodiments, the pharmaceutical composition of the present disclosure comprises: 80 mg/ml sucrose and 0.4 mg/ml polysorbate 80, 20 mM histidine-acetic acid pH 5.5, and 50 mg/ml TIM3 antibody h1799-005.

In some embodiments, the pharmaceutical composition of the present disclosure comprises: 80 mg/ml sucrose and 0.4 mg/ml polysorbate 80, 10 mM histidine-acetic acid pH 6.0, and 40 mg/ml TIM3 antibody h1799-005.

In some embodiments, the pharmaceutical composition of the present disclosure comprising: 80 mg/ml sucrose and 0.4 mg/ml polysorbate 80, 30 mM histidine-acetic acid pH 5.5, and 50 mg/ml TIM3 antibody h1799-005.

In some embodiments, the pharmaceutical composition of the present disclosure comprises: 80 mg/ml sucrose and 0.4 mg/ml polysorbate 80, 20 mM histidine-acetic acid pH 6.0, and 50 mg/ml TIM3 antibody h1799-005.

In some embodiments, the pharmaceutical composition of the present disclosure comprises: 80 mg/ml sucrose and 0.4 mg/ml polysorbate 80, 30 mM histidine-acetic acid pH 5.0, and 60 mg/ml TIM3 antibody h1799-005.

In some embodiments, the pharmaceutical composition of the present disclosure comprises: 80 mg/ml sucrose and 0.4 mg/ml polysorbate 80, 10 mM histidine-acetic acid pH 6.0, and 60 mg/ml TIM3 antibody h1799-005.

In some embodiments, the pharmaceutical composition of the present disclosure comprises: 80 mg/ml sucrose and 0.4 mg/ml polysorbate 80, 20 mM histidine-acetic acid pH 5.0, and 50 mg/ml TIM3 antibody h1799-005.

In some embodiments, the pharmaceutical composition of the present disclosure comprises: 80 mg/ml sucrose and 0.4 mg/ml polysorbate 80, 30 mM histidine-acetic acid pH 6.0, and 60 mg/ml TIM3 antibody h1799-005.

In some embodiments, the pharmaceutical composition of the present disclosure comprises: 80 mg/ml sucrose and 0.4 mg/ml polysorbate 80, 20 mM histidine-acetic acid pH 5.5, and 40 mg/ml TIM3 antibody h1799-005.

In some embodiments, the pharmaceutical composition of the present disclosure comprises: 80 mg/ml sucrose and 0.4 mg/ml polysorbate 80, 30 mM histidine-acetic acid pH 6.0, and 40 mg/ml TIM3 antibody h1799-005.

In some embodiments, the pharmaceutical composition of the present disclosure comprises: 80 mg/ml sucrose and 0.4 mg/ml polysorbate 80, 30 mM histidine-acetic acid pH 5.0, and 40 mg/ml TIM3 antibody h1799-005.

In some embodiments, the pharmaceutical composition of the present disclosure comprises: 80 mg/ml sucrose and 0.4 mg/ml polysorbate 80, 10 mM histidine-acetic acid pH 5.0, and 60 mg/ml TIM3 antibody h1799-005.

In some embodiments, the pharmaceutical composition of the present disclosure comprises: 80 mg/ml sucrose and 0.4 mg/ml polysorbate 80, 10 mM histidine-acetic acid pH 5.0, and 40 mg/ml TIM3 antibody h1799-005.

In some embodiments of the method for preparing the lyophilized formulation comprising TIM3 antibody, the freeze-drying involves the steps of pre-freezing, primary drying and secondary drying, successively. Freeze-drying is performed by freezing the formulation and then sublimating the water at a temperature suitable for primary drying. Under this condition, the product temperature is lower than the eutectic point or decomposition temperature of the formulation. Under a suitable pressure usually in the range of about 50-250 millitorr, the storage temperature for the primary drying is usually about -30 to 25°C (assuming that the product remains frozen during the primary drying process). The size and type of the formulation and container (e.g. glass vial) holding the sample and the volume of the liquid determine the length of time required for drying, which can range from a few hours to several days (e.g. 40-60 hours). The secondary drying stage can be performed at about 0-40°C, depending on the type and size of the container and the type of protein used. The length of time for secondary drying is determined by the desired residual humidity level in the product and usually requires at least about 5 hours. Generally, the moisture content of the lyophilized formulation obtained by lyophilization is less than about 5%, preferably less than about 3%. The pressure can be the same as the pressure applied in the primary drying step. Preferably, the pressure of the secondary drying is lower than that of the primary drying. Conditions for freeze drying can vary with formulation and vial size.

The present disclosure also provides a lyophilized formulation comprising a TIM3 antibody prepared by the method for preparing a lyophilized formulation comprising TIM3 antibody described above.

In some embodiments, the lyophilized formulation is stable at 2-8°C for at least 3 months, at least 6 months, at least 12 months, at least 18 months, or at least 24 months. In some embodiments, the lyophilized formulation is stable at 40°C for at least 7 days, at least 14 days, or at least 28 days.

The present disclosure also provides a method for preparing a reconstitution solution of the lyophilized formulation comprising the TIM3 antibody, which comprises a step of reconstituting the lyophilized formulation described above, the solution used for reconstitution is selected from the group consisting of but not limited to water for injection, physiological saline or glucose solution.

The present disclosure also provides a reconstitution solution of the lyophilized formulation comprising the TIM3 antibody prepared by the method for preparing a reconstitution solution of the lyophilized formulation comprising the TIM3 antibody, as described above.

The present disclosure further provides an article of manufacture or a kit, comprising a container comprising any stable pharmaceutical composition described herein. In some embodiments, the container is an injection bottle made of neutral borosilicate glass.

The present disclosure also provides an article of manufacture, comprising a container comprising the pharmaceutical composition or the lyophilized formulation or the reconstitution solution of lyophilized formulation, described above.

The present disclosure also provides the use of the pharmaceutical composition or the lyophilized formulation or the reconstitution solution of the lyophilized formulation or the article of manufacture described above in the preparation of a diagnostic agent for diseases related to human TIM-3 positive cells.

The present disclosure also provides the use of the pharmaceutical composition or the lyophilized formulation or the reconstitution solution of the lyophilized formulation or the article of manufacture described above in the preparation of medicament for the treatment of diseases related to human TIM-3 positive cells. There is no restriction on the disease related to TIM-3 positive cells, as long as it is a disease related to TIM-3-expressing cells, comprising but not limited to cancer, autoimmune disease and allergic disease.

The present disclosure also provides a method for treating or diagnosing cancer, autoimmune disease and allergic disease, comprising administering the pharmaceutical composition or the lyophilized formulation or the reconstitution solution of the lyophilized formulation or the article of manufacture described above, to a subject. Preferably, the cancer involves blood cancer, breast cancer, uterine cancer, colorectal cancer, esophageal cancer, gastric cancer, ovarian cancer, lung cancer, kidney cancer, rectal cancer, thyroid cancer, cervical cancer, small intestine cancer, prostate cancer and pancreatic cancer. Preferable examples of cancer involve blood cancer, esophageal cancer, gastric cancer, colorectal cancer, liver cancer and prostate cancer. Examples of blood cancers involve acute myeloid leukemia (AML), chronic myeloid leukemia (CML), myelodysplastic syndrome (MDS), multiple myeloma, cutaneous T cell lymphoma (CTCL), peripheral T cell lymphoma (PTCL), anaplastic large cell lymphoma (ALCL), acute lymphocytic leukemia (ALL), chronic lymphocytic leukemia (CLL), other lymphocytic leukemia, NK cell lymphoma, Hodgkin's lymphoma, Non-Hodgkin's lymphoma (such as Burkitt lymphoma), and so on. Particular examples of autoimmune disease involve rheumatoid arthritis, psoriasis, Crohn's disease, ankylosing spondylitis, multiple sclerosis, diabetes type I, hepatitis, myocarditis, Sjogren syndrome, autoimmune hemolysis anemia after transplant rejection, vesicular pemphigoid, Grave's disease, Hashimoto's thyroiditis, systemic lupus erythematosus (SLE), myasthenia gravis, pemphigus, pernicious anemia, etc. Examples of allergic disease involve acute or chronic reactive airway disease, bronchial asthma, atopic dermatitis, allergic rhinitis, urticaria, PIE syndrome, food allergy, hay fever, allergic nose, bronchial asthma, atopic dermatitis, anaphylactic shock.

The present disclosure also provides the use of the pharmaceutical composition or the lyophilized formulation or the reconstitution solution of the lyophilized formulation described above in the preparation of a medicament for treating or inhibiting diseases or disorders related to proliferation or metastasis of tumor cells.

The present disclosure further provides a method for diagnosing or treating diseases related to human TIM-3 positive cells, the method comprises using the pharmaceutical composition or the lyophilized formulation or the reconstitution solution of the lyophilized formulation described above.

As is well known to those skilled in the art, one, some or all features of the various embodiments described in the present disclosure can be further combined to form other embodiments in the present disclosure. The above embodiments of the present disclosure and other embodiments obtained by combination would be further illustrated by the following detailed description.

### DESCRIPTION OF THE DRAWINGS

Figure 1: The curve of cell binding test for candidate TIM-3 antibodies; the data show that the candidate antibodies mAb-1701 and mAb-1799 have strong binding activity to cells expressing antigen.
Figure 2A to Figure 2C: The effect of the candidate antibodies and the antigen-binding fragment thereof of the present disclosure on IFNγ secretion, in the mixed lymphocyte reaction assay; Figure 2A, the effect of h1701-009 and the antigen-binding fragment thereof on IFNγ secretion; Figure 2B, the effect of h1701-005 and the antigen-binding fragment thereof on IFNγ secretion; Figure 2C, the effect of h1701-009, h1799-005 on IFNγ secretion. The results show that both h1701-009 and h1799-005 can effectively stimulate the secretion of IFNγ.
Figure 3A to Figure 3D: Diagrams for PBMC activation test results; Figures 3A to 3D show the proportion of IFNγ positive cells in FACS analysis, the geometric mean of IFNγ in all cells, the proportion of IFNγ and TIM-3 double positive cells (TIM-3+IFNγ+%) and the geometric mean of IFNγ in TIM-3+ cells, respectively; the results show that h1701-009, h1799-005 and AbTIM-3 all have effect on the expression of IFNγ in PBMC cells upon activation, and increase the percentage of intracellular IFNγ positive cells and the expression of IFNγ, to various degrees.
Figure 4A to Figure 4B: Diagrams for SEB stimulation test results, Figure 4A and Figure 4B show the ELISA test results for IL12 and IFNγ; the results show that h1701-009, h1799-005 and AbTIM-3 effectively increased SEB-induced secretion of IL12 and IFNγ, after stimulation of PBMC by SEB for 5 days.
Figure 5: The model fitting results for the components of TIM3 antibody formulation of the present disclosure, by using JMP software.

### DETAILED DESCRIPTION OF THE DISCLOSURE

### Detailed description of terms

In order to make the present disclosure easier to be understood, certain technical and scientific terms are specifically defined below. Unless clearly defined otherwise herein, all other technical and scientific terms used herein have meanings commonly understood by those of ordinary skill in the art to which this disclosure pertains.

The present disclosure incorporates all the contents of application PCT/CN2018/077190 herein by reference.

"Buffer" refers to a buffer that is resistant to changes in pH due to its conjugate acid-base component. Examples of the buffer which controls the pH within an appropriate range involve acetate buffer, succinate buffer, gluconate buffer, histidine salt buffer, oxalate buffer, lactate buffer, phosphate buffer, citrate buffer, tartrate buffer, fumarate buffer, glycyl glycine and other organic acid buffers.

"Histidine salt buffer" refers to a buffer comprising histidine ions. Examples of histidine salt buffers involve histidine-hydrochloride buffer, histidine-acetate buffer, histidine-phosphate buffer, histidine-sulfate buffer, etc., preferably histidine-acetate buffer or histidine-hydrochloric acid buffer; histidine-acetate buffer is prepared by histidine and acetate acid; histidine-hydrochloric acid buffer is prepared by histidine and hydrochloric acid.

"Citrate buffer" refers to a buffer that comprises citrate ions. Examples of the citrate buffer involve citric acid-sodium citrate buffer, citric acid-potassium citrate buffer, citric acid-calcium citrate buffer, citric acid-magnesium citrate buffer, etc. A preferred citrate buffer is citric acid-sodium citrate.

"Succinate buffer" refers to a buffer that comprises succinate ions. Examples of the succinate buffer involve succinic acid-sodium succinate buffer, succinic acid-potassium succinate buffer, succinic acid-calcium succinate buffer, etc. A preferred succinate buffer is succinic acid-sodium succinate.

"Phosphate buffer" refers to a buffer that comprises phosphate ions. Examples of the phosphate buffer involve disodium hydrogen phosphate-sodium dihydrogen phosphate buffer, disodium hydrogen phosphate-potassium dihydrogen phosphate buffer, disodium hydrogen phosphate-citric acid buffer etc. A preferred phosphate buffer is disodium hydrogen phosphate-sodium dihydrogen phosphate.

"Acetate buffer" refers to a buffer that comprises acetate ions. Examples of the acetate buffer involve acetic acid-sodium acetate buffer, acetic acid-histidine salt buffer, acetic acid-potassium acetate buffer, acetic acid-calcium acetate buffer, acetic acid-magnesium acetate buffer, etc. A preferred acetate buffer is acetic acid-sodium acetate.

"Pharmaceutical composition" refers to a mixture comprising one or more compounds described herein or the physiologically/pharmaceutically acceptable salt or the prodrug thereof and other chemical components, wherein the other chemical components are, for example, physiological/pharmaceutically acceptable carriers and excipients. The purpose of the pharmaceutical composition is to maintain the stability of antibody active component, promote the administration to the organism, facilitate the absorption of the active component, and thereby exert biological activity.

As used herein, "pharmaceutical composition" and "formulation" are not mutually exclusive.

With respect to the solution form of the pharmaceutical composition in the present disclosure, unless otherwise specified, the solvent included therein is water.

"Lyophilized formulation" refers to a formulation or pharmaceutical composition obtained by vacuum freeze-drying the liquid form of or solution form of the pharmaceutical composition or formulation.

As used herein, the term "about" refers to a value that is within an acceptable error range for a particular value as determined by one of ordinary skill in the art, which will depend partially on how the value is measured or determined (i.e., the limitation of the measurement system). For example, "about" can indicate a standard deviation within 1 or more than 1 for each practice in the art. Alternatively, "about" or "comprising essentially of" means a range of ± up to 20% from the indicated value that follows. Furthermore, particularly with respect to biological system or process, the term can refer to up to an order of magnitude or up to 5-fold of a value. When applied to particular values mentioned in the application and claims, unless otherwise stated clearly, the meaning of "about" or "comprising essentially of" should be assumed to be within an acceptable error range for that particular value.

The pharmaceutical composition of the present disclosure is capable of achieving a stable effect: the antibody included in the pharmaceutical composition can substantially maintain its physical stability and/or chemical stability and/or biological activity after storage; preferably, the pharmaceutical composition substantially maintains its physical stability, and chemical stability and biological activity after storage. The shelf life is generally determined based on the predetermined shelf life of the pharmaceutical composition. There are currently a number of analytical techniques for measuring protein stability, which can be used to measure the stability after storage for a given period of time at a given temperature.

A stable pharmaceutical formulation of antibody is the one in which no significant change is observed in the following conditions: storage at a refrigerating temperature (2-8°C) for at least 3 months, preferably 6 months, more preferably 1 year, and even more preferably up to 2 years. In addition, the stable liquid formulation comprises such liquid formulation which exhibits desired characteristics upon storage for example, at a temperature of 25°C for a period of 1 month, 3 months, and 6 months. Typically, acceptable criteria for the stability are as follows: typically, no more than about 10%, preferably no more than about 5% of antibody monomers are degraded, as assessed by SEC-HPLC. The pharmaceutical formulation of antibody is light yellow, near colorless, and clear liquid, or colorless, or clear to slightly milk white, by visual analysis. The change in concentration, pH and osmolality of the formulation is no more than ±10%. Typically, a reduction of no more than about 10%, preferably no more than about 5% is observed. Typically, no more than about 10%, preferably no more than about 5% is aggregated.

An antibody is considered to "maintain its physical stability" in a pharmaceutical formulation, if it shows no significant increase of aggregation, precipitation and/or denaturation upon visual examination of color and/or clarity, or as measured by UV light scattering, size exclusion chromatography (SEC) and dynamic light scattering (DLS). The change of protein conformation can be evaluated by fluorescence spectroscopy (which determines the protein tertiary structure), and by FTIR spectroscopy (which determines the protein secondary structure).

An antibody is considered to "retain its chemical stability" in a pharmaceutical formulation, if it shows no significant chemical alteration. Chemical stability can be assessed by detecting and quantifying chemically altered form of the protein. Degradation processes that often alter the chemical structure of a protein comprise hydrolysis or truncation (evaluated by methods such as size exclusion chromatography and SDS-PAGE), oxidation (evaluated by methods such as peptide mapping in conjunction with mass spectroscopy or MALDI/TOF/MS), deamidation (evaluated by methods such as ionexchange chromatography, capillary isoelectric focusing, peptide mapping, isoaspartic acid measurement), and isomerization (evaluated by measuring the isoaspartic acid content, peptide mapping, etc.).

An antibody is considered to "retain its biological activity" in a pharmaceutical formulation, if the biological activity of the antibody at a given time is still within a predetermined range of the biological activity exhibited at the time when the pharmaceutical preparation was prepared. The biological activity of an antibody can be determined, for example, by an antigen binding test.

The three letter codes and single-letter codes for the amino acid used in present disclosure are described in J. Biol. Chem. 243, p. 3558 (1968).

The "antibody" as used in the present disclosure refers to an immunoglobulin, which is a tetra-peptide chain structure connected together by inter-chain disulfide bonds between two identical heavy chains and two identical light chains.

In the present disclosure, the antibody light chain of the present disclosure can further comprise a light chain constant region comprising human or murine κ, λ chain or variant thereof.

In the present disclosure, the antibody heavy chain of the present disclosure can further comprise a heavy chain constant region comprising human or murine IgG1, IgG2, IgG3, IgG4 or variant thereof.

About 110 amino acid sequences adjacent to the N-terminus of the antibody heavy and light chains are highly variable, known as variable regions (Fv regions); the rest of amino acid sequences close to the C-terminus are relatively stable, known as constant regions. The variable region includes three hypervariable regions (HVRs) and four relatively conserved framework regions (FRs). The three hypervariable regions which determine the specificity of the antibody are also known as the complementarity determining regions (CDRs). Each light chain variable region (LCVR) and each heavy chain variable region (HCVR) consists of three CDR regions and four FR regions, with sequential order from the amino terminus to carboxyl terminus in the following order: FR1, CDR1, FR2, CDR2, FR3, CDR3, and FR4. The three CDR regions of the light chain refer to LCDR1, LCDR2, and LCDR3, and the three CDR regions of the heavy chain refer to HCDR1, HCDR2, and HCDR3. The number and position of the CDR amino acid residues in the LCVR region and the HCVR region of the antibody or the antigen-binding fragment thereof described in the present disclosure comply with the known Kabat numbering criteria (LCDR1-3, HCDR1-3).

The antibody of the present disclosure involves murine antibodies, chimeric antibodies, humanized antibodies, preferably humanized antibodies.

The "antibody or antigen binding thereof" or "functional fragment" mentioned in the present disclosure refers to Fab fragment, Fab' fragment, F(ab') 2 fragment, and Fv fragment and scFv fragment that binds to antigen. The Fv fragment comprises antibody heavy chain variable region and light chain variable region, but does not have the constant region, and is known as the smallest antibody fragment having all antigen binding sites. Generally, Fv antibodies also comprise a polypeptide linker between the VH and VL domains, to form a structure required for antigen binding. Different linkers can also be used to connect two antibody variable regions to form a single polypeptide chain, which is referred to as single chain antibody or single chain Fv (sFv).

The term "antigen-binding site" in the present disclosure refers to a continuous or discontinuous three-dimensional spatial site on an antigen that can be recognized by the antibody or antigen-binding fragment thereof of the present disclosure.

The term "murine antibody" in the present disclosure refers to a monoclonal antibody against human TIM3 prepared according to the knowledge and skills of the field. During the preparation, a test subject is injected with TIM3 antigen, and then a hybridoma expressing the antibody having the desired sequence or functional properties is separated.

The term "chimeric antibody" is an antibody which is formed by fusing the variable region of a murine antibody with the constant region of a human antibody, and the chimeric antibody can alleviate the immune response that is induced by murine antibody. To construct a chimeric antibody, a hybridoma that secretes a specific murine monoclonal antibody is constructed, and then variable region genes are cloned from the mouse hybridoma cells. Subsequently, constant region genes of human antibody are cloned as desired. The murine variable region gene is ligated with the human constant region gene to form a chimeric gene which can be inserted into a human vector, and finally a chimeric antibody molecule is expressed in the eukaryotic or prokaryotic industrial system. In a preferred embodiment of the present disclosure, the light chain of the TIM3 chimeric antibody further comprises the light chain constant region(s) of human κ, λ chain, or variant thereof. The heavy chain of the TIM3 chimeric antibody further comprises the heavy chain constant region(s) of human IgG1, IgG2, IgG3, IgG4, or variant thereof. The constant region of a human antibody can be selected from the group consisting of the heavy chain constant region of human IgG1, IgG2, IgG3 or IgG4 or variant thereof, preferably human IgG2 or IgG4 heavy chain constant region, or IgG4 without ADCC toxicity (antibody-dependent cell-mediated cytotoxicity) obtained after amino acid mutation.

The term "humanized antibody", also known as CDR-grafted antibody, refers to an antibody generated by grafting murine CDR sequences into a variable region framework of human antibody, namely an antibody produced from different types of human germline antibody framework sequences. A humanized antibody overcomes disadvantage of the strong heterogenous response induced by the chimeric antibody, which carries large amount of murine protein components. Such framework sequences can be obtained from a public DNA database covering germline antibody gene sequences or published references. For example, germline DNA sequences of human heavy and light chain variable region genes can be found in "VBase" human germline sequence database (available on web www.mrccpe.com.ac.uk/vbase), as well as can be found in Kabat, E A, et al, 1991 Sequences of Proteins of Immunological Interest, 5th Ed. To avoid the decrease in activity along with the decrease in immunogenicity, the framework sequences in the variable region of human antibody are subjected to minimal reverse mutations or back mutations to maintain the activity. The humanized antibody of the present disclosure also comprises humanized antibody on which CDR affinity maturation is performed by phage display.

The term "ADCC" (antibody-dependent cell-mediated cytotoxicity) refers to direct killing of target cells coated with an antibody by cells expressing Fc receptor through recognizing the Fc segment of the antibody. ADCC effector function of the antibody can be reduced or eliminated via modification on Fc segment of IgG. The modification refers to mutations in antibody heavy chain constant region, such as mutations selected from the group consisting of N297A, L234A, L235A in IgG1; IgG2/4 chimera; and F234A/L235A mutations in IgG4.

The "mutation" in mutant sequence described in the present disclosure includes but is not limited to "back mutation", "conservative modification" or "conservative replacement or substitution". The "conservative modification" or "conservative replacement or substitution" used in the present disclosure means the substitution of the amino acids in a protein with other amino acids showing similar characteristics (such as charge, side chain size, hydrophobicity/hydrophilicity, conformation of main chain and rigidity, etc), such substitutions can be frequently performed without changing the biological activity of the protein. Those skilled in the art know that, generally speaking, a single amino acid substitution in a non-essential region of a polypeptide does not substantially change the biological activity (see for example, Watson et al. (1987) Molecular Biology of the Gene, The Benjamin/Cummings Pub. Co., Page 224 (4th edition)). In addition, the substitutions with amino acids having similar structure or function are unlikely to disrupt biological activity.

The "mutated sequence" described in the present disclosure refers to that, the nucleotide sequence and/or amino acid sequence of the present disclosure is subjected to appropriate modification by mutation (substitution, insertion or deletion etc), and the obtained nucleotide sequence and/or amino acid sequence has different percent sequence identity to the nucleotide sequence and/or amino acid sequence of the present disclosure. The sequence identity described in the present disclosure can be at least 85%, 90% or 95%, preferably at least 95%. Non-limiting examples include 85%, 86%, 87%, 88%, 89%, 90%, 91%, 92%, 93%, 94%, 95%, 96%, 97%, 98%, 99%, 100%. Sequence comparison and determination of percent identity between two sequences can be performed by BLASTN/BLASTP algorithm available on the National Center For Biotechnology Institute website, with default settings.

The term "TIM3" is an immunoglobulin widely expressed on the surface of cell membrane, also known as integrin-related protein. Signal regulatory protein (SIRP) is a member of the inhibitory receptor superfamily and belongs to the immunoglobulin superfamily. It is mainly expressed on the surface of macrophages, dendritic cells and nerve cells. Upon contacting with ligands on the cell surface, SIRP regulates cell migration and phagocytic activity, immune homeostasis and neuronal network. TIM3 is an extracellular ligand of human signal regulatory protein α (SIRPα). It reduces phagocytic activity by binding to SIRPα on the surface of macrophages to deliver inhibitory signals, thereby inhibiting the innate immune system. This signal is vividly described as "don't eat me" signal.

The term "binding to TIM3" refers to the ability to interact with human TIM3.

The terms "anti-TIM3 antibody" and "TIM3 antibody" can be used interchangeably, and both refer to the antibody that binds to TIM3.

The fusion protein described in the present disclosure is a protein product obtained by co-expressing two genes through DNA recombination. Recombinant TIM3 extracellular region-Fc fusion protein is a fusion protein obtained by co-expressing TIM3 extracellular region and Fc fragment through DNA recombination. The TIM3 extracellular region refers to TIM3 protein expressed outside the cell membrane, the sequence is shown in SEQ ID NO:1.

Methods for producing and purifying antibodies and antigen-binding fragments are well known in the art and can be found, for example, in Antibody Experimental Technology Guide of Cold Spring Harbor, Chapters 5-8 and 15. Mice can be immunized with human TIM3 or the fragment thereof, and the obtained antibodies can be renatured and purified, and the amino acid sequencing can be performed by conventional methods. Antigen-binding fragments can also be prepared by conventional methods. The antibodies or the antigen-binding fragments thereof of the present disclosure are genetically engineered to add one or more human FR region(s) to non-human derived CDR region(s). Human FR germline sequences can be obtained from ImMunoGeneTics (IMGT) website http://imgt.cines.fr, or from The Immunoglobulin FactsBook, 2001ISBN012441351.

The engineered antibodies or antigen-binding fragments thereof of the present disclosure can be prepared and purified by conventional methods. For example, cDNA sequences encoding a heavy chain and a light chain can be cloned and recombined into a GS expression vector. The recombined immunoglobulin expression vector can then be stably transfected into CHO cells. As a more recommended method well known in the art, mammalian expression systems will result in glycosylation of antibodies, typically at the highly conserved N-terminus in the Fc region. Stable clones can be obtained through expression of an antibody specifically binding to human TIM3. Positive clones can be expanded in serum-free culture medium for antibody production in bioreactors. Culture medium, into which an antibody has been secreted, can be purified by conventional techniques. For example, the purification can be performed by A or G Sepharose FF column that has been equilibrated with adjusted buffer. The column is washed to remove nonspecific binding components. The bound antibody is eluted by pH gradient and antibody fragments are detected by SDS-PAGE, and then collected. The antibody can be filtered and concentrated using common techniques. Soluble mixture and multimers can also be removed by common techniques, such as molecular sieve, ion exchange. The obtained product can be immediately frozen, for example at -70°C, or can be lyophilized.

"Optional" or "optionally" means that the described event or situation that follows can but does not necessarily occur, and the description includes occasions where the event or situation does or does not occur. For example, "optionally, comprising 1-3 antibody heavy chain variable regions" means that the antibody heavy chain variable region with specific sequence can but need not be present.

"Administration" and "treatment", when applying to an animal, human, experimental subject, cell, tissue, organ, or biological fluid, refers to contacting an exogenous pharmaceutical, therapeutic, diagnostic agent, or composition with the animal, human, subject, cell, tissue, organ, or biological fluid. "Administration" and "treatment" can refer, e.g., to therapeutic, pharmacokinetic, diagnostic, research, and experimental methods. Treatment of a cell encompasses contacting a reagent with the cell, as well as contacting a reagent with a fluid, where the fluid is in contact with the cell. "Administration" and "treatment" also mean *in vitro* and *ex vivo* treatments, e.g., of a cell, by a reagent, diagnostic, binding composition, or by another cell. "Treatment", as it applies to a human, veterinary, or a research subject, refers to therapeutic treatment, prophylactic or preventative measures, research and diagnostic applications.

"Treat" means to administer a therapeutic agent, such as a composition comprising any of the binding compounds of the present disclosure, internally or externally to a patient having one or more disease symptoms for which the agent has known therapeutic activity. Typically, the therapeutic agent is administered in an amount effective to alleviate one or more disease symptoms in the treated patient or population, so as to induce the regression or inhibit the progression of such symptom(s) to any clinically measurable degree. The amount of a therapeutic agent that is effective to alleviate any particular disease symptom (also referred to "therapeutically effective amount") can vary according to factors such as the disease state, age, and weight of the patient, and the ability of the agent to elicit a desired response in the patient. Whether a disease symptom has been alleviated can be assessed by any clinical measurement typically used by physicians or other skilled healthcare providers to assess the severity or progression status of that symptom. While an embodiment of the present disclosure (e.g., a treatment method or an article of manufacture) cannot be effective in alleviating each disease symptom of interest, it should alleviate the target disease symptom(s) of interest in a statistically significant number of patients as determined by any statistical test known in the art such as the Student's t-test, the chi-square test, the U-test according to Mann and Whitney, the Kruskal-Wallis test (H-test), Jonckheere-Terpstra-test and the Wilcoxon-test.

The formulations of the present disclosure can be used to treat disease related to TIM-3 positive cells. There is no restriction on the disease related to TIM-3 positive cells, as long as it is a disease related to TIM-3-expressing cells, such as cancer, autoimmune disease and allergic disease. The cancer involves blood cancer, breast cancer, uterine cancer, colorectal cancer, esophageal cancer, gastric cancer, ovarian cancer, lung cancer, kidney cancer, rectal cancer, thyroid cancer, cervical cancer, small intestine cancer, prostate cancer and pancreatic cancer. Preferable examples of cancer involve blood cancer, esophageal cancer, gastric cancer, colorectal cancer, liver cancer and prostate cancer. Examples of blood cancers involve acute myeloid leukemia (AML), chronic myeloid leukemia (CML), myelodysplastic syndrome (MDS), multiple myeloma, cutaneous T cell lymphoma (CTCL), peripheral T cell lymphoma (PTCL), anaplastic large cell lymphoma (ALCL), acute lymphocytic leukemia (ALL), chronic lymphocytic leukemia (CLL), other lymphocytic leukemia, NK cell lymphoma, Hodgkin's lymphoma, Non-Hodgkin's lymphoma (such as Burkitt lymphoma), and so on. Particular examples of autoimmune disease involve rheumatoid arthritis, psoriasis, Crohn's disease, ankylosing spondylitis, multiple sclerosis, diabetes type I, hepatitis, myocarditis, Sjogren syndrome, autoimmune hemolysis anemia after transplant rejection, vesicular pemphigoid, Grave's disease, Hashimoto's thyroiditis, systemic lupus erythematosus (SLE), myasthenia gravis, pemphigus, pernicious anemia, etc. Examples of allergic disease involve acute or chronic reactive airway disease, bronchial asthma, atopic dermatitis, allergic rhinitis, urticaria, PIE syndrome, food allergy, hay fever, allergic nose, bronchial asthma, atopic dermatitis, anaphylactic shock, etc.

"Effective amount" encompasses an amount sufficient to ameliorate or prevent a symptom or sign of a medical condition. Effective amount also means an amount sufficient to allow or facilitate diagnosis. An effective amount for a particular patient or veterinary subject can vary depending on factors such as the condition being treated, the general health of the patient, the route and dose of administration and the severity of side effects. An effective amount can be the maximal dose or dosing protocol that avoids significant side effects or toxic effects.

"Replacement" refers to the replacement of the solvent system that dissolves the antibody protein. For example, the high salt or hypertonic solvent system comprising the antibody protein is replaced by physical manipulation using a buffer system for stable formulation, so that the antibody protein exists in the stable formulation. The physical manipulation involves but is not limited to ultrafiltration, dialysis or reconstitution after centrifugation.

"Pharmaceutical composition" means a mixture comprising one or more compounds described herein or the physiologically/pharmaceutically acceptable salt or prodrug thereof as well as other chemical components, such as physiologically/pharmaceutically acceptable carrier and excipient. The purpose of the pharmaceutical composition is to promote the administration to the organism, facilitate the absorption of the active component and then exert the biological activity.

Exemplary preparation process for antibody pharmaceutical composition (formulation):
Step 1: a certain amount of purified TIM3 antibody solution was subjected to solvent replacement (preferably by ultrafiltration) against a buffer without antibody (such as 10mM, pH 5.5 histidine-acetic acid buffer), the replacement is performed by passing through ultrafiltration membrane with at least 6-fold volume; the protein was concentrated to about 70 mg/mL. A certain volume of sucrose stock solution was added, mixed to obtain a final concentration of sucrose at 80 mg/mL. A certain volume of polysorbate 80 stock solution was added, mixed to obtain a final concentration of polysorbate 80 at 0.4 mg/mL. 10mM pH 5.5 histidine-acetic acid buffer was added to adjust the volume, to make the protein concentration at 50mg/mL (other formulations or stable formulations to be tested were prepared according to similar steps).
   The product was filtered and tested for sterility, by in-process control sampling. The stock solution passed through a 0,22µm PVDF filter and the filtrate was collected.
Step 2: the loading volume was adjusted to 2.15 ml, the filtrate was loaded into a 2 ml stoppered vial; and the volume differences were tested by sampling at the beginning of, during and at the end of the loading procedure, respectively.
Step 3: an aluminum cap was capped by using a capping machine.
Step 4: a visual inspection was performed to confirm whether there is any defect, such as inaccurate loading. A label was printed and applied onto the vial; a label was printed for a paper tray, the paper tray was folded and the vials were placed into the paper tray, and a label was applied onto the paper tray.

Hereinafter, the present disclosure would be further described with reference to examples, however the scope of the present disclosure is not limited thereto. In the examples of the present disclosure, where specific conditions are not described, the experiments are generally conducted under conventional conditions as described in, such as Antibody Experimental Technology Guide and Molecular Cloning, by Cold Spring Harbor; or under conditions proposed by the manufacturers of material or product. Where the source of the reagents is not specifically indicated, the reagents are commercially available conventional reagents.

### Example 1. Preparation of TIM3 antibody

### I. Preparation of TIM-3 antigen and protein used for detection

### 1. Design and expression of TIM-3 antigen

UniProt Hepatitis A virus cellular receptor 2 (human HAVCR2, human TIM-3, Uniprot No: Q8TDQ0) was used as the template of TIM-3 of the present disclosure, the amino acid sequence of the antigen and protein used for detection in the present disclosure were designed, optionally different tags were fused with TIM-3 protein, the fusion was cloned into pHr vector (house-made) or pTargeT vector (promega, A1410), the vector was transiently expressed in 293 cells or stably expressed in CHO-S, and then purified to obtain the encoded antigen and protein used for detection in the present disclosure. Otherwise indicated specifically, the TIM-3 antigen mentioned hereafter refers to human TIM-3.

Fusion protein of TIM-3 extracellular region and hIgG1 Fc: TIM-3-Fc (SEQ ID NO: 1), used for immunization of mouse:

MEFGLSWLFLVAILKGVQCSEVEYRAEVGQNAYLPCFYTPAAPGNLVPVCWGK
GACPVFECGNVVLRTDERDVNYWTSRYWLNGDFRKGDVSLTIENVTLADSGIY
CCRIQIPGIMNDEKFNLKLVIKPAKVTPAPTRQRDFTAAFPRMLTTRGHGPAETQT
LGSLPDINLTQISTLANELRDSRLANDLRDSGATIREPKSSDKTHTCPPCPAPELLG

GPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYT
LPPSRDELTKNQVSLTCLVKGFYPSDIAVEWESNGQPENNYKTTPPVLDSDGSFFLYS
KLTVDKSRWQQGNVFSCSVMHEALHNHYTQKSLSLSPGK;

Note: The underlined part represents signal peptide, and the italicized part represents Fc.

TIM-3 Extracellular region with Flag and His tags: TIM-3-Flag-His (SEQ ID NO: 2), used for detection:

MEFGLSWLFLVAILKGVQCSEVEYRAEVGQNAYLPCFYTPAAPGNLVPVCWGK
GACPVFECGNVVLRTDERDVNYWTSRYWLNGDFRKGDVSLTIENVTLADSGIY
CCRIQIPGIMNDEKFNLKLVIKPAKVTPAPTRQRDFTAAFPRMLTTRGHGPAETQT
LGSLPDINLTQISTLANELRDSRLANDLRDSGATIRGSSDYKDDDDKHHHHHH;

Note: The underlined part represents signal peptide, and the italicized part represents Flag-His tag.

Full-length amino acid sequence for human TIM-3: used to construct TIM-3-overexpressing cell lines, TIM-3-full length (SEQ ID NO: 3):

MFSHLPFDCVLLLLLLLLLTRSSEVEYRAEVGQNAYLPCFYTPAAPGNLVPVCWG
KGACPVFECGNVVLRTDERDVNYWTSRYWLNGDFRKGDVSLTIENVTLADSGI
YCCRIQIPGIMNDEKFNLKLVIKPAKVTPAPTRQRDFTAAFPRMLTTRGHGPAET
QTLGSLPDINLTQISTLANELRDSRLANDLRDSGATIRIGIYIGAGICAGLALALIF
GALIFKWYSHSKEKIQNLSLISLANLPPSGLANAVAEGIRSEENIYTIEENVYEVEE
PNEYYCYVSSRQQPSQPLGCRFAMP;

Note: the underlined part represents the signal peptide, the double-underlined part represents the transmembrane region, the wavy line represents the intracellular region, and the other part represents the extracellular region of TIM-3.

### 2. Purification of TIM-3 related recombinant protein, and purification of hybridoma antibodies and recombinant antibodies

### 2.1 Purification steps of TIM-3-Flag-His recombinant protein:

The sample was centrifuged at high speed to remove impurities and concentrated to an appropriate volume. The NI-NTA affinity column (QIAGEN, Cat No. 30721) was equilibrated with PBS, and was washed with 2-5 column volumes. After removing the impurities, the supernatant sample expressed by cells was loaded onto the column. The column was rinsed with PBS until the A280 reading dropped to baseline. The column was rinsed with PBS to wash undesired proteins, and the target protein was collected. The target protein was eluted with washing buffer (20mM imidazole) and elution buffer (300mM imidazole) successively, and the elution peaks were collected.

The collected eluate was further purified by ion exchange (Hiload 16/600 Superdex 200 column). The column was equilibrated with about 2 column volumes of PBS to ensure pH 7.4. The identified elution buffer comprising the target protein was concentrated, and then loaded onto the column. The sample was collected, identified by using SDS-PAGE and LC-MS and aliquoted for later use.

### 2.2 Purification of hybridomas, recombinant antibodies, and Fc fusion proteins

The supernatant sample expressed by cells was centrifuged at high speed to remove impurities, the supernatant expressed by hybridoma was purified by Protein G column, and supernatants expressed by recombinant antibody and Fc fusion protein were purified by Protein A column. The column was rinsed with PBS until the A280 reading dropped to baseline. The target protein was eluted with 100mM acetic acid pH3.0, and neutralized with 1M Tris-HCl pH8.0. The eluted sample was appropriately concentrated and further purified by PBS-equilibrated gel chromatography Superdex 200 (GE). The peaks (aggregates were removed) were collected and aliquoted for later use.

### II. Preparation of anti-human TIM-3 monoclonal antibody

### 1. Animal immunization

Anti-human TIM-3 monoclonal antibody was produced by immunizing mice. Laboratory SJL white mice, female, 6-8 weeks old (Beijing Charles River Laboratory Animal Technology Co., Ltd., animal production license number: SCXK (Beijing) 2012-0001) were used. Feeding environment: SPF level. After the mice were purchased, they were adapted to the laboratory environment for 1 week, 12/12 hours light/dark cycle adjustment, temperature 20-25°C; humidity 40-60%. Mice that have been adapted to the environment were immunized according to the following protocol. The antigen for immunization is the extracellular region of human TIM-3 with Fc-tag (SEQ ID NO: 1).

Immunization protocol: QuickAntibody-Mouse5W (KX0210041) was used to immunize mice. The ratio of antigen to adjuvant is 1:1, 10µg/mouse/time (first immunization/booster immunization). The antigen and adjuvant were quickly and thoroughly mixed and then inoculated. The inoculation period involved an interval of 21 days between the first and second immunizations, and the interval between later immunizations was 14 days. Blood was taken 7 days after each immunization, and the antibody titer in the mouse serum was determined by ELISA. The mice with high antibody titer in serum and with its titer reaching to the plateau were selected for splenocyte fusion. Three days before the fusion of splenocytes, the booster immunization was performed by intraperitoneally (IP) injecting 20 µg/mouse of antigen solution prepared with physiological saline .

### 2. Splenocyte fusion

Optimized PEG-mediated fusion steps were used to fuse splenic lymphocytes with myeloma Sp2/0 cells (ATCC® CRL-8287™) to obtain hybridoma cells. The fused hybridoma cells were re-suspended in complete medium (DMEM medium comprising 20% FBS, 1×HAT and 1×OPI) at a density of 4 × 10⁵ to 5 × 10⁵/ml, and were seeded onto a 96-well plate at 100 µl/well, and incubated at 37°C in 5% CO₂ for 3-4 days, and then HAT complete medium was added at 100µl/well to further cultivate the cells for 3-4 days until pinpoint-like clones were formed. The supernatant was removed, 200µl/well of HT complete medium (RPMI-1640 medium comprising 20% FBS, 1×HT and 1×OPI) was added, and incubated at 37°C in 5% CO₂ for 3 days, and then ELISA detection was performed.

### 3. Screening of hybridoma cell

According to the growth density of hybridoma cells, the hybridoma culture supernatant was detected by binding ELISA method (see Test Example 1). TIM-3 overexpressing cell binding test was performed using cell supernatant in positive wells which were identified in the ELISA method (see Test Example 2). The cells in wells that are positive for both protein-binding and cell-binding should be expanded in time for cryopreservation, and subcloned two to three times until single cell clone can be obtained.

TIM-3 binding ELISA and cell binding tests were performed for each sub-cloned cells. The hybridoma clones were screened through the above tests, and the secreted antibodies mAb-1701 and mAb-1799 were obtained. The antibodies were further prepared by the serum-free cell culture method. The antibodies were purified according to the purification example, and were provided for use in the test examples.

### 4. Sequencing of hybridoma positive clones

The process for cloning the sequences from the positive hybridoma was as follows. The hybridoma cells at logarithmic growth phase were collected, RNA was extracted by Trizol (Invitrogen, Cat No. 15596-018) according to the kit instructions, and PrimeScript™ Reverse Transcriptase kit was used for reverse transcription (Takara, Cat No. 2680A). The cDNA obtained by reverse transcription was amplified by PCR using mouse Ig-Primer Set (Novagen, TB326 Rev. B 0503) and was delivered to company for sequencing. The amino acid sequences corresponding to the DNA sequences of heavy chain and light chain variable region(s) of mAb-1701 and mAb-1799 were obtained:
mAb-1701 heavy chain variable region (SEQ ID NO: 4) 
EVQLQQSGPELVKPGASVKISCKASGYTFTDYYMNWVKQSHGKSLEWIADIIPN
NGGSKYNQKFKDKATLTVDKSSSTAYMELRSLTSEDSAVYYCATWGYGSSYRW
FDYWGQGTLVSVSA;
mAb-1701 light chain variable region (SEQ ID NO: 5) 
DIQMTQSPASQSASLGESVTITCLASQPIGIWLAWYQQKPGKSPQLLIYAATSLAD
GVPSRFSGSGSGTKFSFKISSLQAEDFVSYYCQQLYSSPWTFGGGTKLEIK;
mAb-1799 heavy chain variable region (SEQ ID NO: 6) 
EVKLVESEGGLVQPGSSMKLSCTASGFTFSDYYMAWVRQVPEKGLEWVANINY
DGSSTYYLDSLKSRFIISRDNAKNILYLQMNSLKSDDTATYYCARDVGYYGGNY
GFAYWGQGTLVTVSA;
mAb-1799 light chain variable region (SEQ ID NO: 7)

DIQMTQSPASLSASVGETVTITCRASDNIYSYLAWYQQKQGKSPQLLVYNAKTL
AEGVPSRFSGSGSGTQFSLKINSLQPEDFGSYYCQQHYGSPLTFGAGTKLELK;

In the light and heavy chain variable region sequences of mAb-1701 and mAb-1799 described above, the underlined part represents the CDR region, and the arrangement for each sequence is FR1-CDR1-FR2-CDR2-FR3-CDR3-FR4. The CDR sequences in the light and heavy chains of each antibody are shown in Table 1.

**Table 1. Sequences of CDR regions for each heavy chain and light chain**

| Ab | Heavy chain | | Light chain | |
|---|---|---|---|---|
| mAb - 1701 | HCDR1 | DYYMN SEQ ID NO: 8 | LCDR1 | LASQPIGIWLA SEQ ID NO: 11 |
| | HCDR2 | DIIPNNGGSKYNQKFKD SEQ ID NO: 9 | LCDR2 | AATSLAD SEQ ID NO: 12 |
| | HCDR3 | WGYGSSYRWFDY SEQ ID NO: 10 | LCDR3 | QQLYSSPWT SEQ ID NO: 13 |
| mAb - 1799 | HCDR1 | DYYMA SEQ ID NO: 14 | LCDR1 | RASDNIYSYLA SEQ ID NO: 17 |
| | HCDR2 | NINYDGSSTYYLDSLKS SEQ ID NO: 15 | LCDR2 | NAKTLAE SEQ ID NO: 18 |
| | HCDR3 | DVGYYGGNYGFAY SEQ ID NO: 16 | LCDR3 | QQHYGSPLT SEQ ID NO: 19 |

### III. Humanization of anti-human TIM-3 murine hybridoma monoclonal antibody

### 1. Humanization of anti-TIM-3 antibody mAb-1701

By aligning against IMGT germline gene database of human antibody heavy and light chain variable region by MOE software, the heavy chain and light chain variable region germline genes with high homology to mAb-1701 antibody were separately selected as templates, and the CDRs of murine antibody were respectively grafted onto the corresponding human template to form the variable region sequence in the order of FR1-CDR1-FR2-CDR2-FR3-CDR3-FR4. The amino acid residues were identified and annotated by Kabat numbering system.

### 1.1 Selection of framework for humanized hybridoma clone mAb-1701

The light chain templates for humanization of the murine antibody mAb-1701 were IGKV1-33*01 and hjk4.1, and the heavy chain templates for humanization were IGHV1-18*01 and hjh4.1. The humanized variable region sequences are as follows:
h1701VH-CDR graft (SEQ ID NO: 20) 
QVQLVQSGAEVKKPGASVKVSCKASGYTFTDYYMNWVRQAPGQGLEWMGDIIPNN
GGSKYNQKFKDRVTMTTDTSTSTAYMELRSLRSDDTAVYYCARWGYGSSYRWFDY
WGQGTLVTVSS;
h1701VL-CDR graft (SEQ ID NO: 21)

DIQMTQSPSSLSASVGDRVTITCLASQPIGIWLAWYQQKPGKAPKLLIYAATSLADG
VPSRFSGSGSGTDFTFTISSLQPEDIATYYCQQLYSSPWTFGGGTKVEIK;

Note: Arranged in the order of FR1-CDR1-FR2-CDR2-FR3-CDR3-FR4, the italics in the sequence represent FR sequence, and the underlined represents CDR sequences.

### 1.2 Template selection and back-mutation(s) design for h1701

The specific mutation design is shown in Table 2 below:

**Table 2. Template selection and back-mutation(s) design for h1701**

| **h1701_VL** | | **h1701_VH** | |
|---|---|---|---|
| h1701_VL.1 | Grafted | h1701_VH.1 | Grafted |
| h1701_VL.1A | A43S | h1701_VH.1A | M48I |
| | | h1701_VH.1B | R98T |
| | | h1701_VH.1C | M48I, R98T |
| | | h1701_VH.1D | M48I, R98T, R38K, D89E |
| | | h1701_VH.1E | M48I, R98T, G49A, V68A, M70L |
| | | h1701_VH.1F | M48I, R98T, G49A, V68A, M70L, R38K, D89E |

Note: For example, A43S means that A at position 43 is mutated back to S, according to the natural numbering. "Grafted" represents the sequence of murine antibody CDRs implanted into human germline FR regions.

**Table 3. Combinations of heavy chain variable region and light chain variable region of h1701 humanized antibody**

| | **h1701**_**VL.1** | **h1701**_**VL.1A** |
|---|---|---|
| h1701_VH.1 | h1701-005 | h1701-006 |
| h1701_VH.1A | h1701-007 | h1701-008 |
| h1701_VH.1B | h1701-009 | h1701-010 |
| h1701_VH.1C | h1701-011 | h1701-012 |
| h1701_VH.1D | h1701-013 | h1701-014 |
| h1701_VH.1E | h1701-015 | h1701-016 |
| h1701_VH.1F | h1701-017 | h1701-018 |

Note: This table shows the resulting sequences of various mutation combinations. As indicated by h1701-007, the humanized murine antibody h1701-007 has two mutants (light chain h1701_VL.1A and heavy chain h1701_VH.1A). Others can be indicated in similar way.

The particular humanized sequences of 1701 are as follows:
>h1701_VH. 1 (the same as h1701VH-CDR graft, SEQ ID NO: 22) 
QVQLVQSGAEVKKPGASVKVSCKASGYTFTDYYMNWVRQAPGQGLEWMGDII
PNNGGSKYNQKFKDRVTMTTDTSTSTAYMELRSLRSDDTAVYYCARWGYGSSY
RWFDYWGQGTLVTVSS;
>h1701h1701_VH.1A (SEQ ID NO: 23) 
QVQLVQSGAEVKKPGASVKVSCKASGYTFTDYYMNWVRQAPGQGLEWIGDIIP
NNGGSKYNQKFKDRVTMTTDTSTSTAYMELRSLRSDDTAVYYCARWGYGSSYR
WFDYWGQGTLVTVSS;
>h1701_VH.1B (SEQ ID NO: 24) 
QVQLVQSGAEVKKPGASVKVSCKASGYTFTDYYMNWVRQAPGQGLEWMGDII
PNNGGSKYNQKFKDRVTMTTDTSTSTAYMELRSLRSDDTAVYYCATWGYGSSY
RWFDYWGQGTLVTVSS;
>h1701_VH.1C (SEQ ID NO: 25) 
QVQLVQSGAEVKKPGASVKVSCKASGYTFTDYYMNWVRQAPGQGLEWIGDIIP
NNGGSKYNQKFKDRVTMTTDTSTSTAYMELRSLRSDDTAVYYCATWGYGSSYR
WFDYWGQGTLVTVSS;
>h1701_VH.1D (SEQ ID NO: 26) QVQLVQSGAEVKKPGASVKVSCKASGYTFTDYYMNWVKQAPGQGLEWIGDII 
PNNGGSKYNQKFKDRVTMTTDTSTSTAYMELRSLRSEDTAVYYCATWGYGSSY
RWFDYWGQGTLVTVSS;
>h1701_VH.1E (SEQ ID NO: 27) 
QVQLVQSGAEVKKPGASVKVSCKASGYTFTDYYMNWVRQAPGQGLEWIADIIP
NNGGSKYNQKFKDRATLTTDTSTSTAYMELRSLRSDDTAVYYCATWGYGSSYR
WFDYWGQGTLVTVSS;
>h1701_VH.1F (SEQ ID NO: 28) 
QVQLVQSGAEVKKPGASVKVSCKASGYTFTDYYMNWVKQAPGQGLEWIADII
PNNGGSKYNQKFKDRATLTTDTSTSTAYMELRSLRSEDTAVYYCATWGYGSSYR
WFDYWGQGTLVTVSS;
>h1701_VL.1 (the same as h1701VL-CDR graft, SEQ ID NO: 29) 
DIQMTQSPSSLSASVGDRVTITCLASQPIGIWLAWYQQKPGKAPKLLIYAATSLA
DGVPSRFSGSGSGTDFTFTISSLQPEDIATYYCQQLYSSPWTFGGGTKVEIK;
>h1701_VL.1A (SEQ ID NO: 30)

DIQMTQSPSSLSASVGDRVTITCLASQPIGIWLAWYQQKPGKSPKLLIYAATSLAD
GVPSRFSGSGSGTDFTFTISSLQPEDIATYYCQQLYSSPWTFGGGTKVEIK.

### 2. Humanization of anti-TIM-3 antibody mAb-1799

By aligning against IMGT germline gene database of human antibody heavy and light chain variable region by MOE software, the heavy chain and light chain variable region germline genes with high homology to mAb-1799 antibody were separately selected as templates, and the CDRs of murine antibody were respectively grafted onto the corresponding human template to form the variable region sequence in the order of FR1-CDR1-FR2-CDR2-FR3-CDR3-FR4. The amino acid residues were identified and annotated by Kabat numbering system.

### 2.1 Selection of framework for humanized hybridoma clone 1799

The light chain templates for humanization of the murine antibody 1799 were IGKV1-39*01 and hjk2.1, and the heavy chain templates for humanization were IGHV3-7*01 and hjh4.1. The humanized variable region sequences are as follows:
h1799VH-CDR graft (SEQ ID NO: 31) 
EVQLVESGGGLVQPGGSLRLSCAASGFTFSDYYMAWVRQAPGKGLEWVANINYDG
SSTYYLDSLKSRFTISRDNAKNSLYLQMNSLRAEDTAVYYCARDVGYYGGNYGFAY
WGQGTLVTVSS;
h1799VL-CDR graft (SEQ ID NO: 32)

DIQMTQSPSSLSASVGDRVTITCRASDNIYSYLAWYQQKPGKAPKLLIYNAKTLAEG
VPSRFSGSGSGTDFTLTISSLQPEDFATYYCQQHYGSPLTFGQGTKLEIK;

Note: Arranged in the order of FR1-CDR1-FR2-CDR2-FR3-CDR3-FR4, the italics in the sequence represent FR sequence, and the underlined represents CDR sequences.

### 2.2 Template selection and back-mutation(s) design for hybridoma clone 1799, see Table 4 below:

**Table 4. Template selection and back-mutation(s) design for h1799**

| **h1799**_**VL** | | **h1799**_**VH** | |
|---|---|---|---|
| h1799_VL.1 | Grafted | h1799_VH.1 | Grafted |
| h1799_VL.1A | I48V | h1799_VH.1A | Q3K |
| h1799_VL.1B | I48V, K45Q | h1799_VH.1B | Q3K, R87K |
| h1799_VL.1C | I48V, K45Q, A43S | | |
| h1799_VL.1D | I48V, K45Q, A43S, T85S | | |

Note: For example, I48V means that I at position 48 is mutated back to V, according to the natural numbering. "Grafted" represents the sequence of murine antibody CDRs implanted into human germline FR region.

**Table 5. Combinations of humanized antibody heavy chain variable region and light chain variable region sequences, for murine antibody 1799**

| | **h1799_VL. 1** | **h1799_VL.1 A** | **h1799_VL.1 B** | **h1799_VL.1 C** | **h1799_VL.1 D** |
|---|---|---|---|---|---|
| h1799_VH.1 | h1799-005 | h1799-006 | h1799-007 | h1799-008 | h1799-009 |
| h1799_VH.1 A | h1799-010 | h1799-011 | h1799-012 | h1799-013 | h1799-014 |
| h1799_VH.1B | h1799-015 | h1799-016 | h1799-017 | h1799-018 | h1799-019 |

Note: This table shows the resulting sequences of various mutation combinations. As indicated by h1799-005, the humanized murine antibody h1799-005 has two mutants (light chain h1799_VL.1A and heavy chain h1799_VH.1). Others can be indicated in similar way.

The particular humanized sequences of 1799 are as follows:
>h1799_VH.1 (the same as h1799VH-CDR graft, SEQ ID NO: 33) 
EVQLVESGGGLVQPGGSLRLSCAASGFTFSDYYMAWVRQAPGKGLEWVANINY
DGSSTYYLDSLKSRFTISRDNAKNSLYLQMNSLRAEDTAVYYCARDVGYYGGN
YGFAYWGQGTLVTVSS;
>h1799_VH. 1A (SEQ ID NO: 34) 
EVKLVESGGGLVQPGGSLRLSCAASGFTFSDYYMAWVRQAPGKGLEWVANINY
DGSSTYYLDSLKSRFTISRDNAKNSLYLQMNSLRAEDTAVYYCARDVGYYGGN
 YGFAYWGQGTLVTVSS;
>h1799_VH.1B (SEQ ID NO: 35) 
EVKLVESGGGLVQPGGSLRLSCAASGFTFSDYYMAWVRQAPGKGLEWVANINY
DGSSTYYLDSLKSRFTISRDNAKNSLYLQMNSLKAEDTAVYYCARDVGYYGGN
YGFAYWGQGTLVTVSS;
>h1799_VL.1 (the same as h1799VL-CDR graft, SEQ ID NO: 36) 
DIQMTQSPSSLSASVGDRVTITCRASDNIYSYLAWYQQKPGKAPKLLIYNAKTL
AEGVPSRFSGSGSGTDFTLTISSLQPEDFATYYCQQHYGSPLTFGQGTKLEIK;
>h1799_VL.1A (SEQ ID NO: 37) 
DIQMTQSPSSLSASVGDRVTITCRASDNIYSYLAWYQQKPGKAPKLLVYNAKTL
AEGVPSRFSGSGSGTDFTLTISSLQPEDFATYYCQQHYGSPLTFGQGTKLEIK;
>h1799_VL.1B (SEQ ID NO: 38) 
DIQMTQSPSSLSASVGDRVTITCRASDNIYSYLAWYQQKPGKAPQLLVYNAKTL
AEGVPSRFSGSGSGTDFTLTISSLQPEDFATYYCQQHYGSPLTFGQGTKLEIK;
>h1799_VL.1C (SEQ ID NO: 39) 
DIQMTQSPSSLSASVGDRVTITCRASDNIYSYLAWYQQKPGKSPQLLVYNAKTL
AEGVPSRFSGSGSGTDFTLTISSLQPEDFATYYCQQHYGSPLTFGQGTKLEIK;
>h1799_VL.1D (SEQ ID NO: 40)

DIQMTQSPSSLSASVGDRVTITCRASDNIYSYLAWYQQKPGKSPQLLVYNAKTL
AEGVPSRFSGSGSGTDFTLTISSLQPEDFASYYCQQHYGSPLTFGQGTKLEIK.

### IV. Preparation of recombinant chimeric antibody and humanized antibody

For preparation of the antibody, the constant region of human heavy chain IgG4/light chain kappa was combined with each variable region, and the S228P mutation was introduced in the Fc segment to increase the stability of the IgG4 antibody. Other mutations known in the art can also be used to improve its performance.

The sequence of the heavy chain constant region is shown in SEQ ID NO: 41:

ASTKGPSVFPLAPCSRSTSESTAALGCLVKDYFPEPVTVSWNSGALTSGVHTFPA
VLQSSGLYSLSSVVTVPSSSLGTKTYTCNVDHKPSNTKVDKRVESKYGPPCPPCP
APEFLGGPSVFLFPPKPKDTLMISRTPEVTCVVVDVSQEDPEVQFNWYVDGVEV
HNAKTKPREEQFNSTYRVVSVLTVLHQDWLNGKEYKCKVSNKGLPSSIEKTISK
AKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLG
K;

The sequence of the light chain constant region is as shown in SEQ ID NO: 42: 

;
 wherein, the full-length sequence for the heavy chain of antibody h1799-005 is as shown in SEQ ID NO: 73: 
EVQLVESGGGLVQPGGSLRLSCAASGFTFSDYYMAWVRQAPGKGLEWVANINYDG
SSTYYLDSLKSRFTISRDNAKNSLYLQMNSLRAEDTAVYYCARDVGYYGGNYGFAY
WGQGTLVTVSSASTKGPSVFPLAPCSRSTSESTAALGCLVKDYFPEPVTVSWNSG
ALTSGVHTFPAVLQSSGLYSLSSVVTVPSSSLGTKTYTCNVDHKPSNTKVDKRVE
SKYGPPCPPCPAPEFLGGPSVFLFPPKPKDTLMISRTPEVTCVVVDVSQEDPEVQF
NWYVDGVEVHNAKTKPREEQFNSTYRVVSVLTVLHQDWLNGKEYKCKVSNK
GLPSSIEKTISKAKGQPREPQVYTLPPSQEEMTKNQVSLTCLVKGFYPSDIAVEWE
SNGQPENNYKTTPPVLDSDGSFFLYSRLTVDKSRWQEGNVFSCSVMHEALHNH
YTQKSLSLSLGK;
 the full-length sequence for the light chain of antibody h1799-005 is as shown in SEQ ID NO: 74:

DIQMTQSPSSLSASVGDRVTITCRASDNIYSYLAWYQQKPGKAPKLLIYNAKTLAEG
VPSRFSGSGSGTDFTLTISSLQPEDFATYYCQQHYGSPLTFGQGTKLEIKRTVAAPSV
FIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC;

Note: In the full-length sequence of the antibody h1799-005 described above, the italics represents FR region sequence, the underlined part represents the CDR region sequence, and the wavy line represents the constant region sequence.

### 1. Molecular cloning of recombinant chimeric antibody

The positive antibody molecules obtained from hybridoma screening are sequenced to obtain the sequence of variable region coding gene. The forward and reverse primers were designed based on the sequence obtained by sequencing, and the sequenced gene was served as template; various antibody VH/VK gene fragments were constructed by PCR, and then homologously recombined with expression vector pHr (with signal peptide and hIgG4/hkappa constant region gene (CH1-FC/CL) fragment), and full-length expression plasmid VH-CH1-FC-pHr/VL-CL -pHr for recombinant chimeric antibody was constructed to form two chimeric antibodies Ch1701 and Ch1799.

### 2. Molecular cloning of humanized antibodies

Codon optimization was performed on the designed humanized antibody sequences to produce the coding gene sequence having human codon preference. Primers were designed to construct various antibody VH/VK gene fragments by PCR, and then the fragments were homologously recombined with expression vector pHr (with signal peptide and hIgG4/hkappa constant region gene (CH1-FC/CL) fragment) to construct humanized antibody full-length expression plasmid VH-CH1-FC-pHr/VL-CL-pHr.

### 3. Expression and purification of recombinant chimeric antibody and humanized antibody

The plasmids separately expressing antibody light chain and heavy chain were transfected into HEK293E cells at a ratio of 1:1.2; the expression supernatant was collected 6 days later and centrifuged at high speed to remove impurities; and was purified with Protein A column. The column was rinsed with PBS until the A280 reading dropped to baseline. The target protein was eluted with pH 3.0-pH 3.5 acidic elution solution, and was neutralized with 1M Tris-HCl pH 8.0-9.0. The eluted sample was appropriately concentrated and then further purified by PBS-equilibrated gel chromatography Superdex 200 (GE) to remove the aggregates; the monomer peaks were collected and aliquoted for later use.

### V. Point mutation of h1701 antibody

Deamidation modification is a common chemical modification in antibodies that can affect the stability at later stage. Particularly, some amino acids in the CDR region(s) are highly deamidated, oxidized or isomerized; generally such mutations shall be avoided or reduced. According to accelerated stability tests and computer-simulated antibody structure as well as hotspot prediction, the NNG in the heavy chain CDR2 of the h1701 antibody is a site susceptible to deamidation. The NNG described above is located at positions 54-56 (natural numbering) in the heavy chain variable region of h1701 antibody. According to properties of amino acids and technology for computer-simulated antibody structure, the amino acids at the above positions can be replaced with any amino acid. Preferably, the CDR2 mutant of h1701 is: DIIPX₁X₂X₃GSKYNQKFKD (SEQ ID NO: 43), where X₁, X₂ and X₃ are amino acid residues at positions 54-56 in h1701 antibody heavy chain variable region; X₁ is selected from the group consisting of Asn, Leu, Val, Met and Glu; X₂ is selected from the group consisting of Asn, Glu, Met, His, Lys, Leu, Ala and Val; and X₃ is selected from the group consisting of Gly and Ala.

Further, the CDR2 comprising mutations at positions 54-56 described above and FR region comprising different back-mutation(s) can constitute the following heavy chain variable regions: >h1701_VH.1-CDR2 mutant (SEQ ID NO: 44)

QVQLVQSGAEVKKPGASVKVSCKASGYTFTDYYMNWVRQAPGQGLEWMGDII
PX1X2X3GSKYNQKFKDRVTMTTDTSTSTAYMELRSLRSDDTAVYYCARWGYGS
SYRWFDYWGQGTLVTVSS;

>h1701_VH.1A-CDR2 mutant (SEQ ID NO: 45)

QVQLVQSGAEVKKPGASVKVSCKASGYTFTDYYMNWVRQAPGQGLEWIGDIIP
X1X2X3GSKYNQKFKDRVTMTTDTSTSTAYMELRSLRSDDTAVYYCARWGYGSS
YRWFDYWGQGTLVTVSS;

>h1701_VH.1B-CDR2 mutant (SEQ ID NO: 46)

QVQLVQSGAEVKKPGASVKVSCKASGYTFTDYYMNWVRQAPGQGLEWMGDII
PX1X2X3GSKYNQKFKDRVTMTTDTSTSTAYMELRSLRSDDTAVYYCATWGYGS
SYRWFDYWGQGTLVTVSS;

>h1701_VH.1C-CDR2 mutant (SEQ ID NO: 47)

QVQLVQSGAEVKKPGASVKVSCKASGYTFTDYYMNWVRQAPGQGLEWIGDIIP
X1X2X3GSKYNQKFKDRVTMTTDTSTSTAYMELRSLRSDDTAVYYCATWGYGSS
YRWFDYWGQGTLVTVSS;

>h1701_VH.1D-CDR2 mutant (SEQ ID NO: 48)

QVQLVQSGAEVKKPGASVKVSCKASGYTFTDYYMNWVKQAPGQGLEWIGDII
PX1X2X3GSKYNQKFKDRVTMTTDTSTSTAYMELRSLRSEDTAVYYCATWGYGSS
YRWFDYWGQGTLVTVSS;

>h1701_VH.1E-CDR2 mutant (SEQ ID NO: 49)

QVQLVQSGAEVKKPGASVKVSCKASGYTFTDYYMNWVRQAPGQGLEWIADIIP
X1X2X3GSKYNQKFKDRATLTTDTSTSTAYMELRSLRSDDTAVYYCATWGYGSSY
RWFDYWGQGTLVTVSS;

>h1701_VH.1F-CDR2 mutant (SEQ ID NO: 50)

QVQLVQSGAEVKKPGASVKVSCKASGYTFTDYYMNWVKQAPGQGLEWIADII
PX1X2X3GSKYNQKFKDRATLTTDTSTSTAYMELRSLRSEDTAVYYCATWGYGSS
YRWFDYWGQGTLVTVSS.

Exemplary sequences related to the HCDR2 mutants of h1701 and the humanized sequence h1701_VH.1B-CDR2 mutant (SEQ ID NO: 46) comprising the corresponding CDR2 mutants are shown in the following mutants and Table 6.

As an example, the NNG in HCDR2 of h1701-009 was designed to be mutated to NLG, NVG, NNA, NMA, NEA, NHA, NMG, NEG, NKG, NAG, NHG (the sequences of the heavy chain variable region CDR2 mutants described above are as shown in SEQ ID NOs: 51-61 respectively). The expression plasmid construction and 293E expression were carried out by method of molecular cloning, and the affinity and stability of the mutant antibodies were further tested after purification. A series of amino acid mutations were performed on h1701-009; particular related sequences involve but are not limited to those described in Table 6.

**Table 6. Sequences of heavy chain variable region mutants of h1701-009 comprising anti-deamidation modification**

| Heavy chain variable region | SEQ ID NO. for VH | Corresponding HCDR2 sequence |
|---|---|---|
| h1701-009 | SEQ ID NO: 24 | DIIPNNGGSKYNQKFKD (SEQ ID NO: 9) |
| h1701-009NLG | SEQ ID NO: 51 | DIIPNLGGSKYNQKFKD (SEQ ID NO: 62) |
| h1701-009NVG | SEQ ID NO: 52 | DIIPNVGGSKYNQKFKD (SEQ ID NO: 63) |
| h1701-009NNA | SEQ ID NO: 53 | DIIPNNAGSKYNQKFKD (SEQ ID NO: 64) |
| h1701-009NMA | SEQ ID NO: 54 | DIIPNMAGSKYNQKFKD (SEQ ID NO: 65) |
| h1701-009NEA | SEQ ID NO: 55 | DIIPNEAGSKYNQKFKD (SEQ ID NO: 66) |
| h1701-009NHA | SEQ ID NO: 56 | DIIPNHAGSKYNQKFKD (SEQ ID NO: 67) |
| h1701-009NMG | SEQ ID NO: 57 | DIIPNMGGSKYNQKFKD (SEQ ID NO: 68) |
| h1701-009NEG | SEQ ID NO: 58 | DIIPNEGGSKYNQKFKD (SEQ ID NO: 69) |
| h1701-009NKG | SEQ ID NO: 59 | DIIPNKGGSKYNQKFKD (SEQ ID NO: 70) |
| h1701-009NAG | SEQ ID NO: 60 | DIIPNAGGSKYNQKFKD (SEQ ID NO: 71) |
| h1701-009NHG | SEQ ID NO: 61 | DIIPNHGGSKYNQKFKD (SEQ ID NO: 72) |

### VI. Detection of performance and effect of the antibody

The following test methods are used to verify the performance and beneficial effects of the antibodies of the present disclosure:

### Test Example 1: Binding test of anti-TIM-3 antibody to human TIM-3 overexpressing CHO-s cells

The binding ability of the anti-TIM-3 antibody was tested by the binding of antibody to TIM-3 protein overexpressing CHO-S cells. The TIM-3 full-length plasmid (house-made, SEQ ID NO: 3) was transfected into CHO-S cells by electro-transfection, and after two weeks of screening under stress, the expression level of TIM-3 was detected. The overexpressing cells were fixed on the bottom of 96-well plate, and then the signal strength after the addition of the antibody was used to determine the binding activity of the antibody to Tim-3 overexpressing CHO-S cells. The particular experimental method is as follows. The positive control antibody used is AbTim-3, the sequence is from Table 2 in US20150218274A1, prepared by the applicant.

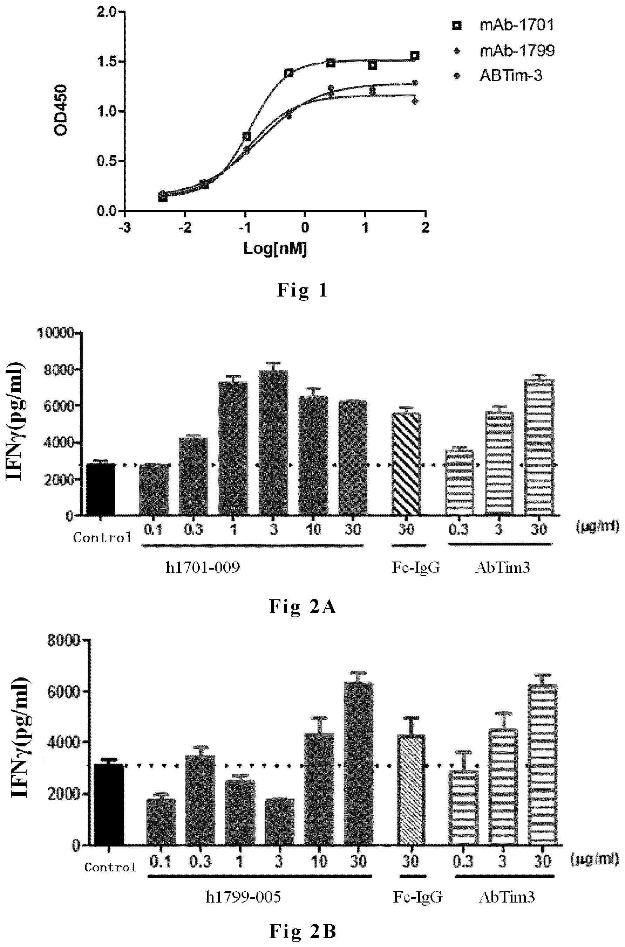

100 µl/well of the cells were seeded in a 96-well plate at a density of 4×10⁵/ml, and cultivated for overnight. The supernatant was removed, the plate was washed with PBS for three times, 100µl/well of 4% PFA was added for fixation for half an hour at room temperature, and the plate was washed with PBS for three times. The liquid was removed, and then the blocking solution comprising 5% skimmed milk (skimmed milk powder from Bright Dairy) diluted with PBS was added at 200µl/well, and incubated for 2.5 hours in a 37°C incubator for blocking. At the end of blocking, the blocking solution was removed and the plated was washed for 5 times with PBST buffer (pH7.4 PBS comprising 0.05% Tween-20); the antibody to be tested (antibody purified from hybridoma or humanized antibody) was diluted with sample diluent to various concentrations was added at 50µl/well, and was incubated for 1 hour in a 37°C incubator. After the incubation was finished, the plate was washed for 5 times with PBST, and 100µl/well of HRP-labeled goat anti-mouse secondary antibody (Jackson Immuno Research, Cat No. 115-035-003) or goat anti-human secondary antibody (Jackson Immuno Research, Cat No. 109-035-003) diluted with sample diluent was added, and incubated at 37°C for 1 hour. After the plate was washed for 6 times with PBST, 50µl/well of TMB chromogenic substrate (KPL, Cat No. 52-00-03) was added, and incubated at room temperature for 5 to 15min; 50µl/well of 1M H₂SO₄ was added to stop the reaction, the absorbance value at a wavelength of 450nm was read using NOVOStar plate reader; EC50 value showing the binding of TIM-3 antibody to Tim-3 overexpressing CHO-S cells was calculated. The test results are shown in Figure 1 and Table 7.

**Table 7. Determination of EC50 for the candidate antibodies in cell binding tests**

| **Candidate antibody** | **Cell-based binding ELISA EC50 (nM)** | **Candidate antibody** | **Cell-based binding ELISA EC50 (nM)** |
|---|---|---|---|
| mAb-1701 | 0.122 | Ch1799 | 0.080 |
| mAb-1799 | 0.117 | h1799-005 | 0.063 |
| Ch1701 | 0.166 | h1799-006 | 0.095 |
| h1701-005 | 0.602 | h1799-007 | 0.109 |
| h1701-006 | 0.735 | h1799-008 | 0.067 |
| h1701-007 | - | h1799-009 | 0.076 |
| h1701-008 | 2.422 | h1799-010 | 0.077 |
| h1701-009 | 0.187 | h1799-011 | 0.114 |
| h1701-010 | 0.069 | h1799-012 | 0.158 |
| h1701-011 | 0.188 | h1799-013 | 0.139 |
| h1701-012 | 0.221 | h1799-014 | 0.116 |
| h1701-013 | 0.199 | h1799-015 | 0.188 |
| h1701-014 | 0.124 | h1799-016 | 0.117 |
| h1701-015 | 0.265 | h1799-017 | 0.194 |
| h1701-016 | 0.058 | h1799-018 | 0.215 |
| h1701-017 | 0.105 | h1799-019 | 0.119 |
| h1701-018 | 0.087 | | |

The results indicate that TIM-3 antibodies No. 1701 and No. 1799, and the humanized antibodies thereof show favorable binding activity to CHO-s cells that overexpress the full-length human TIM-3 protein.

The binding activity to human TIM-3 overexpressing cells was tested on h1701-009 antibodies having HCDR2 point mutation. The results are shown in Table 8.

**Table 8. Determination of EC50 for candidate antibodies in cell binding tests**

| **Candidate antibody** | **Binding ELISA EC50 (nM)** |
|---|---|
| h1701-009 | 0.503 |
| h1701-009 NNA | 0.515 |
| h1701-009 NHA | 0.530 |
| h1701-009 NEA | 0.667 |
| h1701-009 NMA | 0.629 |
| h1701-009 NMG | 0.603 |
| h1701-009 NEG | 0.673 |
| h1701-009 NVG | 0.717 |
| h1701-009 NLG | 0.637 |
| h1701-009 NKG | 0.438 |
| h1701-009 NAG | 0.512 |
| h1701-009 NHG | 0.317 |

The results indicate that the h1701-009 antibodies having HCDR2 point mutation still exhibits strong binding activity to CHO-s cells that overexpress human TIM-3.

### Test Example 2: Competition test of anti-TIM-3 antibodies

The antibody A was diluted to a concentration of 2µg/ml with pH7.4 PBS buffer (Sigma, Cat No. P4417-100TAB), and was added to a 96-well microtiter plate at 50µl/well (Corning, Cat No. CLS3590-100EA), and was placed in 37°C incubator for 2 hours. The liquid was removed, and blocking solution comprising 5% skimmed milk (skimmed milk powder from Bright Dairy) diluted with PBS was added at 200µl/well, and incubated for 2.5 hours in a 37°C incubator or overnight (16-18 hours) at 4°C for blocking. At the end of blocking, the blocking solution was removed and the plated was washed for 5 times with PBST buffer (pH7.4 PBS comprising 0.05% Tween-20); Pre-mixed solution of antibody B diluted to 20µg/ml and 4µg/ml with sample diluent (pH 7.4 PBS containing 1% BSA) and biotin-labeled TIM-3-Fc protein (house-made, SEQ ID NO: 1) with a final concentration of 0.4µg/ml was added at 50µl/well, and was incubated for 1 hour in a 37°C incubator. After the incubation was finished, the reaction solution was removed from the ELISA plate, the plate was washed for 5 times with PBST; 100µl/well of HRP-labeled streptavidin (Sigma, Cat No. S243 8) diluted with sample diluent was added, and incubated at 37°C for 1 hour. After the plate was washed for 5 times with PBST, 50µl/well of TMB chromogenic substrate (KPL, Cat No. 52-00-03) was added, and incubated at room temperature for 5 to 15min; 50µl/well of 1M H₂SO₄ was added to stop the reaction, the absorbance value at a wavelength of 450nm was read using NOVOStar plate reader. The competitive effect of different antibodies for binding to Tim-3 was calculated.

**Table 9. Competition test of mAb-1701 and mAb-1799 to bind to TIM-3**

| | Concentration (µg/ml) | mAb-1701 | mAb-1799 |
|---|---|---|---|
| mAb-1701 | 20 | 0.054 | 1.599 |
| | 4 | 0.072 | 1.685 |
| mAb-1799 | 20 | 0.835 | 0.091 |
| | 4 | 0.86 | 0.077 |

The results indicate that there is no competitive relationship between mAb-1701 and mAb-1799 for binding to TIM-3.

### Test Example 3: Affinity test of anti-TIM-3 antibody by using BIAcore detection

### 1. BIAcore affinity detection of murine antibody

According to the method described in the instruction of the mouse antibody capture kit (Cat. #BR-1008-38, GE), the mouse antibody-capture antibody was covalently conjugated onto the CM5 biosensor chip (Cat. #BR-1000-12, GE) to affinity-capture the antibody to be tested. TIM-3-His (Sino. Biol, Cat. 10390-H03H) antigen then flew through the surface of the chip, and the Biacore instrument was used to detect the real-time reaction signal to obtain curves of association and dissociation; affinity value was obtained by fitting. After each cycle of dissociation was completed in the test, the biochip was washed and regenerated with the regeneration solution provided in the mouse antibody capture kit. The results are shown in Table 10.

**Table 10. Determination of the affinity of candidate antibodies**

| **Candidate antibody** | **Mobile phase** | **Affinity (M)** |
|---|---|---|
| h1701 | TIM-3-His | 1.82E-10 |
| h1799 | | 2.36E-10 |

The results indicate that TIM-3 antibodies mAb-1701 and mAb-1799 have strong binding activity and affinity to human TIM-3 protein.

### 2. BIAcore affinity detection for chimeric antibody and humanized antibody

According to the method described in the instruction of the human antibody capture kit (Cat. #BR-1008-39, GE), the human antibody-capture antibody was covalently conjugated onto the CM5 biosensor chip (Cat. #BR-1000-12, GE) to affinity-capture the antibody to be tested, and then TIM-3-Flag-His (house-made, SEQ ID No:2) antigen flew through the surface of the chip, and the Biacore instrument was used to detect the real-time reaction signal to obtain curves of association and dissociation; affinity value was obtained by fitting (see Table 11). After each cycle of dissociation was completed in the test, the biochip was washed and regenerated with the regeneration solution provided in the human antibody capture kit.

**Table 11. Affinity of anti-TIM-3 antibodies**

| **Candidate antibody** | **mobile phase** | **Affinity (M)** |
|---|---|---|
| Ch1701 | TIM-3-His | 1.39E-9 |
| h1701-009 | | 1.34E-9 |
| h1701-010 | | 2.25E-9 |
| h1701-016 | | 1.60E-9 |
| Ch1799 | | 1.76E-9 |
| h1799-005 | | 1.78E-9 |
| h1799-006 | | 1.89E-9 |
| h1799-010 | | 2.48E-9 |

The results indicate that the humanized antibodies screened in the present disclosure have strong binding activity and affinity to human TIM-3 protein.

The affinity of h1701-009 antibodies having HCDR2 point mutation was detected by BIAcore. The results are shown in Table 12.

**Table 12. Affinity of anti-TIM-3 antibodies**

| Stationary phase | Mobile phase | Affinity (M) |
|---|---|---|
| h1701-009 | TIM-3-His | 5.99E-10 |
| h1701-009 NAG | | 5.79E-10 |
| h1701-009 NEG | | 6.40E-10 |
| h1701-009 NEA | | 6.53E-10 |
| h1701-009 NHG | | 6.40E-10 |
| h1701-009 NHA | | 5.97E-10 |
| h1701-009 NKG | | 4.72E-10 |
| h1701-009 NLG | | 6.40E-10 |
| h1701-009 NMG | | 6.75E-10 |
| h1701-009 NMA | | 6.53E-10 |
| h1701-009 NNA | | 5.69E-10 |
| h1701-009 NVG | | 5.81E-10 |

The results indicate that the above h1701-009 mutants still have strong TIM-3 affinity. The mutations at the NNG site do not affect the affinity of the antibody to the antigen.

### Test Example 4: In vitro test of immune cell functions

To study the effect of anti-TIM-3 antibody on activation of T lymphocyte, we established 3 *in vitro* functional tests: mixed lymphocyte reaction assay (MLR assay), PBMC activation test and super-antigen *Staphylococcus aureus* enterotoxin B (SEB) stimulation test.

### 1. Mixed lymphocyte reaction assay

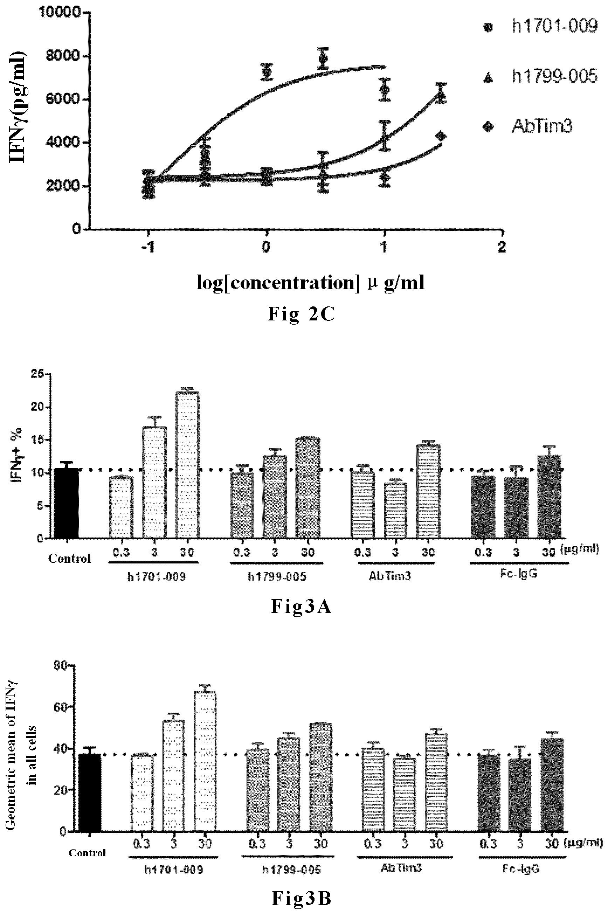

The experiment process is briefly described as follows:
1) Human peripheral blood mononuclear cells (PBMCs) were collected and purified;
2) PBMCs were inoculated in 6-well NUNC plate at a density of 2×10⁶ cell/ml and cultivated for 2 hours;
3) Non-adherent cells were removed, GM-CSF (100ng/ml) and IL-4 (100ng/ml) were used to stimulate adherent cells for 5 days, and then the cells were incubated with TNFα (100ng/ml) for 2 days to induce DC cell maturation;
4) The mature DC cells were mixed with PBMCs from different donors at a ratio of 1:5 in a 96-well plate pre-coated with 10ng/ml CD3 antibody, and different concentrations of h1701-009, h1799-005, AbTIM-3 and negative control Fc-IgG were added in parallel;
5) 5 days later, the concentration of IFNγ in the supernatant was detected by ELISA (the results are shown in Figure 2A to Figure 2C).

The results indicate that h1701-009, h1799-005 and AbTIM-3 all effectively stimulate the secretion of IFNγ, wherein h1701-009 and h1799-005 exhibit stronger stimulating effects.

### 2. PBMC activation test

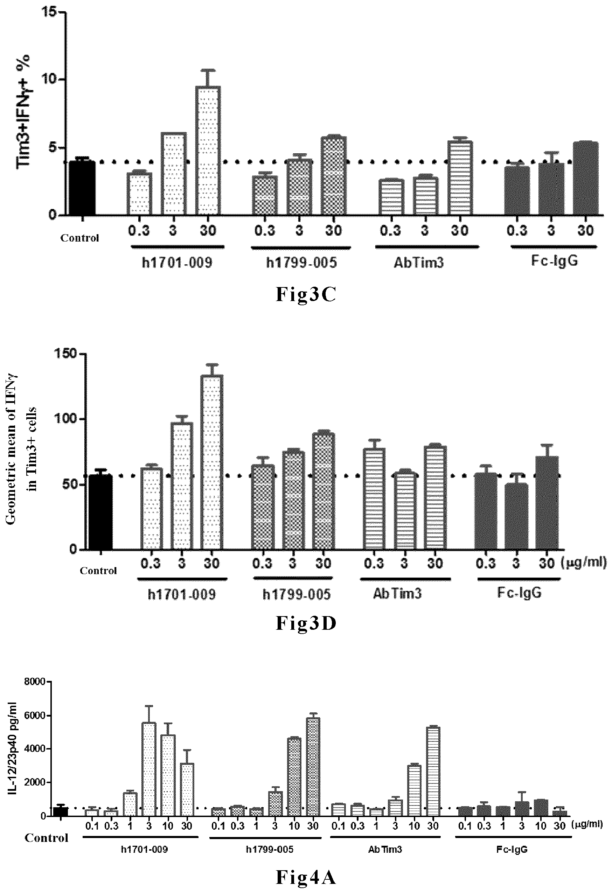

The experiment process is briefly described as follows:
1) Human peripheral blood mononuclear cells (PBMCs) were collected and purified, and a 96-well plate was coated with CD3 antibody and CD28 antibody at a concentration of 10ng/ml;
2) PBMCs were inoculated in a coated 96-well plate at a density of 1x10⁵ cell/well. Different concentrations of TIM-3 antibody and negative control Fc-IgG were added in parallel;
3) 6 days later, cells were collected for intracellular IFNγ and TIM-3 staining. FACS was used to analyze the proportion of IFNγ positive cells (IFNγ+%), IFNγ Geo mean value in all cells, the proportion of IFNγ and TIM-3 double positive cells (TIM-3+IFNγ+%) and IFNγ Geo mean value on TIM-3+ cells (Figure 3A to Figure 3D).

The results indicate that h1701-009, h1799-005 and AbTIM-3 increase the percentage of intracellular IFNγ-positive cells and the expression of IFNγ to varying degrees, wherein h1701-009 and h1799-005 have stronger effect.

### 3. SEB stimulation test

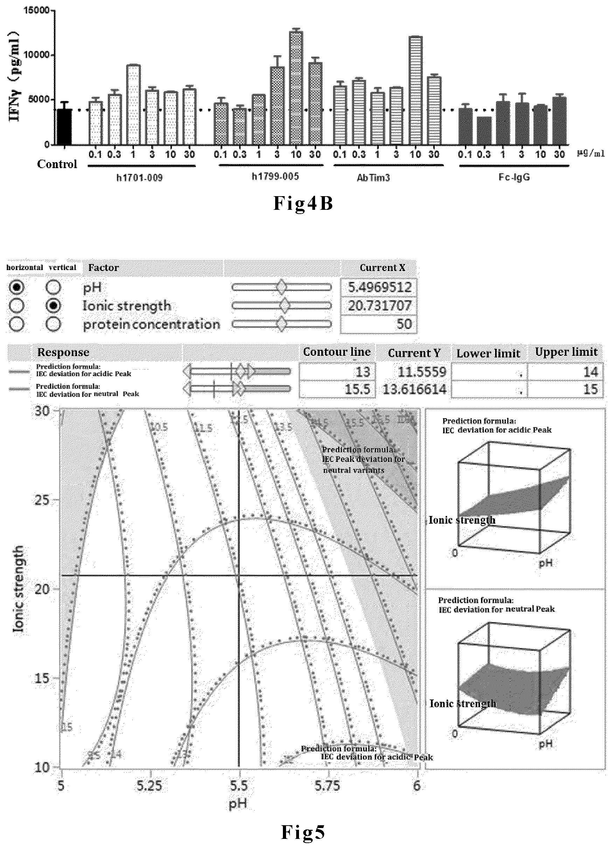

The experiment process is briefly described as follows:
1) Human peripheral blood mononuclear cells (PBMCs) were collected and purified;
2) PBMCs were inoculated in a 96-well plate at a density of 1 × 10⁵ cell/well. 1ng/ml SEB and different concentrations of TIM-3 antibody and negative control Fc-IgG were added in parallel;
3) 5 days later, the supernatant was collected for IL12 and IFNγ detection by ELISA (see Figure 4A and Figure 4B, respectively);

The results indicate that h1701-009, h1799-005 and AbTIM-3 all effectively increase the SEB-induced secretion of IL12 and IFNγ, wherein h1701-009 and h1799-005 exhibit higher advantage at low dose.

### Test Example 5: PK determination for anti-TIM-3 humanized antibody, h1701-009 and h1799-005 in rat

12 SD rats, weighing 180-240g, were purchased from Sippr-BK laboratory animal Co. Ltd. The animals were allowed free access to food and water during the feeding period; and animals were allowed for adapting laboratory environment no less than 3 days, with 12/12 hour light/dark cycle adjustment, temperature 16-26 °C, relative humidity 40-70%. The day before the start of the test, the SD rats were numbered and randomly divided into groups, with 3 animals per group (2 males and 1 female). On the day of the test, two groups of rats were injected intravenously with the test agent h1701-009 at a dose of 3 mg/kg and 10 mg/kg respectively; and the other two groups of rats were intravenously injected with the test agent h1799-005 at a dose of 3 mg/kg and 10 mg/kg respectively. The volume for intravenous injection was 5 ml/kg.

Blood was collected at various time points, i.e., before administration, 5min, 8h, 1d, 2d, 4d, 7d, 10d, 14d, 21d and 28d after administration. 0.2 ml of whole blood was collected from each animal without adding anticoagulant reagent. After the blood was collected, it was placed at 4°C for 30 minutes, centrifuged at 1000g for 15 minutes, and the supernatant was transferred into EP tube and stored at 80°C.

The ELISA method was used to detect the concentration of the antibody in the serum, and the Winnolin software was used to calculate the pharmacokinetic parameters of the tested agents. The main pharmacokinetic parameters obtained are shown in Table 13.

**Table 13. Half-life of h1799-005 and h1701-009 in rats**

| | h1799-005 | | h1701-009 | |
|---|---|---|---|---|
| Dose (mg/kg) | 3 | 10 | 3 | 10 |
| t_{1/2} (day) | 13.6±1.1 | 11.4±1.7 | 10.6±1.8 | 15.5±2.5 |

Intravenous injections of 10 mg/kg anti-TIM-3 antibody h1701-009 and h1799-005 into SD rats result in similar exposure amounts in rats; the increase in exposure amount of antibody h1799-005 at a dose of 3 or 10 mg/kg is correlated linearly with the increase in dose; the increase in exposure amount of antibody h1701-009 at a dose of 3 or 10 mg/kg is slightly higher than the increase in dose; and the clearance half-life of antibody h1701-009 is similar to that of h1799-005.

### Test Example 6: Detection of the thermal stability of h1701-009 and h1799-005 antibodies by DSC

Different buffer systems with different pH conditions were compared for thermal stability. Exemplary buffer systems corresponding to different pH values are as follows: 10mM PB (pH7), 15mM His (pH6.0), and 10mM acetate (pH5.2). The sample was replaced into the corresponding buffer, the sample concentration was controlled at about 1mg/ml; and MicroCal* VP-Capillary DSC (Malvern) was used for detection. Before testing, each sample and blank buffer was degassed with a vacuum degasser for 1 to 2 minutes. 400µl sample or blank buffer was added into each well of the sample plate (the loading volume into the instrument is 300µl). 14% Decon 90 and ddH₂O were added into the last two pairs of well plates for cleaning purpose; the sample plate was loaded, and then covered with a plastic soft cover. The scanning temperature started from 25°C and ended at 100°C, and the scanning rate was 60°C/h. The particular results are shown in Table 14. In several test systems, both h1701-009 and h1799-005 exhibit favorable thermal stability.

**Table 14. Monitoring table of the thermal stability of h1701-009 and h1799-005 antibody at different pH**

| Sample | Buffer | Tm-onset (°C) | TM (°C) |
|---|---|---|---|
| h1701-009 | pH7.0 | 63.09 | 74.59 |
| | pH 6.0 | 59.1 | 77.24 |
| | pH 5.2 | 60.32 | 77.13 |
| h1799-005 | pH7.0 | 62.99 | 76.63 |
| | pH 6.0 | 60.01 | 77.49 |
| | pH 5.2 | 60.2 | 77.69 |

### Test Example 7: Monitoring the purity of the sample by SEC-HPLC to investigate the periodic stability under certain concentration

Under exemplary conditions, such as the sample concentration was controlled at about 50mg/ml, different antibodies in PBS (pH7.4) system were compared, for example, for their stability after repeated freezing-thawing for 5 times at -80°C, being stored at 4°C or 40°C for one month. Xbridge protein BEH SEC 200A (Waters) HPLC column was used to detect antibody purity; after observation for one month, both h1701-009 and h1799-005 exhibit favorable stability, and there was no significant change in accelerated SEC purity at 40°C for one month.

### Test Example 8: Chemical stability of antibodies

The h1701-009, h1799-005 and h1701-009 mutants as shown in SEQ ID NOs: 51-55 were tested for chemical stability.

500µg of the antibody to be tested was dissolved in 500µl pH 7.4 PBS in 40°C water bath; samples were taken on Day 0, 14, and 28 for enzymatic hydrolysis tests. 100 µg samples taken at different time points were dissolved in 100 µl 0.2 M His-HCl, 8M Gua-HCl solution, pH 6.0, and 3 µl 0.1g/mL DTT was added, the samples were incubated in 50°C water bath for 1 hour, and then ultrafiltered twice by using 0.02 M His-HCl solution, pH 6.0, 3µL of 0.25mg/mL trypsin was added, and the samples were hydrolyzed overnight in 37°C water bath. Agilent 6530 Q-TOF was used for LC-MS detection and analysis. The results indicate that h1799-005 does not show significant isomerization, oxidation and deamidation modification at 40°C for one month acceleration, suggesting that the molecule has favorable chemical stability. For the detection of h1701-009, no significant oxidation and isomerization modification is found, but there is a strong deamidation modification at N54N55G56 site; and a series of amino acid mutations were introduced into the above site. Exemplary mutations involve such as NLG, NVG, NMA, NEA, NNA etc.. The *in vitro* activity verified that these mutations do not affect the biological activity. After accelerating test, LC-MS analysis indicates that these mutants can effectively reduce deamidation modification. Table 15 shows the ratio of deamidation modification of different mutants after acceleration.

**Table 15. Monitoring table of the chemical stability of h1701-009 antibodies with CDR2 mutations**

| | Day 0 | one week | two weeks |
|---|---|---|---|
| h1701-009 | 11.16% | 17.55% | 34.62% |
| h1701-009NLG | 0 | 0 | 0 |
| h1701-009NVG | 0 | 0 | 0 |
| h1701-009NMA | 0 | 0 | 0 |
| h1701-009NEA | 0 | 0 | 0 |
| h1701-009NNA | 0 | 0 | 13.58% |

### Example 2. Screening of pH value of anti-TIM3 antibody formulation buffer system

The following buffers were used to prepare anti-TIM3 antibody formulation, in which the antibody concentration was 50 mg/ml (h1799-005, prepared according to Example 1, similarly for those used hereafter):
1) 10mM histidine-acetic acid, pH 4.5
2) 10mM histidine-acetic acid, pH 5.0
3) 10mM histidine-acetic acid, pH 5.5
4) 10mM histidine-acetic acid, pH 6.0

The samples were collected for studying the stability at high temperature (40°C). The results indicate (see Table 16) that pH range of 5.5 to 6.0 is suitable for anti-TIM3 antibody.

**Table 16. Results of screening PH values for anti-TIM3 antibody**

| No. | Preservation period | SEC % monomer | ICE % | |
|---|---|---|---|---|
| | | | neutral peak | alkaline peak |
| 1 | D0 | 96.1 | 67.7 | 6.5 |
| | 40°C D7 | 95.5 | 57.5 | 18.1 |
| 2 | D0 | 96.3 | 67.7 | 6.4 |
| | 40°C D7 | 95.0 | 59.7 | 15.4 |
| 3 | D0 | 96.1 | 67.9 | 6.2 |
| | 40°C D7 | 94.9 | 63.4 | 10.5 |
| 4 | D0 | 96.0 | 68.9 | 5.8 |
| | 40°C D7 | 95.6 | 64.5 | 8.3 |

| | | | | |
|---|---|---|---|---|
| Note: D represents day; for example, D7 represents the seventh day. | | | | |

### Example 3. Screening of buffer system for anti-TIM3 antibody formulation

The following buffers were used to prepare anti-TIM3 antibody (h1799-005) formulation, in which the antibody concentration was 50 mg/ml:
1) 10mM histidine-acetic acid, pH 6.0
2) 10mM citric acid-sodium citrate, pH 6.0
3) 10mM histidine-hydrochloric acid, pH 6.0
4) 10mM sodium dihydrogen phosphate-disodium hydrogen phosphate, pH 6.0
5) 10mM histidine-acetic acid, pH 5.5
6) 10mM acetic acid-sodium acetate, pH 5.5
7) 10mM succinic acid-sodium succinate, pH 5.5
8) 10mM succinic acid-sodium succinate, pH 6.0
9) 10mM histidine-hydrochloric acid, pH 5.5
10) 10mM citric acid-sodium citrate, pH 5.5

The samples were collected to study the stability at high temperature (40°C) and in the light at low temperature (4°C/4500 lx). The results indicate (see Table 17) that more suitable buffer systems for anti-TIM3 antibody are histidine-hydrochloric acid (His-HCl), acetic acid-sodium acetate (AA) and histidine-acetic acid (His-AA) buffer. Considering that the actual pH of the actual production sample can drift slightly from the target pH, the optimal buffer system for the stability of TIM3 is His-AA system, followed by His-HCl and AA system.

**Table 17. Screening results of buffer systems with different pH values for anti-TIM3 antibodies**

| N o. | Time point | Appearance | SEC (%) monomer | ICE (%) | | non-reducing CE(%) |
|---|---|---|---|---|---|---|
| | | | | acidic peak | neutral peak | |
| 1 | D0 | Clear and transparent, with trace amount of fine particles | 95.4 | 25.6 | 66.8 | 95.8 |
| | 40 °C D21 | Clear and transparent, blue opalescence | 96.1 | 39.1 | 52.5 | 93.8 |
| | Light D10 | Clear and transparent, with trace amount of fine particles | 92.8 | 30.5 | 61.2 | 95.9 |
| 2 | D0 | fine particles | 95.6 | 26.1 | 65.5 | 95.7 |
| | 40°C D21 | Clear and transparent, blue opalescence | 95.8 | 37.0 | 54.5 | 93.9 |
| | Light D10 | fine particles | 93.3 | 26.2 | 65.7 | 95.8 |
| 3 | D0 | fine particles | 95.4 | 25.9 | 65.5 | 95.7 |
| | 40 °C D21 | Clear and transparent, blue opalescence | 96.2 | 38.6 | 51.9 | 94.2 |
| | Light D10 | fine particles | 93.0 | 29.5 | 64.9 | 95.9 |
| 4 | D0 | fine particles | 95.5 | 24.8 | 67.3 | 95.8 |
| | 40 °C D21 | Clear and transparent, blue opalescence | 95.7 | 36.3 | 54.1 | 92.8 |
| | Light D10 | fine particles | 91.7 | 28.3 | 63.6 | 95.2 |
| 5 | D0 | fine particles | 95.6 | 25.4 | 66.1 | 95.6 |
| | 40 °C D21 | Clear and transparent, blue opalescence | 96.3 | 35.5 | 53.5 | 94.2 |
| | Light D10 | fine particles | 93.4 | 28.1 | 62.8 | 96.1 |
| 6 | D0 | fine particles | 95.5 | 25.7 | 65.1 | 95.7 |
| | 40 °C D21 | Clear and transparent, blue opalescence | 96.0 | 35.3 | 53.0 | 94.1 |
| | Light D10 | fine particles | 93.0 | 28.7 | 61.2 | 96.1 |
| 7 | D0 | Clear and transparent | 95.4 | 24.7 | 66.4 | 95.7 |
| | 40 °C D21 | Clear and transparent, blue opalescence | 96.0 | 36.4 | 51.2 | 94.4 |
| | Light D10 | Clear and transparent | 93.3 | 24.8 | 65.6 | 95.9 |
| 8 | D0 | fine particles | 95.1 | 25.3 | 65.3 | 95.5 |
| | 40 °C D21 | Clear and transparent, blue opalescence | 95.7 | 38.6 | 52.4 | 93.7 |
| | Light D10 | fine particles | 92.2 | 26.7 | 66.2 | 95.6 |
| 9 | D0 | Clear and transparent | 95.4 | 25.1 | 66.7 | 95.7 |
| | 40 °C D21 | Clear and transparent, blue opalescence | 96.4 | 33.2 | 54.2 | 94.2 |
| | Light D10 | Clear and transparent | 93.5 | 28.2 | 64.2 | 96.1 |
| 10 | D0 | fine particles | 95.7 | 25.0 | 66.2 | 95.8 |
| | 40 °C D21 | Clear and transparent, blue opalescence | 95.8 | 36.0 | 50.0 | 94.0 |
| | Light D10 | fine particles | 93.5 | 26.4 | 64.3 | 96.1 |

| | | | | | | |
|---|---|---|---|---|---|---|
| Note: D represents day; for example, D21 represents the 21^{st} day. | | | | | | |

### Example 4. Screening of stabilizers for anti-TIM3 antibody formulation

The anti-TIM3 antibody (h1799-005) formulation in which the antibody concentration was 50 mg/ml was prepared with different saccharides and buffers.
1) 10mM histidine- acetic acid pH 6.0, 70 mg/ml sucrose;
2) 10mM histidine-acetic acid pH 6.0, 70 mg/ml α,α-trehalose dihydrate;

The samples of the formulations prepared above were subjected to stability study at high temperature (40°C) and in the light at low temperature (5 ± 3 °C, 4500 lx). The results are shown in Table 18. The results indicate that sucrose and trehalose have similar effect on stability of TIM3 antibody. When the sucrose concentration is 80 mg/ml, the osmotic pressure is about 300 mosm/kg, which is close to isotonic pressure. Therefore, preferably the sucrose concentration is 80 mg/ml.

**Table 18. Results of screening test for stabilizers**

| Components of formulation | Time point | Appearance | SEC (%) | | |
|---|---|---|---|---|---|
| | | | poly mer | mono mer | fragm ent |
| 50 mg/ml anti-TIM3 antibody, 10mM histidine-acetic acid, pH 6.0, 70 mg/ml sucrose | D0 | Clear and transparent | 1.3 | 95.8 | 2.9 |
| | 40 °C D21 | Clear and transparent, blue opalescence | 2.2 | 96.3 | 1.5 |
| | Light D10 | Clear and transparent | 2.0 | 93.0 | 5.0 |
| 50 mg/ml anti-TIM3 antibody, 10mM histidine-acetic acid, pH 6.0, 70 mg/ml α,α-trehalose dihydrate | D0 | Clear and transparent | 1.3 | 95.6 | 3.1 |
| | 40 °C D21 | Clear and transparent, blue opalescence | 2.2 | 96.4 | 1.5 |
| | Light D10 | Clear and transparent | 2.0 | 93.0 | 5.0 |

| | | | | | |
|---|---|---|---|---|---|
| Note: D represents day, for example, D21 represents the 21st day. | | | | | |

### Example 5. Screening of surfactants for anti-TIM3 antibody formulation

The following buffers comprising different concentrations and different types of surfactants were used to prepare anti-TIM3 antibody (h1799-005) formulation, in which the sucrose concentration was 70 mg/ml and the antibody concentration was 50 mg/ml:
1) 10mM histidine-acetic acid, pH 5.5;
2) 10mM histidine-acetic acid, pH 5.5, 0.2 mg/ml polysorbate 80;
3) 10mM histidine-acetic acid, pH 5.5, 0.4 mg/ml polysorbate 80;
4) 10mM histidine-acetic acid, pH 5.5, 0.6 mg/ml polysorbate 80;
5) 10mM histidine-acetic acid, pH 5.5, 0.4 mg/ml polysorbate 20;

The samples of anti-TIM3 antibody formulations (groups 1-5) prepared according to the method above, were diluted in 0.9% sodium chloride injection solution, with protein concentration diluted to 0.5 mg/ml; the insoluble particles of each group of samples after dilution were observed. The test results are shown in Table 19. The results indicate that no less than 0.4 mg/ml polysorbate 80 provides better stability for anti-TIM3 antibody, therefore 0.4 to 0.6 mg/ml polysorbate 80 was selected, preferably 0.4 mg/ml polysorbate 80 was served as the surfactant for anti-TIM3 antibody.

**Table 19. Screening results of polysorbate dilution for anti-TIM3 antibody**

| No. | Time | Insoluble particles after dilution (particles/ml) | | |
|---|---|---|---|---|
| | | 2 µm | 10 µm | 25 µm |
| 1 | 0 | 4710 | 642 | 35 |
| | 24 h | 2705 | 614 | 70 |
| 2 | 0 | 856 | 45 | 2 |
| | 24 h | 299 | 20 | 1 |
| 3 | 0 | 248 | 7 | 0 |
| | 24 h | 87 | 4 | 0 |
| 4 | 0 | 174 | 24 | 1 |
| | 24 h | 77 | 9 | 1 |
| 5 | 0 | 429 | 81 | 9 |
| | 24 h | 96 | 10 | 0 |

### Example 6. Comprehensive screening of components in formulation

In order to further optimize the protein concentration, type of buffer system, and pH, JMP software was used to design of experiment (DoE), and RSM model was used to obtain a series of formulations (see below). The anti-TIM3 antibody (h1799-005) formulations comprising different concentrations of anti-TIM3 antibody, 80 mg/ml sucrose and 0.4 mg/ml polysorbate 80 were prepared in the following buffer systems with different ionic strength and different pH:
1) 20mM histidine-acetic acid, pH 5.5, 60 mg/ml TIM3 antibody;
2) 10mM histidine-acetic acid, pH 5.5, 50 mg/ml TIM3 antibody;
3) 20mM histidine-acetic acid, pH 5.5, 50 mg/ml TIM3 antibody;
4) 10mM histidine-acetic acid, pH 6.0, 40 mg/ml TIM3 antibody;
5) 30mM histidine-acetic acid, pH 5.5, 50 mg/ml TIM3 antibody;
6) 20mM histidine-acetic acid, pH 6.0, 50 mg/ml TIM3 antibody;
7) 30mM histidine-acetic acid, pH 5.0, 60 mg/ml TIM3 antibody;
8) 10mM histidine-acetic acid, pH 6.0, 60 mg/ml TIM3 antibody;
9) 20mM histidine-acetic acid, pH 5.0, 50 mg/ml TIM3 antibody;
10) 30mM histidine-acetic acid, pH 6.0, 60 mg/ml TIM3 antibody;
11) 20mM histidine-acetic acid, pH 5.5, 40 mg/ml TIM3 antibody;
12) 30mM histidine-acetic acid, pH 6.0, 40 mg/ml TIM3 antibody;
13) 30mM histidine-acetic acid, pH 5.0, 40 mg/ml TIM3 antibody;
14) 10mM histidine-acetic acid, pH 5.0, 60 mg/ml TIM3 antibody;
15) 10mM histidine-acetic acid, pH 5.0, 40 mg/ml TIM3 antibody;

The samples of anti-TIM3 antibody (groups 1-15) formulations prepared according to the method above were stored at 40°C for stability analysis, and the difference between the IEC neutral peak and the acidic peak was used as evaluation indicator, and the results were statistically analyzed by the least square method. The test results are shown in Figure 5 and Table 20. The results indicate that histidine-acetic acid system with the ionic strength of 10mM, pH 5.5 is more favorable to the stability of anti-TIM3 antibody, and the protein is more stable at the concentration between 40 to 60mg/ml. The median value of 50 mg/ml is served as the final protein concentration of anti-TIM3 antibody.

**Table 20. Test results of stability of TIM3 antibody stored at high temperature**

| Batch No. | Time | SEC (%) monomer | IEC (%) | | | NR-CE (%) |
|---|---|---|---|---|---|---|
| | | | acidic peak | neutral peak | alkaline peak | |
| 1 | D0 | 98.7 | 18.8 | 67.3 | 13.9 | 97.1 |
| | 40 °C M1 | 97.1 | 28.8 | 54.3 | 16.8 | 96.3 |
| 2 | D0 | 98.3 | 19.0 | 67.7 | 13.3 | 97.3 |
| | 40 °C M1 | 97.1 | 29.7 | 54.6 | 15.7 | 96.8 |
| 3 | D0 | 98.8 | 19.1 | 67.6 | 13.3 | 97.1 |
| | 40 °C M1 | 97.1 | 30.1 | 53.5 | 16.4 | 96.4 |
| 4 | D0 | 98.7 | 19.1 | 68.1 | 12.8 | 97.5 |
| | 40 °C M1 | 97.3 | 33.2 | 54.5 | 12.3 | 96.7 |
| 5 | D0 | 98.9 | 19.1 | 67.6 | 13.2 | 97.4 |
| | 40 °C M1 | 97.3 | 32.8 | 54.0 | 13.1 | 96.2 |
| 6 | D0 | 98.8 | 19.3 | 67.9 | 12.8 | 97.4 |
| | 40 °C M1 | 97.4 | 32.9 | 54.1 | 13.0 | 96.9 |
| 7 | D0 | 98.8 | 19.1 | 67.4 | 13.5 | 97.1 |
| | 40 °C M1 | 97.0 | 26.6 | 51.5 | 21.9 | 96.2 |
| 8 | D0 | 98.7 | 19.2 | 67.9 | 12.9 | 97.2 |
| | 40 °C M1 | 96.9 | 34.0 | 55.0 | 11.1 | 96.3 |
| 9 | D0 | 98.5 | 19.1 | 67.6 | 13.3 | 97.2 |
| | 40 °C M1 | 96.7 | 30.0 | 53.4 | 16.6 | 96.1 |
| 10 | D0 | 98.9 | 19.1 | 67.8 | 13.0 | 97.1 |
| | 40 °C M1 | 97.0 | 36.3 | 50.0 | 13.7 | 96.3 |
| 11 | D0 | 98.8 | 19.1 | 67.6 | 13.3 | 97.6 |
| | 40 °C M1 | 97.2 | 31.9 | 51.6 | 16.4 | 97.0 |
| 12 | D0 | 98.8 | 19.3 | 68.3 | 12.4 | 97.4 |
| | 40 °C M1 | 96.5 | 37.8 | 49.9 | 12.3 | 96.5 |
| 13 | D0 | 98.8 | 19.1 | 66.8 | 14.1 | 97.4 |
| | 40 °C M1 | 97.0 | 27.0 | 47.7 | 25.4 | 97.1 |
| 14 | D0 | 98.5 | 18.9 | 67.2 | 13.9 | 97.4 |
| | 40 °C M1 | 96.9 | 27.4 | 50.6 | 22.0 | 97.0 |
| 15 | D0 | 98.6 | 19.0 | 67.1 | 13.9 | 97.5 |
| | 40 °C M1 | 97.0 | 27.7 | 50.7 | 21.7 | 97.0 |

| | | | | | | |
|---|---|---|---|---|---|---|
| Note: M represents month, for example, M1 means one month. | | | | | | |

### Example 7. Alternative optional formulations

In addition, the present disclosure also provides other anti-TIM3 antibody formulations, comprising but not limited to:
(1) 50mg/ml TIM3 antibody (h1799-005), 80 mg/ml sucrose, 0.6 mg/ml polysorbate 80, and 10 mM acetic acid-sodium acetate buffer pH 6.0;
(2) 50mg/ml TIM3 antibody (h1799-005), 80 mg/ml sucrose, 0.6 mg/ml polysorbate 80, and 20 mM acetic acid-sodium acetate buffer pH 5.5;
(3) 50mg/ml TIM3 antibody (h1799-005), 80 mg/ml sucrose, 0.6 mg/ml polysorbate 80, and 10 mM acetic acid-sodium acetate buffer pH 6.5;
(4) 100mg/ml TIM3 antibody (h1799-005), 80 mg/ml sucrose, 0.6 mg/ml polysorbate 80, and 10 mM acetic acid-sodium acetate buffer pH 5.4;
(5) 100mg/ml TIM3 antibody (h1799-005), 80 mg/ml sucrose, 0.6 mg/ml polysorbate 80, and 20 mM acetic acid-sodium acetate buffer pH 5.4;
(6) 50mg/ml TIM3 antibody (h1799-005), 90 mg/ml trehalose, 0.6 mg/ml polysorbate 80, and 10 mM acetic acid-sodium acetate buffer pH 5.2;
(7) 60mg/ml TIM3 antibody (h1799-005), 70 mg/ml sucrose, 0.8 mg/ml polysorbate 80, and 10 mM acetic acid-sodium acetate buffer pH 5.2;
(8) 40mg/ml TIM3 antibody (h1799-005), 70 mg/ml sucrose, 0.8 mg/ml polysorbate 80, and 10 mM acetic acid-sodium acetate buffer pH 5.4;
(9) 50mg/ml TIM3 antibody (h1799-005), 90 mg/ml sucrose, 0.5 mg/ml polysorbate 80, and 30 mM acetic acid-sodium acetate buffer pH 5.5;
(10) 40mg/ml TIM3 antibody (h1799-005), 90 mg/ml trehalose, 0.5 mg/ml polysorbate 80, and 20 mM acetic acid-sodium acetate buffer pH 6.0;
(11) 50mg/ml TIM3 antibody (h1799-005), 70 mg/ml sucrose, 0.6mg/ml polysorbate 80, and 10mM acetic acid-sodium acetate buffer pH 5.2;
(12) 60mg/ml TIM3 antibody (h1799-005), 75 mg/ml sucrose, 0.6mg/ml polysorbate 80, and 10mM acetic acid-sodium acetate buffer pH 5.2;
(13) 55mg/ml TIM3 antibody (h1799-005), 75 mg/ml sucrose, 0.5mg/ml polysorbate 80, and 10mM acetic acid-sodium acetate buffer pH 5.8;
(14) 45mg/ml TIM3 antibody (h1799-005), 85 mg/ml sucrose, 0.7mg/ml polysorbate 80, and 10mM acetic acid-sodium acetate buffer pH 5.8;
(15) 50mg/ml TIM3 antibody (h1799-005), 80 mg/ml sucrose, 0.6mg/ml polysorbate 80, and 10mM acetic acid-sodium acetate buffer pH 5.9;
(16) 100mg/ml TIM3 antibody (h1799-005), 80 mg/ml sucrose, 0.6 mg/ml polysorbate 80, and 10 mM histidine-acetic acid buffer pH 5.5;
(17) 70mg/ml TIM3 antibody (h1799-005), 90 mg/ml sucrose, 0.8 mg/ml polysorbate 80, and 5 mM histidine-acetic acid buffer pH 5.5;
(18) 1mg/ml TIM3 antibody (h1799-005), 60 mg/ml sucrose, 0.6 mg/ml polysorbate 80, and 20 mM histidine-acetic acid buffer pH 5.8;
(19) 90 mg/ml TIM3 antibody, 60 mg/ml sucrose, 0.6 mg/ml polysorbate 80, and 20 mM histidine-acetic acid buffer with pH 5.8.

## Claims

1. A pharmaceutical composition, comprising a TIM-3 antibody or antigen-binding fragment thereof, and a buffer, wherein the buffer is selected from the group consisting of acetate, histidine salt, succinate, phosphate and citrate buffer, preferably the buffer is acetate buffer or histidine salt buffer, and the pH of the buffer is 5.0 to 6.5, preferably 5.5 to 6.0, most preferably about 5.5.

2. The pharmaceutical composition according to claim 1, wherein the acetate buffer is acetic acid-sodium acetate buffer; the histidine salt buffer is histidine-acetic acid buffer or histidine-hydrochloric acid buffer; the succinate buffer is succinic acid-sodium succinate buffer; the phosphate buffer is disodium hydrogen phosphate-sodium dihydrogen phosphate buffer; preferably the buffer is histidine-hydrochloric acid, acetic acid-sodium acetate or histidine-acetic acid buffer, most preferably histidine-acetic acid buffer.

3. The pharmaceutical composition according to claim 1 or 2, wherein the concentration of the buffer is 5 mM to 30 mM, preferably 10 mM to 20 mM, most preferably about 10 mM.

4. The pharmaceutical composition according to any one of claims 1 to 3, wherein the concentration of the TIM-3 antibody or antigen-binding fragment thereof is 1 mg/ml to 100 mg/ml, preferably 40 mg/ml to 60 mg/ml, most preferably about 50 mg/ml.

5. The pharmaceutical composition according to any one of claims 1 to 4, further comprising a saccharide, and preferably the saccharide is trehalose or sucrose, most preferably sucrose.

6. The pharmaceutical composition according to claim 5, wherein the concentration of the saccharide is 50 mg/ml to 100 mg/ml, preferably 70 mg/ml to 90 mg/ml, most preferably about 80 mg/ml.

7. The pharmaceutical composition according to any one of claims 1 to 6, further comprising a surfactant, and preferably the surfactant is polysorbate, more preferably polysorbate 80.

8. The pharmaceutical composition according to claim 7, wherein the concentration of the surfactant is 0.2mg/ml to 0.8mg/ml, preferably 0.4mg/ml to 0.6mg/ml, most preferably about 0.4 mg/ml.

9. The pharmaceutical composition according to any one of claims 1 to 8, comprising:
(a) about 1mg/ml to 100mg/ml of the TIM-3 antibody or antigen-binding fragment thereof,
(b) about 5mM to 30mM of the acetate buffer or of the histidine salt buffer,
(c) about 50mg/ml to 100mg/ml of the saccharide, and
(d) about 0.2mg/ml to 0.8mg/ml of the surfactant;
preferably, the pharmaceutical composition comprising: (a) 40mg/ml to 60mg/ml of the TIM-3 antibody or antigen-binding fragment thereof, (b) 10mM to 30mM histidine-acetic acid buffer or acetic acid-sodium acetate buffer, pH of about 5.0 to 6.0, (c) 70mg/ml to 90mg/ml sucrose or trehalose, and (d) 0.4mg/ml to 0.6mg/ml polysorbate; most preferably, the pharmaceutical composition comprising: (a) about 50mg/ml of the TIM-3 antibody or antigen-binding fragment thereof, (b) about 10mM histidine-acetic acid buffer or acetic acid-sodium acetate buffer, pH of about 5.5, (c) about 80mg/ml sucrose, and (d) about 0.4mg/ml polysorbate 80.

10. The pharmaceutical composition according to any one of claims 1 to 9, wherein the TIM-3 antibody or antigen-binding fragment thereof is any monoclonal antibody or antigen-binding fragment thereof selected from the following (i) or (ii):
(i) a monoclonal antibody or antigen-binding fragment thereof, comprising: antibody heavy chain variable region HCDR1-3 regions as shown in sequence SEQ ID NOs: 8, 43 and 10 respectively, or having at least 95% sequence identity to SEQ ID NOs: 8, 43 and 10 respectively; and/or antibody light chain variable region LCDR1-3 regions as shown in sequence SEQ ID NOs: 11, 12 and 13 respectively, or having at least 95% sequence identity to SEQ ID NOs: 11, 12 and 13 respectively;
wherein, SEQ ID NO: 43 is as shown in sequence DIIPX₁X₂X₃GSKYNQKFKD, wherein X₁ is selected from the group consisting of N, L, V, M and E, X₂ is selected from the group consisting of N, E, M, H, K, L, A and V, X₃ is selected from the group consisting of G and A; or
(ii) a monoclonal antibody or antigen-binding fragment thereof, comprising: antibody heavy chain variable region HCDR1-3 regions as shown in sequence SEQ ID NOs: 14, 15 and 16 respectively, or having at least 95% sequence identity to SEQ ID NOs: 14, 15 and 16 respectively; and/or antibody light chain variable region LCDR1-3 regions as shown in sequence SEQ ID NOs: 17, 18 and 19 respectively, or having at least 95% sequence identity to SEQ ID NOs: 17, 18 and 19 respectively.

11. The pharmaceutical composition according to claim 10, wherein the TIM-3 antibody or antigen-binding fragment thereof is any monoclonal antibody or antigen-binding fragment thereof selected from the following (a) to (m):
(a) a monoclonal antibody or antigen-binding fragment thereof, comprising: antibody heavy chain variable region HCDR1-3 regions as shown in sequence SEQ ID NOs: 8, 9 and 10 respectively; and antibody light chain variable region LCDR1-3 regions as shown in sequence SEQ ID NOs: 11, 12 and 13 respectively;
(b) a monoclonal antibody or antigen-binding fragment thereof, comprising: antibody heavy chain variable region HCDR1-3 regions as shown in sequence SEQ ID NOs: 8, 62 and 10 respectively; and antibody light chain variable region LCDR1-3 regions as shown in sequence SEQ ID NOs: 11, 12 and 13 respectively;
(c) a monoclonal antibody or antigen-binding fragment thereof, comprising: antibody heavy chain variable region HCDR1-3 regions as shown in sequence SEQ ID NOs: 8, 63 and 10 respectively; and antibody light chain variable region LCDR1-3 regions as shown in sequence SEQ ID NOs: 11, 12 and 13 respectively;
(d) a monoclonal antibody or antigen-binding fragment thereof, comprising: antibody heavy chain variable region HCDR1-3 regions as shown in sequence SEQ ID NOs: 8, 64 and 10 respectively; and antibody light chain variable region LCDR1-3 regions as shown in sequence SEQ ID NOs: 11, 12 and 13 respectively;
(e) a monoclonal antibody or antigen-binding fragment thereof, comprising: antibody heavy chain variable region HCDR1-3 regions as shown in sequence SEQ ID NOs: 8, 65 and 10 respectively; and antibody light chain variable region LCDR1-3 regions as shown in sequence SEQ ID NOs: 11, 12 and 13 respectively;
(f) a monoclonal antibody or antigen-binding fragment thereof, comprising: antibody heavy chain variable region HCDR1-3 regions as shown in sequence SEQ ID NOs: 8, 66 and 10 respectively; and antibody light chain variable region LCDR1-3 regions as shown in sequence SEQ ID NOs: 11, 12 and 13 respectively;
(g) a monoclonal antibody or antigen-binding fragment thereof, comprising: antibody heavy chain variable region HCDR1-3 regions as shown in sequence SEQ ID NOs: 8, 67 and 10 respectively; and antibody light chain variable region LCDR1-3 regions as shown in sequence SEQ ID NOs: 11, 12 and 13 respectively;
(h) a monoclonal antibody or antigen-binding fragment thereof, comprising: antibody heavy chain variable region HCDR1-3 regions as shown in sequence SEQ ID NOs: 8, 68 and 10 respectively; and antibody light chain variable region LCDR1-3 regions as shown in sequence SEQ ID NOs: 11, 12 and 13 respectively;
(i) a monoclonal antibody or antigen-binding fragment thereof, comprising: antibody heavy chain variable region HCDR1-3 regions as shown in sequence SEQ ID NOs: 8, 69 and 10 respectively; and antibody light chain variable region LCDR1-3 regions as shown in sequence SEQ ID NOs: 11, 12 and 13 respectively;
(j) a monoclonal antibody or antigen-binding fragment thereof, comprising: antibody heavy chain variable region HCDR1-3 regions as shown in sequence SEQ ID NOs: 8, 70 and 10 respectively; and antibody light chain variable region LCDR1-3 regions as shown in sequence SEQ ID NOs: 11, 12 and 13 respectively;
(k) a monoclonal antibody or antigen-binding fragment thereof, comprising: antibody heavy chain variable region HCDR1-3 regions as shown in sequence SEQ ID NOs: 8, 71 and 10 respectively; and antibody light chain variable region LCDR1-3 regions as shown in sequence SEQ ID NOs: 11, 12 and 13 respectively;
(l) a monoclonal antibody or antigen-binding fragment thereof, comprising: antibody heavy chain variable region HCDR1-3 regions as shown in sequence SEQ ID NOs: 8, 72 and 10 respectively; and antibody light chain variable region LCDR1-3 regions as shown in sequence SEQ ID NOs: 11, 12 and 13 respectively; and
(m) a monoclonal antibody or antigen-binding fragment thereof, comprising: antibody heavy chain variable region HCDR1-3 regions as shown in sequence SEQ ID NOs: 14, 15 and 16 respectively; and antibody light chain variable region LCDR1-3 regions as shown in sequence SEQ ID NOs: 17, 18 and 19 respectively.

12. The pharmaceutical composition according to claim 10 or 11, wherein the TIM-3 antibody or antigen-binding fragment thereof is selected from the group consisting of murine antibody, chimeric antibody and humanized antibody.

13. The pharmaceutical composition according to claim 12, wherein the TIM-3 antibody or antigen-binding fragment thereof is humanized antibody, wherein the sequences of light chain FR regions and heavy chain FR regions of the humanized antibody are from human germline light chain and heavy chain respectively, or mutant sequences thereof.

14. The pharmaceutical composition according to claim 13, wherein the TIM-3 antibody or antigen-binding fragment thereof comprises the heavy chain variable region as shown in SEQ ID NO: 20 or 31 or variant thereof, the variant has 1-10 amino acid back mutations in FR regions of the heavy chain variable region as shown in SEQ ID NO: 20 or 31; preferably, the back mutation is at least one selected from the group consisting of D89E, R98T, G49A, M48I, M70L, R38K, and V68A in SEQ ID NO: 20, or the back mutation is Q3K and/or R87K in SEQ ID NO: 31.

15. The pharmaceutical composition according to claim 13 or 14, wherein the TIM-3 antibody or antigen-binding fragment thereof comprises the light chain variable region as shown in SEQ ID NO: 21 or 32 or variant thereof, the variant has 1-10 amino acid back mutations in the light chain variable region as shown in SEQ ID NO: 21 or 32; preferably, the back mutation is A43S in SEQ ID NO: 21, or at least one selected from the group consisting of Q3K, I48V, K45Q, A43S and T85S in SEQ ID NO: 32.

16. The pharmaceutical composition according to any one of claims 1 to 15, wherein the TIM-3 antibody or antigen-binding fragment thereof comprising:
a heavy chain variable region sequence as shown in any one selected from the group consisting of SEQ ID NO: 44, 45, 46, 47, 48, 49 and 50; and/or a light chain variable region sequence as shown in SEQ ID NO: 29 or 30; or comprising:
a heavy chain variable region sequence as shown in any one selected from the group consisting of SEQ ID NO: 22, 23, 24, 25, 26, 27 and 28; and/or a light chain variable region sequence as shown in SEQ ID NO: 29 or 30; or comprising:
a heavy chain variable region sequence as shown in any one selected from the group consisting of SEQ ID NO: 33, 34 and 35; and/or a light chain variable region sequence as shown in any one selected from the group consisting of SEQ ID NO: 36, 37, 38, 39 and 40; or comprising:
a heavy chain variable region sequence as shown in any one selected from the group consisting of SEQ ID NO: 51, 52, 53, 54, 55, 56, 57, 58, 59, 60 and 61; and/or a light chain variable region sequence as shown in SEQ ID NO: 29 or 30; or comprising:
a heavy chain variable region sequence as shown in SEQ ID NO: 4 and/or a light chain variable region sequence as shown in SEQ ID NO: 5; or comprising:
a heavy chain variable region sequence as shown in SEQ ID NO: 6 and/or a light chain variable region sequence as shown in SEQ ID NO: 7;
preferably comprising:
a heavy chain variable region sequence as shown in SEQ ID NO: 24 and a light chain variable region sequence as shown in SEQ ID NO: 29; or
a heavy chain variable region sequence as shown in SEQ ID NO: 33 and a light chain variable region sequence as shown in SEQ ID NO: 36; or
a heavy chain variable region sequence as shown in SEQ ID NO: 51 and a light chain variable region sequence as shown in SEQ ID NO: 29; or
a heavy chain variable region sequence as shown in SEQ ID NO: 52 and a light chain variable region sequence as shown in SEQ ID NO: 29.

17. The pharmaceutical composition according to any one of claims 1 to 16, wherein the antibody further comprises a human antibody constant region; preferably comprises a human heavy chain constant region sequence as shown in SEQ ID NO: 41 and a human light chain constant region sequence as shown in SEQ ID NO: 42; most preferably, the antibody comprises a heavy chain as shown in SEQ ID NO: 73 and a light chain as shown in SEQ ID NO: 74.

18. The pharmaceutical composition according to any one of claims 1 to 17, comprising a TIM-3 antibody or antigen-binding fragment thereof, wherein the antibody competes to bind to TIM-3 with the antibody or antigen-binding fragment thereof according to any one of claims 10 to 17.

19. A method for preparing the pharmaceutical composition according to any one of claims 1 to 18, comprising a step of replacing a stock solution of TIM3 antibody with buffer.

20. A lyophilized formulation comprising a TIM3 antibody, wherein the lyophilized formulation is obtained by freeze-drying the pharmaceutical composition according to any one of claims 1 to 18.

21. A reconstitution solution comprising a TIM3 antibody, **characterized in that** the reconstitution solution is prepared by reconstituting the lyophilized formulation according to claim 20.

22. A product comprising a container, the container comprising the pharmaceutical composition according to any one of claims 1 to 18, or comprising the lyophilized formulation according to claim 20, or comprising the reconstitution solution according to claim 21.

23. Use of the pharmaceutical composition according to any one of claims 1 to 18 or the lyophilized formulation according to claim 20 or the reconstitution solution according to claim 21 or the product according to claim 22 in the preparation of a medicament for treating or diagnosing disease related to human TIM-3 positive cells.

24. A method for treating cancer, autoimmune disease and allergic disease, comprising administering a therapeutically effective amount of the pharmaceutical composition according to any one of claims 1 to 18 or the lyophilized formulation according to claim 20 or the reconstitution solution according to claim 21 or the product according to claim 22, to a patient with the disease.

25. The method according to claim 24, wherein the cancer is selected from the group consisting of lung cancer, squamous cell lung cancer, melanoma, kidney cancer, breast cancer, IM-TN breast cancer, colorectal cancer, leukemia, bladder cancer, cervical cancer, colon cancer, gallbladder cancer, laryngeal cancer, liver cancer, thyroid cancer, gastric cancer, salivary gland cancer, prostate cancer, pancreatic cancer and Merkel cell carcinoma; wherein the autoimmune disease is selected from the group consisting of multiple sclerosis, diabetes Type I, rheumatoid arthritis, scleroderma, Crohn's disease, psoriasis, systemic lupus erythematosus (SLE) and ulcerative colitis.
